# FASCICULE DE BREVET EUROPEEN

(11) **EP 1 807 421 B1**
(45) Date de publication et mention de la délivrance du brevet: **16.03.2011**
(21) Numéro de dépôt: 05809089.5
(22) Date de dépôt: 17.10.2005
(51) Int. Cl.: C07D 417/12, C07D 417/14, A61K 31/427

(54) **DERIVES DE 2-AMIDO-4-PHENYLTHIAZOLE, LEUR PREPARATION ET LEUR APPLICATION EN THERAPEUTIQUE**
2-AMIDO-4-PHENYLTHIAZOL-DERIVATE, IHRE HERSTELLUNG UND IHRE THERAPEUTISCHE VERWENDUNG
2-AMIDO-4-PHENYLTHIAZOLE DERIVATIVES, THE PREPARATION AND THE THERAPEUTIC USE THEREOF

(30) Priorité: 19.10.2004 FR 0411083
(43) Date de publication de la demande: 18.07.2007
(73) Titulaire: Sanofi-Aventis, 75013 Paris (FR)
(72) Inventeur: CASELLAS, Pierre, F-34090 Montpellier (FR); FLOUTARD, Daniel, F-34980 Combaillaux (FR); FRAISSE, Pierre, F-34990 Juvignac (FR); JEGHAM, Samir, F-34980 Montferrier-sur-Lez (FR)
(74) Mandataire: Ribard, Carin Marie Lisa
(86) Numéro de dépôt international: PCT/FR2005/002565
(87) Numéro de publication internationale: WO 2006/042954

(56) Documents cités:
- EP-A- 0 001 727
- EP-A- 1 344 525
- WO-A-03/088908
- WO-A-2004/096798
- US-A1- 2002 115 863
- US-A1- 2002 119 962

## Description

L'invention se rapporte à des dérivés de 2-amido-4-phénylthiazole, à leur préparation et à leur application en thérapeutique.

La demande de brevet US 2002/0115863-A décrit des composés de formule :

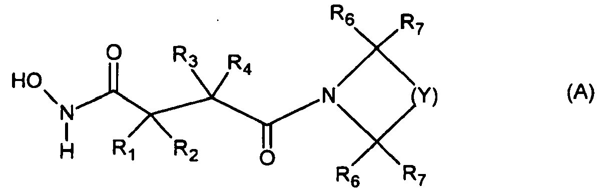

Le substituant sur l'atome d'azote de l'hétérocycle est un dérivé d'acide hydroxamique.

La demande de brevet US 2002/0119962 A décrit des composés de formule :

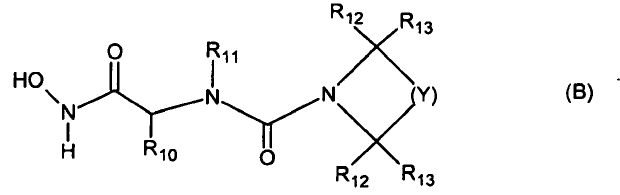

Le substituant sur l'atome d'azote de l'hétérocycle est un dérivé d'acide hydroxamique.

La demande internationale WO 03/088908 décrit des composés de formule : 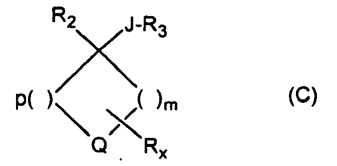 dans laquelle R₂ est un alkyle, cycloalkyle ou aryle.

La demande de brevet EP 1 344 525 décrit des composés de formule: 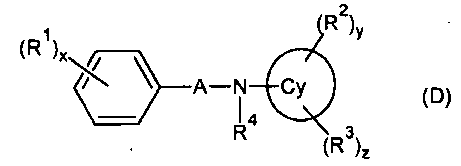 dans laquelle A représente un groupe -CO- ou un groupe SO₂ qui est substitué par un phényle.

La demande de brevet EP 0 001 727 décrit des composés de formule : 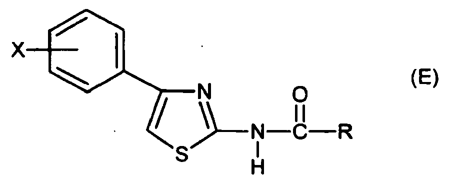 dans laquelle R est un hydrogène, un halogénoalcoxy, un aryl substitué ou non, un pyridyl, un aryloxy ou un carboxyalcoyl.

L'invention a pour objet des composés répondant à la formule (I) ci-après : 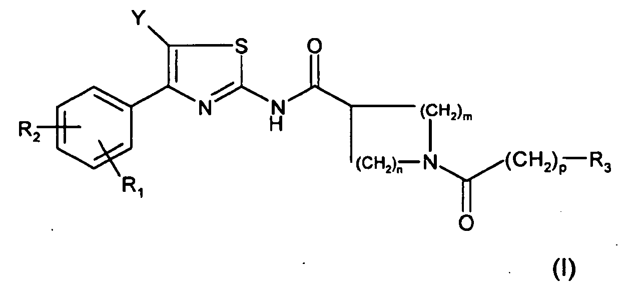 dans laquelle :
- R₁ représente un atome d'hydrogène ou d'halogène, un groupe (C₁-C₄)alkyle, trifluoro(C₁-C₄)alkyle, hydroxyle, (C₁-C₄)alcoxy, trifluoro(C₁₋C₄)alcoxy, (C₃-C₁₀)cycloalkyl(C₁-C₄)alcoxy, (C₃-C₁₀)cycloalkyloxy, allyloxy, (C₁-C₄)alkylthio ;
- R₂ représente un atome d'hydrogène ou d'halogène, un groupe hydroxyle, (C₁-C₄)alkyle, trifluoro(C₁-C₄)alkyle, perfluoro(C₁-C₄)alkyle, (C₃-C₁₀)cycloalkyle, (C₁-C₄)alcoxy, (C₃-C₁₀)cycloalkyl(C₁-C₄)alcoxy, (C₃-C₁₀)cycloalkyloxy, allyloxy, (C₃-C₈)cycloalkyl(C₁-C₄)alkyle ;
- Y représente un atome d'hydrogène ou un halogène ;
- R₃ représente :
   - un groupe phényle substitué par un ou plusieurs atomes d'halogène et/ou par un ou plusieurs groupes :
      (C₁-C₆)alkyle,
      (C₁-C₆)alcoxy,
      -NO₂,
      cyano,
      -COR₄,
      -SO₂R₄,
      -CO₂R₄, dans lequel R₄ représente un groupe (C₁-C₄)alkyle,
      -(CH₂)q NR₅R₆, dans lequel R₅ et R₆ représentent indépendamment l'un de l'autre un atome d'hydrogène, un groupe (C₁-C₄)alkyle ou R₅ et R₆ forment ensemble avec l'atome d'azote auquel ils sont liés un groupe cycloalkyle à 5 ou 6 chaînons et q représente 0, 1 ou 2 ;
   - un groupe hétérocyclique éventuellement substitué par un ou plusieurs halogènes et/ou par un ou plusieurs groupes :
      (C₁-C₄)alkyle,
      hydroxyle,
      (C₁-C₄)alcoxy,
      cyano,
      morpholine,
      trifluoro(C₁-C₄₎alkyle,
      -COR₄,
      -SO₂R₄, dans lequel R₄ est tel que défini dans ce qui précède,
      -(CH₂)_{q}NR₅R₆, dans lequel R₅, R₆ et q sont tels que définis dans ce qui précède,
      phényle,
      pyridine,
      -SCH₃ ;
      le ou les atomes d'azote du groupe hétérocyclique étant éventuellement substitués par un groupe (C₁-C₄)alkyle,
      le ou les atomes d'azote du groupe hétérocyclique étant éventuellement sous leur forme N-oxyde ;
   - un groupe hétérobicyclique éventuellement substitué par un ou plusieurs halogènes et/ou par un ou plusieurs groupes hydroxyles, (C₁-C₄)alkyle ou (C₁-C₄)alcoxy ;
      - m représente 2, 3 ou 4 ;
      - n représente 0, 1 ou 2 ;
      - p représente 0, 1, 2 ou 3.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- (Cₜ-C_{z}) où t et z peuvent prendre les valeurs de 1 à 10, une chaîne carbonée pouvant avoir de t à z atomes de carbone, par exemple (C₁-C₃) une chaîne carbonée qui peut avoir de 1 à 3 atomes de carbone ;
- Hal : un atome d'halogène tel qu'un fluor, un chlore, un brome ou un iode ;
- un groupe alkyle : un groupe aliphatique saturé linéaire ou ramifié. A titre d'exemples, on peut citer les groupes méthyle, éthyle, propyle, isopropyle, butyle, isobutyle, tertbutyle, pentyle, etc ;
- un groupe cycloalkyle : un groupe alkyle cyclique. A titre d'exemples, on peut citer les groupes cyclopropyle, cyclobutyle, cyclopentyle, cyclohexyle, etc ;
- un groupe alcoxy : un groupe -O-alkyle où le groupe alkyle est tel que précédemment défini ;
- un groupe cycloalkyloxy : un groupe -O-cycloalkyle où le groupe cycloalkyle est tel que défini précédemment ;
- un groupe allyloxy : -O-allyl ;
- un groupe perfluoroalkyle : un groupe alkyle, tel que défini précédemment, pour lequel tous les atomes de carbone sont substitués par des atomes de fluor ;
- un groupe hétérocyclique: un groupe cyclique, aromatique ou non, comprenant un ou plusieurs hétéroatomes tels que l'azote, l'oxygène ou le soufre. A titre d'exemples de groupes hétérocycliques, on peut citer les groupes pyridinyle, pyrazinyle, pyrimidinyle, pyridazinyle, pyrazolyle, imidazolyle, thiazolyle, isothiazolyle, oxazolyle, isooxazolyle, oxadiazolyle, thiophényle, pyrrolyle, furanyle ;
- un groupe hétérobicyclique : un groupe hétérocyclique tel que défini ci-dessus, fusionné avec un autre groupe hétérocyclique ou avec un groupe phényle. A titre d'exemples de groupes hétérobicycliques, on peut citer les groupes hétérocyliques ci-dessus fusionnés avec un noyau phényle, par exemple les groupes quinoxaline, quinoline, isoquinoline, indole, isoindole, indoline, indazole, benzothiazole, benzimidazole, benzoxazole ;
- un groupe hétérocyclique sous forme N-oxyde : un groupe de formule suivante : 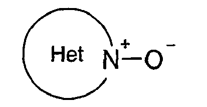 dans laquelle Het représente un groupe hétérocyclique tel que défini dans ce qui précède.

Parmi les composés objets de l'invention, on peut citer un premier groupe de composés de formule (I) dans laquelle :
- R₁ et R₂ sont tels que définis dans ce qui précède ;
- Y représente un atome d'hydrogène ou un halogène ;
- R₃ représente :
   - un groupe phényle, substitué par un ou plusieurs atomes d'halogène et/ou par un ou plusieurs groupes :
      (C₁-C₆)alkyle,
      (C₁-C₆)alcoxy,
      -NO₂,
      cyano,
      -COR₄,
      -SO₂R₄,
      -CO₂R₄, dans lequel R₄ représente un groupe (C₁-C₄)alkyle,
      -(CH₂)_{q}NR₅R₆, dans lequel R₅, R₆ et q sont tels que définis dans ce qui précède ;
   - un groupe hétérocyclique éventuellement substitué par un ou plusieurs halogènes et/ou par un ou plusieurs groupes :
      (C₁-C₄)alkyle,
      hydroxyle,
      (C₁-C₄)alcoxy,
      cyano,
      morpholine,
      trifluoro(C₁-C₄)alkyle,
      -COR₄,
      -SO₂R₄, dans lequel R₄ est tel que défini dans ce qui précède,
      -(CH₂)_{q}NR₅R₆, dans lequel R₅, R₆ et q sont tels que définis dans ce qui précède,
      phényle,
      pyridine,
      -SCH₃ ;
      le ou les atomes d'azote du groupe hétérocyclique étant éventuellement substitués par un groupe (C₁-C₄)alkyle,
      le ou les atomes d'azote du groupe hétérocyclique étant éventuellement sous leur forme N-oxyde ;
   - un groupe hétérobicyclique éventuellement substitué par un ou plusieurs halogènes et/ou par un ou plusieurs groupes hydroxyles, (C₁-C₄)alkyle ou (C₁-C₄)alcoxy ;
      - m représente 3 ;
      - n représente 1 ;
      - p représente 0.

Parmi les composés objets de l'invention, on peut citer un deuxième groupe de composés de formule (I) dans laquelle :
- R₁ et R₂ sont tels que définis dans ce qui précède ;
- Y est tel que défini dans ce qui précède ;
- R₃ représente :
   - un groupe phényle, substitué par un ou plusieurs atomes d'halogène et/ou par un ou plusieurs groupes :
      (C₁-C₆)alkyle,
      (C₁-C₆)alcoxy,
      -NO₂,
      cyano,
      -COR₄,
      -SO₂R₄,
      -CO₂R₄, dans lequel R₄ représente un groupe (C₁-C₄)alkyle,
      -(CH₂)_{q}NR₅R₆, dans lequel R₅ , R₆ et q sont tels que définis dans ce qui précède ;
   - un groupe pyridinyle, pyrazinyle, pyrimidinyle, pyridazinyle, pyrazolyle, imidazolyle, thiazolyle, isothiazolyle, oxazolyle, isooxazolyle, oxadiazolyle, thiophényle, pyrrolyle, furanyle, lesdits groupes étant éventuellement substitués par un ou plusieurs halogènes et/ou par un ou plusieurs groupes :
      (C₁-C₄)alkyle,
      hydroxyle,
      (C₁-C₄)alcoxy,
      cyano,
      morpholine,
      trifluoro( C₁-C₄)alkyle,
      -COR₄,
      -SO₂R₄, dans lequel R₄ est tel que défini dans ce qui précède,
      -(CH₂)_{q}NR₅R₆, dans lequel R₅, R₆ et q sont tels que définis dans ce qui précède,
      phényle,
      pyridine,
      -SCH₃ ;
      le ou les atomes d'azote desdits groupes cités ci-dessus étant éventuellement substitués par un groupe (C₁-C₄)alkyle,
      le ou les atomes d'azote desdits groupes cités ci-dessus étant éventuellement sous leur forme N-oxyde ;
   - un groupe quinoxaline, quinoline, isoquinoline, indole, isoindole, indoline, indazole, benzothiazole, benzimidazole, benzoxazole, lesdits groupes étant éventuellement substitués par un ou plusieurs halogènes et/ou par un ou plusieurs groupes hydroxyles, (C₁-C₄)alkyle ou (C₁-C₄)alcoxy ;
      - m représente 3 ;
      - n représente 1 ;
      - p représente 0.

Parmi les composés objets de l'invention, on peut citer un troisième groupe de composés de formule (I) dans laquelle:
- R₁ et R₂ sont tels que définis dans ce qui précède ;
- Y représente un atome d'hydrogène ou un halogène ;
- R₃ représente :
   - un groupe phényle, substitué par un ou plusieurs atomes d'halogène et/ou par un ou plusieurs groupes :
      (C₁-C₆)alkyle,
      (C₁-C₆)alcoxy,
      -NO_{2,}
      cyano,
      -COR₄,
      -SO₂R₄,
      -CO₂R₄, R₄ représentant un groupe (C₁-C₄)alkyle,
      -(CH₂)_{q}NR₅R₆, dans lequel R₅ , R₆ et q sont tels que définis dans ce qui précède ;
   - un groupe hétérocyclique éventuellement substitué par un ou plusieurs halogènes et/ou par un ou plusieurs groupes :
      (C₁-C₄)alkyle,
      hydroxyle,
      (C₁-C₄)alcoxy,
      cyano,
      morpholine,
      trifluoro(C₁-C₄)alkyle,
      -COR₄,
      -SO₂R₄, dans lequel R₄ est tel que défini dans ce qui précède,
      -(CH₂)_{q}NR₅R₆, dans lequel R₅, R₆ et q sont tels que définis ce qui précède,
      phényle,
      pyridine,
      -SCH₃ ;
      le ou les atomes d'azote du groupe hétérocyclique étant éventuellement substitués par un groupe (C₁-C₄)alkyle,
      le ou les atomes d'azote du groupe hétérocyclique étant éventuellement sous leur forme N-oxyde ;
   - un groupe hétérobicyclique éventuellement substitué par un ou plusieurs halogènes et/ou par un ou plusieurs groupes hydroxyles, (C₁-C₄)alkyle ou (C₁-C₄)alcoxy ;
      - m représente 2 ou 4 ; et/ou
      - n représente 0, 1 ou 2; et/ou
      - p représente 0, 1 ou 2.

Parmi les composés objets de l'invention, on peut citer un quatrième groupe de composés de formule (I) dans laquelle :
- R₁ et R₂ sont tels que définis ce qui précède ;
- Y représente un atome d'hydrogène ou un halogène ;
- R₃ représente :
   - un groupe phényle, substitué par un ou plusieurs atomes d'halogène et/ou par un ou plusieurs groupes :
      (C₁-C₆)alkyle,
      (C₁-C₆)alcoxy,
      -NO₂,
      cyano,
      -COR₄,
      -SO₂R₄,
      -CO₂R₄, dans lequel R₄ représente un groupe (C₁-C₄)alkyle,
      -(CH₂)_{q}NR₅R₆, dans lequel R₅ , R₆ et q sont tels que définis dans ce qui précède ;
   - un groupe pyridinyle, pyrazinyle, pyrimidinyle, pyridazinyle, pyrazolyle, imidazolyle, thiazolyle, isothiazolyle, oxazolyle, isooxazolyle, oxadiazolyle, thiophényle, pyrrolyle, furanyle, lesdits groupes étant éventuellement substitués par un ou plusieurs halogènes et/ou par un ou plusieurs groupes :
      (C₁-C₄)alkyle,
      hydroxyle,
      (C₁-C₄)alcoxy,
      cyano,
      morpholine,
      trifluoro(C₁-C₄)alkyle,
      -COR₄,
      -SO₂R₄, dans lequel R₄ est tel que défini dans ce qui précède,
      -(CH₂)_{q}NR₅R₆, dans lequel R₅, R₆ et q sont tels que définis dans ce qui précède,
      phényle,
      pyridine,
      -SCH₃ ;
      le ou les atomes d'azote desdits groupes cités ci-dessus étant éventuellement substitués par un groupe (C₁-C₄)alkyle,
      le ou les atomes d'azote desdits groupes cités ci-dessus étant éventuellement sous leur forme N-oxyde ;
   - un groupe quinoxaline, quinoline, isoquinoline, indole, isoindole, indoline, indazole, benzothiazole, benzimidazole, benzoxazole, lesdits groupes précités étant éventuellement substitués par un ou plusieurs halogènes et/ou par un ou plusieurs groupes hydroxyles, (C₁-C₄)alkyle ou (C₁-C₄)alcoxy ;
      - m représente 2 ou 4 ; et/ou
      - n représente 0, 1 ou 2 ; et/ou
      - p représente 0, 1 ou 2.

Parmi les composés objets de l'invention, on peut citer un cinquième groupe de composés de formule (I) dans laquelle :
- R₁ et R₂ sont tels que définis dans ce qui précède ;
- Y représente un atome d'hydrogène ou un halogène ;
- R₃ représente :
   - un groupe phényle, substitué par un ou plusieurs atomes d'halogène et/ou par un ou plusieurs groupes :
      (C₁-C₆)alkyle,
      (C₁-C₆)alcoxy,
      -NO₂,
      cyano,
      -COR₄,
      -SO₂R₄,
      -CO₂R₄, dans lequel R₄ représente un groupe (C₁-C₄)alkyle,
      -(CH₂)_{q}NR₅R₆, dans lequel R₅, R₆ et q sont tels que définis dans ce qui précède ;
   - un groupe pyridinyle, pyrazinyle, pyrimidinyle, pyridazinyle, pyrazolyle, imidazolyle, thiazolyle, isothiazolyle, oxazolyle, isooxazolyle, oxadiazolyle, thiophényle, pyrrolyle, furanyle, lesdits groupes précités étant éventuellement substitués par un ou plusieurs halogènes et/ou par un ou plusieurs groupes :
      (C₁-C₄)alkyle,
      hydroxyle,
      (C₁-C₄)alcoxy,
      cyano,
      morpholine,
      trifluoro(C₁-C₄)alkyle,
      -COR₄,
      -SO₂R₄, dans lequel R₄ est tel que défini dans ce qui précède,
      -(CH₂)_{q}NR₅R₆, dans lequel R₅, R₆ et q sont tels que définis dans ce qui précède,
      phényle,
      pyridine,
      -SCH₃ ;
      le ou les atomes d'azote desdits groupes cités ci-dessus étant éventuellement substitués par un groupe (C₁-C₄)alkyle,
      le ou les atomes d'azote desdits groupes cités ci-dessus étant éventuellement sous leur forme N-oxyde ;
   - un groupe quinoxaline, quinoline, isoquinoline, indole, isoindole, indoline, indazole, benzothiazole, benzimidazole, benzoxazole, lesdits groupes précités étant éventuellement substitués par un ou plusieurs halogènes et/ou par un ou plusieurs groupes hydroxyles, (C₁-C₄)alkyle ou (C₁-C₄)alcoxy ;
      - m représente 2 ou 4 ; et/ou
      - n représente 0, 1 ou 2 ; et/ou
      - p représente 0.

Des composés de formule (I) selon l'invention sont ceux dans lesquels R₁ est en position 2 du phényle et R₂ est en position 5 du phényle.

Des composés de formule (I) selon l'invention sont ceux dans lesquels R₁ est en position 2 du phényle, R₁ représentant notamment un groupe (C₁-C₄)alcoxy et R₂ est en position 5 du phényle, R₂ représentant notamment un groupe (C₁-C₄)alcoxy, (C₁-C₄)alkyle ou (C₃-C₁₀)cycloalkyle.

Parmi les composés de formule (I) de l'invention, on peut notamment citer les composés suivants :
- Acide 1-(1,5-diméthyl-1H-pyrazole-3-carbonyl)-pipéridine-3-carboxylique-[4-(5-cyclohexyl-2-méthoxy-phényl)-thiazol-2-yl]-amide (composé n° 114)
- Acide 1-(1-méthyl-1H-imidazole-2-carbonyl)-pipéridine-3-carboxylique-[4-(5-cyclohexyl-2-méthoxy-phényl)-thiazol-2-yl]-amide (composé n° 112),
- Acide 1-(pyrimidine-4-carbonyl)-pipéridine-3-carboxylique-[4-(5-cyclohexyl-2-méthoxy-phényl)-thiazol-2-yl]-amide (composé n° 107),
- Acide 1-(pyrazine-2-carbonyl)-pipéridine-3-carboxylique-[4-(5-cyclohexyl-2-éthoxy-phényl)-thiazol-2-yl]-amide (composé n° 98),
- Acide 1-(1-oxy-pyridine-2-carbonyl)-pipéridine-3-carboxylique-[4-(5-cyclopentyl-2-méthoxy-phényl)-thiazol-2-yl]-amide (composé n° 118),
- Acide 1-(2-hydroxy-pyridine-3-carbonyl)-pipéridine-3-carboxylique-[4-(5-cyclohexyl-2-méthoxy-phényl)-thiazol-2-yl]-amide (composé n° 101),
- Acide 1-(1-oxy-pyridine-3-carbonyl)-pipéridine-3-carboxylique-[4-(5-cyclohexyl-2-méthoxy-phényl)-thiazol-2-yl]-amide (composé n° 115),
- Acide 1-(5-chloro-1-méthyl-1H-pyrazole-4-carbonyl)-pipéridine-3-carboxylique-[4-(5-cyclohexyl-2-méthoxy-phényl)-thiazol-2-yl]-amide (composé n° 27),
- Acide 1-(furan-2-carbonyl)-pipéridine-3-carboxylique-[4-(5-cyclohexyl-2-méthoxy-phényl)-thiazol-2-yi]-amide (composé n°28),
- Acide 1-(pyrazine-2-carbonyl)-pipéridine-4-carboxylique-[4-(5-cyclohexyl-2-méthoxy-phényl)-thiazol-2-yl]-amide (composé n° 120),
- Acide 1-(pyrazine-2-carbonyl)-pipéridine-2-carboxylique-[4-(5-cydohexyl-2-méthoxy-phényl)-thiazol-2-yl]-amide (composé n° 109),
- Acide 1-(2-pyridin-2-yl-acétyl)-pipéridine-3-carboxylique-[4-(5-cyclohexyl-2-méthoxy-phényl)-thiazol-2-yl]-amide (composé n° 60),
- Acide 1-(1-oxy-pyridine-2-carbonyl)-pipéridine-3-carboxylique-[4-(5-cyclohexyl-2-méthoxy-phényl)-thiazol-2-yl]-amide (composé n° 102), .
- Acide (S)-1-(1-oxy-pyridine-2-carbonyl)-pipéridine-3-carboxylique-[4-(5-cyclohexyl-2-méthoxy-phényl)-thiazol-2-yl]-amide(composé n° 124),
- Acide 1-(pyrazine-2-carbonyl)-pipéridine-3-carboxylique-[4-(5-cyclohexyl-2-méthoxy-phényl)-thiazol-2-yl]-amide (composé n° 97),
- Acide (S)-1-(pyrazine-2-carbonyl)-pipéridine-3-carboxylique-[4-(5-cyclohexyl-2-méthoxy-phényl)-thiazol-2-yl]-amide (composé n° 108),
- Acide 1-(1-oxy-pyridine-2-carbonyl)-pipéridine-3-carboxylique-[4-(5-butyl-2-méthoxy-phényl)-thiazol-2-yl]-amide (composé n° 117),
- Acide 1-(pyrazine-2-carbonyl)-pipéridine-3-carboxylique-[4-(5-cyclopentyl-2-méthoxy-phényl)-thiazol-2-yl]-amide (composé n° 100),
- Acide (R)-1-(pyrazine-2-carbonyl)-pipéridine-3-carboxylique-[4-(5-cyclohexyl-2-méthoxy-phényl)-thiazol-2-yl]-amide (composé n° 121)
- Acide 1-(1-Oxy-pyridine-2-carbonyl)-pyrrolidine-3-carboxylique-[4-(5-cyclohexyl-2-méthoxy-phényl)-thiazol-2-yl]-amide (composé n° 105), • Acide (R)-1-(1-Oxy-pyridine-2-carbonyl)-pyrrolidine-3-carboxylique-[4-(5-cyclohexyl-2-méthoxy-phényl)-thiazol-2-yl]-amide (composé n°239).

L'invention a également pour objet un procédé de préparation des composés de formule (I).

Dans ce qui suit, on entend par groupe protecteur Gp un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont donnés dans « Protective Groups in Organic Synthesis », Green et al., 2nd Edition (John Wiley & Sons, Inc., New York).

On entend dans ce qui suit par groupe partant X un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans « Advances in Organic Chemistry », J. March, 3rd Edition, Wiley Intersciences, p. 310-316.

Dans ce qui suit, on entend par précurseur de R₁ et/ou R₂, un substituant R'₁ et/ou R'₂ pouvant être transformé en R₁ et/ou R₂ par une ou plusieurs réactions chimiques connues par l'homme du métier, ou R₁ et/ou R₂ représentent R₁ et/ou R₂.

On entend par groupe Z dans ce qui suit, un groupe partant ou un dérivé fonctionnel issu d'une fonction acide, tel qu'un chlorure d'acide, un anhydride mixte ou symétrique, ou encore l'acide opportunément activé par exemple avec l'hexafluoro phosphate de benzotriazol-1-yloxytris(diméthylamino) phosphonium (BOP), le O-benzotriazol-1-yl-N,N,N',N'-tétraméthyluronium hexafluorophosphate (HBTU) ou le O-benzotriazol-1-yl-N,N,N',N'-tétraméthyluronium tétrafluoroborate (TBTU).

Lorsqu'un ou plusieurs substituants R'₁ et/ou R'₂ représentent un groupement contenant une fonction amine ou hydroxyle, ces fonctions peuvent être protégées intermédiairement : une fonction amine peut être protégée par un groupement alcanoyle, benzyle, tert-butoxycarbonyle (Boc), benzyloxycarbonyle, ou 9-fluorénylméthoxycarbonyle (Fmoc), par exemple ; une fonction hydroxyle peut être protégée sous forme d'éther ou d'ester, par exemple.

Les composés de l'invention peuvent être préparés selon différentes méthodes décrites dans ce qui suit.

Avant d'aborder ces méthodes, il est décrit ci-après les méthodes de préparation des dérivés aminothiazole de formule (II) qui sont utilisés pour la préparation des composés de formule (I) de l'invention.

Dans ce qui suit :
- THF = tétrahydrofuranne,
- BOP = benzotriazole-1-yle-oxy-tris(diméthylamino)-phosphoniumhéxafluorophosphate,
- CDI = 1,1'-carbonyl-diimidazole,
- DMF = diméthylformamide,
- TMS = tétraméthylsilane,
- DMSO = dimethylsulfoxyde,
- DCM = dichlorométhane,
- TBTU = 2-(1H-Benzotriazole-1-yl)-1,1,3,3-tetraméthyluroniumtétrafluoroborate.

Les dérivés aminothiazole de formule (II) peuvent être préparés par des méthodes connues telles que celles décrites dans les documents EP 518 731, EP 611 766 et WO 99/15525.

De façon générale, quand Y = H, on fait réagir la thiourée avec une cétone halogénée de formule 1 selon le schéma réactionnel suivant :

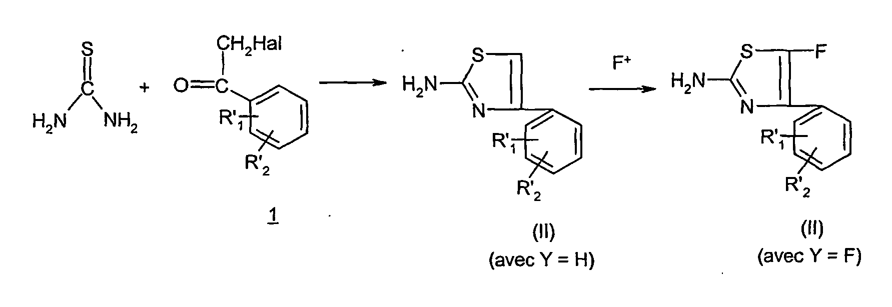

Les substituants R'₁ et R'₂ ont les valeurs indiquées plus haut, c'est à dire que R'₁ et R'₂ représentent respectivement R₁ et R₂ tels que définis pour le composé de formule générale (I) ou des groupes précurseurs de R₁ et R₂; Hal représente un atome d'halogène, de préférence le brome, le chlore ou l'iode.

Comme indiqué sur le schéma précédent, les composés de type (II) avec Y = H, R'₁, et R'₂ ayant les valeurs indiquées plus haut, peuvent être transformés en composés de type (II) avec Y = F et R'₁ et R'₂ ayant les valeurs indiquées plus haut par réaction avec un agent de fluoration comme par exemple le Selectfluor™ dans un solvant comme le DMF ou le DCM à une température variant de 0°C à 50°C.

Les cétones halogénées de formule 1 peuvent être préparées par des procédés connus de l'homme de l'art. Par exemple, les bromocétones peuvent être obtenues par action du brome, du bromure cuivrique ou du phényltriméthylammoniumtribromure (PTT) sur un dérivé d'acétophénone de formule 2 : 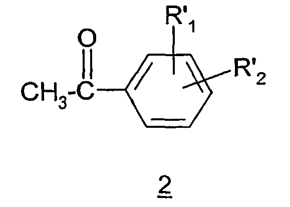 dans laquelle R'₁ et R'₂ ont les valeurs indiquées ci-dessus, dans un solvant organique tel que l'acétate d'éthyle, un solvant chloré ou leur mélange ou encore un alcool.

Lorsque le dérivé acétophénone de formule 2 n'est pas disponible commercialement, on peut le préparer par différentes méthodes :
- une réaction de Friedel-Crafts sur le benzène substitué par R'₁ et R'₂ que l'on fait réagir sur le chlorure d'acétyle ou l'anhydride acétique, en présence d'un acide de Lewis tel que AlCl₃ ou TiCl₄, par exemple;
- l'action du chlorure d'acétyle en présence de Palladium sur le benzène substitué par R'₁ et R'₂ après déprotonation du benzène, par exemple par action du butyllithium puis addition du chlorure de zinc ou de l'iodure de manganèse. Ce mode opératoire est utilisable pour préparer un dérivé d'acétophénone de formule 2 dans laquelle R'₂ = R₂ = (C₁-C₄)perfluoroalkyle ;
- un réarrangement de Fries à partir d'un dérivé d'acétoxybenzène de formule 3 : 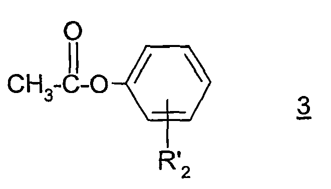 par action d'un acide de Lewis, on obtient un dérivé d'hydroxyacétophénone de formule 4 :

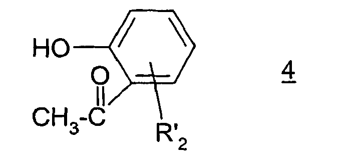

La fonction hydroxyle correspond à un groupement R'₁ que l'on peut transformer dans une étape ultérieure en un groupement -O-W tel que (C₁-C₆)alcoxy, trifluorométhoxy, trifluoroéthoxy, allyloxy, (C₃-C₁₀)cycloalkylméthoxy, (C₃-C₁₀)cydoalkyloxy.

La transformation de R'₁, en R₁ peut être effectuée soit sur l'aminothiazole de formule (II), soit sur un composé de formule (I).

Les dérivés du benzène substitués par R'₁ et R'₂ sont disponibles commercialement ou sont préparés par des méthodes connues de l'homme de l'art.

Par exemple, pour préparer un composé dans lequel R₁ est un groupement -O-W tel que défini ci-dessus, on opère de la façon suivante :

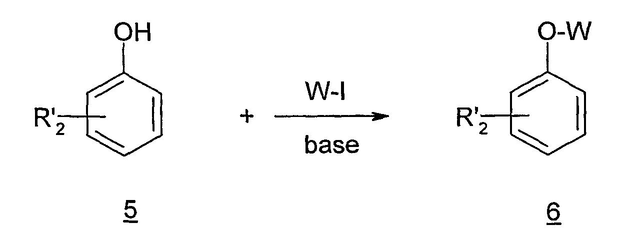

On peut également substituer un dérivé d'halogénobenzène selon le schéma ci-après :

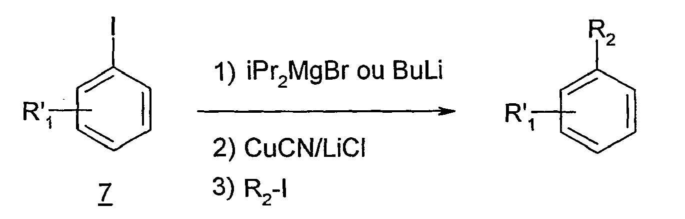

Dans le cas particulier où R₂ représente un (C₁-C₄)perfluoroalkyle, on peut également procéder selon le schéma réactionnel ci-après :

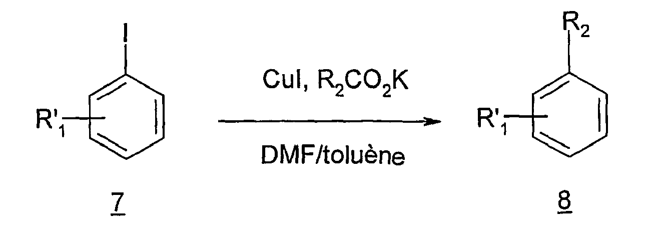

Des exemples de préparation de dérivés aminothiazole de formule (II) sont donnés à titre illustratif dans ce qui suit.

Les composés de formule (I) de l'invention peuvent être préparés selon le schéma général 1 ci-après.

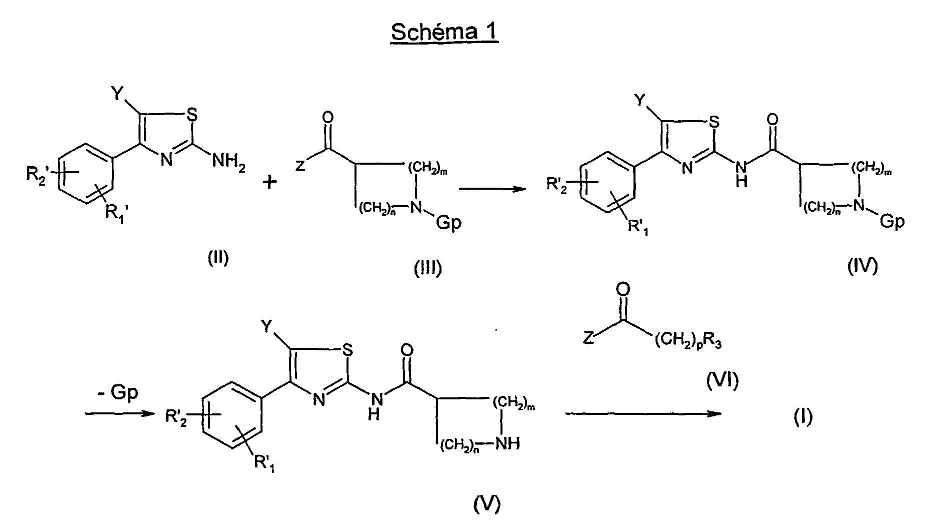

Selon le schéma 1, on couple un composé de formule (II) dans laquelle Y, R'₁ et R'₂ sont tels que définis dans ce qui précède, avec un composé de formule (III) dans laquelle Z, Gp, m et n sont tels que définis dans ce qui précède, via une acylation ou un couplage de type peptidique, en présence d'une base telle que K₂CO₃, la triéthylamine, le carbonate de césium ou d'un réactif de couplage comme le BOP, le TBTU ou le CDI, dans un solvant choisi parmi le THF, l'acétonitrile ou le DMF notamment, à des températures comprises entre 0°C et 150°C. Le composé de formule (IV) ainsi obtenu est déprotégé pour donner le dérivé amine de formule (V). Dans le composé de formule (IV), Y, m, n, Gp, R'₁ et R'₂ sont tels que définis dans ce qui précède. On fait ensuite réagir le composé de formule (V) dans laquelle Y, m, n, R'₁ et R'₂ sont tels que définis dans ce qui précède, avec un acide ou un dérivé d'acide de formule (VI) dans laquelle R₃, Z et p sont tels que définis dans ce qui précède, en présence d'une base telle que K₂CO₃, la triéthylamine, le carbonate de césium ou d'un réactif de couplage comme le BOP, le TBTU ou le CDI, dans un solvant choisi parmi le THF, l'acétonitrile ou le DMF notamment, à des températures comprises entre 0°C et 150°C.

Les composés de formule (III), lorsqu'ils ne sont pas disponibles commercialement, peuvent être préparés à partir des procédés décrits dans la littérature, par exemple dans la publication H. Rapoport, J. Org. Chem. 1974, 39, 893 et dans le document WO 97/41102.

On peut également préparer les composés de type (I) selon le schéma 2 suivant :

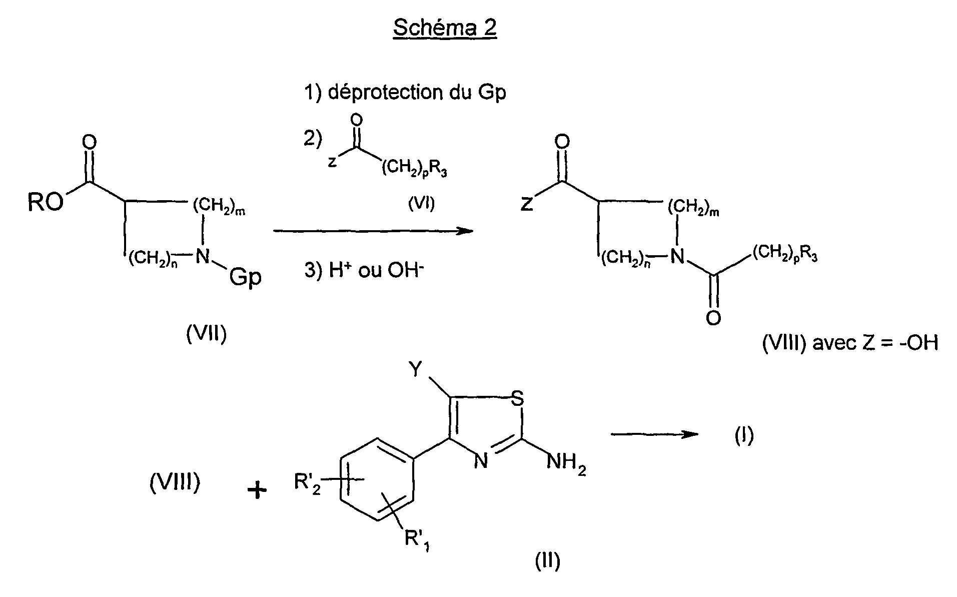

Selon le schéma 2, on déprotège puis on condense un composé de formule (VII), dans laquelle R représente un groupe (C₁-C₄)alkyle et Gp un groupe protecteur tel que défini dans ce qui précède, avec un composé de formule (VI) dans laquelle R₃, Z et p sont tels que définis dans ce qui précède, en présence d'une base telle que K₂CO₃, la triéthylamine, le carbonate de césium ou d'un réactif de couplage comme le BOP, le TBTU ou le CDI, dans un solvant choisi parmi le THF, l'acétonitrile ou le DMF notamment, à des températures comprises entre 0°C et 150°C. On obtient un dérivé acide de formule (VIII) après saponification ou hydrolyse acide. On condense ensuite le composé de formule (VIII) dans laquelle R₃, m, n et p sont tels que définis dans ce qui précède, avec un composé de formule (II) telle que définie précédemment, en présence d'une base telle que K₂CO₃, la triéthylamine, le carbonate de césium ou d'un réactif de couplage comme le BOP, le TBTU ou le CDI, dans un solvant choisi parmi le THF, l'acétonitrile ou le DMF notamment, à des températures comprises entre 0°C et 150°C, pour obtenir le composés de formule (I).

Les composés de formule (VII) ainsi que leurs dérivés déprotégés sont disponibles commercialement ou peuvent être préparés à partir des procédés décrits dans le document WO 92/15585.

Dans les deux schémas généraux 1 et 2 décrits ci-dessus, le composé (VI) est disponible commercialement ou peut être obtenu par homologation de l'acide carboxylique commercial selon des méthodes classiques, telles que des réactions de type Arndt-Eistert (Tet. Lett., 1979, 29, 2667 "Advanced in Organic Chemistry", J. March, 3nd Edition, Wiley Interscience, p. 1405-1407), selon le schéma ci-après :

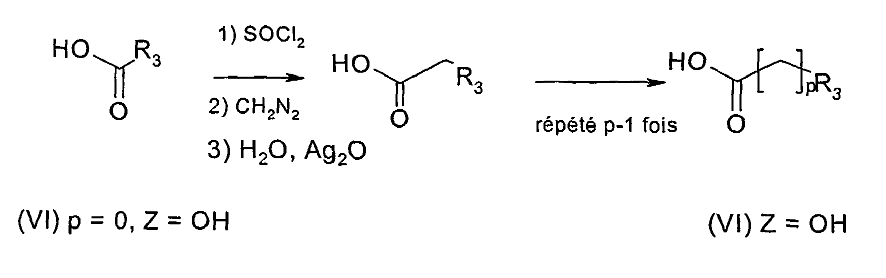

Dans les exemples qui suivent, lorsque le composé (VI) avec p = 0 et Z = -OH n'est pas disponible commercialement, il est préparé selon le procédé décrit dans J. Med. Chem. 1977, 20, 1312.

Dans les schémas généraux de synthèse 1 et 2, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

L'invention, selon un autre de ses aspects, a également pour objet les composés de formule (V') qui suit : 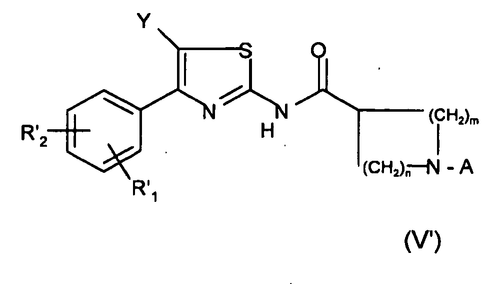 dans laquelle R'₁ et R'₂ représentent des précurseurs de R₁ et R₂ tels que définis dans ce qui précède, Y, m et n sont tels que définis dans ce qui précède et A représente un atome d'hydrogène ou un groupe protecteur Gp qui est le *tert-*butoxycarbonyle (Boc).

Ces composés sont utiles comme intermédiaires de synthèse des composés de formule (I). Des exemples de composés de formule (V') sont donnés dans le tableau II ci-après.

Les exemples qui suivent décrivent la préparation de composés de formule (I) conformes à l'invention. Ces exemples ne sont pas limitatifs et ne font qu'illustrer l'invention. Les numéros des composés exemplifiés renvoient à ceux donnés dans le tableau III, qui illustre les structures chimiques et les propriétés physiques de quelques composés selon l'invention.

Les numéros des préparations des composés de formule (II) renvoient à ceux donnés dans le tableau I.

Dans les préparations et exemples qui suivent :
- TA = température ambiante,
- DCM = dichlorométhane,
- DIPEA = diisopropyléthylamine,
- THF = tétrahydrofuranne,
- BOP = benzotriazole-1-yle-oxy-tris(diméthylamino)-phosphonium hexafluorophosphate,
- DMF = diméthylformamide,
- Boc = *tert*butyloxycarbonyle,
- TFA = trifluoroacétic acid (acide trifluoroacétique, en français),
- TBTU = 2-(1H-Benzotriazole-1-yl)-1,1,3,3-tétraméthyluroniumtétrafluoroborate,
- HOBT = hydroxybenzotriazole,
- BSA = bis(triméthylsilyl)acétamide,
- AcOEt = acétate d'éthyle,
- AcCI = chlorure d'acétyle.
- PF = Point de Fusion (en degré Celsius) tel que mesuré sur un appareil Büchi B545 avec un gradient de température de 1 °C par minute.
- MH+ = Spectre de Masse. Les composés sont analysés par couplage HPLC - UV - MS (chromatographie liquide - détection UV - spectrométrie de masse). L'appareil utilisé, commercialisé par Agilent, est composé d'un chromatographe HP1100 équipé d'un détecteur à barrette de diodes Agilent et d'un spectromètre de masse quadripolaire MSD Quad.

Les conditions analytiques sont les suivantes :
Colonne : Symmetry C18 (50 x 2,1 mm; 3,5 µm)
Eluant A : H₂O + TFA 0,005 % à pH 3,15
Eluant B : CH₃CN + TFA 0,005 %
Gradient :

| Temps (min) | % B |
|---|---|
| 0 | 0 |
| 10 | 90 |
| 15 | 90 |
| 16 | 0 |
| 20 | 0 |

Température de la colonne : 30°C
Débit: 0,4 mL / min
Détection : λ = 210 nm
- tr = temps de rétention.
- RMN = résonance magnétique nucléaire réalisée avec un spectromètre Bruker Avance 200 (200 MHz). Le solvant employé est le DMSO deutéré et les déplacements chimiques sont exprimés par rapport au TMS. Les abréviations utilisées sont :
- s = singulet,
- d = doublet,
- d.d = doublet dédoublé,
- t = triplet,
- m = multiplet,
- sél = singulet élargi.
- L'analyse de la pureté optique est mesurée par HPLC sur colonne Chiralpak AD (250 mm x 4,6) éluée par un mélange CO₂ / MeOH 80 / 20 à 30 °C avec un débit de 3 mL / min à P = 20 MPa. Les composés sont détectés à 220 nm.
- α_{D} = pouvoir rotatoire. Les pouvoirs rotatoires ont été déterminés avec un polarimètre Perkin-Elmer 241-MC pour la raie D du sodium (λ = 589 nm), les concentrations sont exprimées en 10 mg/mL, les mesures sont effectuées à température ambiante.

### I. Préparation de dérivés aminothiazole de formule (II)

### Préparation I.1

### 4-(2-Méthoxy-5-propoxyphényl)-1,3-thiazol-2-amine.

### A) 1-(2-Hydroxy-5-propoxyphényl)-éthanone.

Dans un ballon de 500 mL sont placés 10 g de 2,5-dihydroxyacétophénone en suspension dans 100 mL d'acétone, 9,14 g de K₂CO₃ anhydre sont ajoutés suivis par 12,4 g d'iodure de propyle. Le milieu réactionnel est chauffé à reflux pendant 30 heures. Après retour à température ambiante, le milieu est filtré sur Célite^{®} puis concentré. L'huile brune obtenue est reprise dans l'AcOEt, filtrée, lavée à l'eau, avec une solution d'HCl 2M, puis avec une solution saturée de NaCl. La phase organique est évaporée pour donner une pâte noire. La pâte est reprise dans le chloroforme et filtrée. Le milieu est concentré pour donner 11,4 g d'un solide noir. Ce dernier est repris dans l'éthanol absolu. La solution est placée 10 minutes au congélateur, un solide précipite, il est collecté par filtration. Le filtrat est concentré, repris dans l'éthanol, refroidi au congélateur puis filtré à nouveau. Cette opération est répétée 4 fois pour donner 8,35 g du composé attendu sous forme d'une poudre.

### B) 1-(2-Méthoxy-5-propoxyphényl)-éthanone.

A une solution de 35 g du solide précédent dans 350 mL de DMF, on ajoute 49,8 g de K₂CO₃, puis 22,4 mL d'iodure de méthyle. Le milieu réactionnel est chauffé pendant 12 heures à 60°C. Après retour à température ambiante, le milieu est filtré sur Célite^{®}, dilué dans l'éther et lavé avec une solution d'HCl 2M. La phase aqueuse est extraite 2 fois dans l'éther. Les phases organiques rassemblées sont lavées avec une solution de soude diluée, puis lavées à l'eau 2 fois et avec une solution saturée de NaCl. La phase organique est séchée sur MgSO₄, puis évaporée pour donner 35,55 g d'une huile brune. L'huile est distillée sous pression réduite à 115°C, pour donner 32,8 g du composé attendu sous forme d'une huile.

### C) 2-Bromo-1-(2-méthoxy-5-propoxyphényl)-éthanone.

A une solution de 16,4 g de l'huile obtenue à l'étape précédente dans 100 mL de méthanol, on ajoute au goutte-à-goutte 4,8 mL de brome. Le milieu est agité 30 minutes à température ambiante, puis évaporé. L'huile obtenue est reprise dans le dichlorométhane, lavée 3 fois dans l'eau, puis séchée sur MgSO₄ et évaporée pour donner 24,5 g d'une huile brune.

### D) 4-(2-Méthoxy-5-propoxyphényl)-1,3-thiazol-2-amine.

A une solution de 42 g de la bromocétone préparée à l'étape précédente dans 200 mL d'éthanol, sont ajoutés 24,5 g de thiourée. Le milieu est porté à reflux pendant 1 heure 30 minutes. Le milieu est alors placé au réfrigérateur pendant 12 heures, puis filtré. Le solide ainsi collecté est rincé avec un peu d'éthanol froid, puis à l'éther. On récupère 25 g de bromhydrate.

Le solide est mis en suspension dans un mélange eau/dichlorométhane et on effectue un retour à la base par addition de soude. La phase aqueuse est extraite 2 fois dans le dichlorométhane. Les phases organiques rassemblées sont séchées sur MgSO₄, puis évaporées. L'huile obtenue est chromatographiée sur gel de silice pour donner 12 g du produit attendu sous forme de poudre.
PF = 76°C

### Préparation I.2

### 4-(5-Butyl-2-méthoxyphényl)-1,3-thiazol-2-amine

### A) 4-Butylphényl acétate

Une solution de 10 g de 4-n-butylphénol, 10 mL d'Ac₂O et 8 mL de pyridine est agitée à reflux dans 10 mL de dichlorométhane. Après 2 heures, le milieu est refroidi à température ambiante, dilué dans le dichlorométhane, lavé à l'eau, lavé avec une solution d'HCl 1 M, lavé dans une solution de CuSO₄ saturée, lavé à l'eau et séché sur MgSO₄. Après évaporation, on récupère 10,8 g du composé attendu sous forme d'une huile.

### B) 1-(5-Butyl-2-hydroxyphényl)-éthanone

A 5 g de l'huile obtenue à l'étape précédente dans un ballon de 100 mL, on ajoute en plusieurs fois 3,22 g d'AlCl₃. Le milieu est chauffé à 130°C pendant 1 heure. Après retour à température ambiante, une solution d'eau glacée acidifiée par de l'HCl 35 % est coulée sur le brut réactionnel. Le milieu est placé dans un bain à ultrasons. On additionne de l'AcOEt pour obtenir, après 15 minutes, la solubilisation du milieu. La phase aqueuse est extraite 3 fois dans l'AcOEt, les phases organiques sont lavées à l'eau, puis avec une solution saturée de NaCl. Après séchage sur MgSO₄ et évaporation, on récupère 4,5 g d'une huile jaune.

### C) 1-(5-Butyl-2-méthoxyphényl)-éthanone

A une solution d'1 g de l'huile obtenue à l'étape précédente dans 10 mL de DMF, on ajoute 1,44 g de K₂CO₃ puis 0,648 mL d'iodure de méthyle. Le milieu est chauffé à 60°C pendant une nuit. Après retour à température ambiante, le milieu est filtré sur Célite^{®}, dilué dans l'éther et lavé avec une solution d'HCl 2M. La phase aqueuse est extraite 2 fois dans l'éther. Les phases organiques rassemblées sont lavées avec une solution de soude diluée, puis lavées à l'eau deux fois et avec une solution saturée de NaCl. La phase organique est séchée sur MgSO₄, puis évaporée pour donner 1,27 g d'une huile brune. L'huile est purifiée par chromatographie et on obtient 0,66 g du composé attendu.

### D) 4-(5-Butyl-2-méthoxyphényl)-1,3-thiazol-2-amine

A une solution de 0,66 g du produit de l'étape précédente dans 10 mL de méthanol, on additionne 0,19 mL de brome. Le milieu est agité pendant 10 minutes, puis évaporé et repris dans le dichlorométhane. La phase organique est lavée 3 fois à l'eau, puis séchée sur MgSO₄. On récupère 0,79 g du produit attendu après évaporation. Ce composé est dissout dans 5 mL d'éthanol en présence de 0,46 g de thiourée et le milieu est porté à reflux pendant 2 heures 30 minutes. Un solide précipite lors du retour à température ambiante. Le solide ainsi collecté est rincé avec un peu d'éthanol froid, puis à l'éther. On récupère ainsi 0,6 g du bromhydrate.

Le solide est mis en suspension dans un mélange eau/dichlorométhane et on effectue un retour à la base par addition de soude. La phase aqueuse est extraite 2 fois dans le dichlorométhane. Les phases organiques rassemblées sont séchées sur MgSO₄, Puis évaporées pour donner 0,34 g d'une huile jaune qui cristallise lentement. Les eaux mères sont évaporées, puis agitées dans un mélange eau/dichlorométhane et on effectue un retour à la base par addition de soude. La phase aqueuse est extraite 2 fois dans le dichlorométhane. Les phases organiques rassemblées sont séchées sur MgSO₄, puis évaporées. L'huile obtenue est chromatographiée sur gel de silice pour obtenir 0,18 g du composé attendu.
PF = 48°C.

### Préparation I.22

### 4-(5-Pentafluoroéthyl-2-méthoxy-phényl)-1,3-thiazol-2-amine

### A) 1-Méthoxy-4-pentafluoroéthyl-benzène

Sous atmosphère inerte, dans un tricol de 500 mL équipé d'un Dean-Starck et d'un réfrigérant, on introduit 8,3 g de pentafluoropropionate de potassium et 9,8 g de Cul. On ajoute 90 mL de DMF et 110 mL de toluène. Le milieu est chauffé à 140°C sous azote, 80 mL de toluène sont distillés. Le milieu est alors refroidi à TA puis dé-oxygéné avec un bullage à l'azote. On ajoute ensuite 6 g de iodoanisole puis on chauffe à 155°C pendant 20 h. Après retour à TA, le milieu est dilué dans 200 mL d'un mélange eau / éther éthylique. Le milieu est alors filtré sur Célite^{®}. La phase organique est lavée 3 fois à l'eau, séchée sur MgSO₄ puis évaporée pour donner 4,3 g d'une huile marron.

### B) 1-(2-Méthoxy-5-pentafluoroéthyl-phényl)-éthanone

A une solution de 3,5 g de 1-Méthoxy-4-pentafluoroéthyl-benzène dans 50 mL de THF anhydre, on additionne, à -70°C, 7,4 mL de BuLi à 2,5 M dans l'hexane. Le milieu est agité 30 min à -70°C puis 45 min à 0°C. On additionne alors 15,5 mL d'une solution de chlorure de zinc 1 M dans l'éther. Après 10 min d'agitation à 0°C, 1,33 mL de chlorure d'acétyle est ajouté. Le milieu est alors dé-oxygéné à l'azote et 332 mg de palladium benzyl(chloro)-bis(triphénylphosphine) dans 5 mL de THF anhydre sont introduits. Le milieu est agité 2 h 30 à 0°C puis 72 heures à TA. Le milieu est coulé sur une solution d'HCl 2,5 M puis extrait dans l'éther. La phase organique est lavée au NaHCO₃ à 5 % dans l'eau, à l'eau puis avec une solution saturée de NaCl. Après séchage sur MgSO₄ et évaporation, le brut est purifié par flash-chromatographie sur silice pour donner 2,25 g d'un solide blanc. PF = 47°C.

### C) 4-(5-Pentafluoroéthyl-2-méthoxy-phényl)-1,3-thiazol-2-amine

A une solution de 2,25 g du produit obtenu à l'étape précédente dans 10 mL de méthanol, on additionne 0,5 mL de brome en solution dans 8 mL de méthanol. Le milieu est agité pendant 10 min puis évaporé et repris dans le dichlorométhane. La phase organique est lavée 3 fois à l'eau puis séchée sur MgSO₄. On récupère 2,63 g du produit bromé après évaporation. Ce composé est dissout dans 15 mL de méthanol en présence de 1,25 g de thiourée et le milieu est porté à reflux pendant 2 h. Un solide précipite lors du retour à TA. Le solide ainsi collecté est rincé à l'éther éthylique. Le solide est mis en suspension dans un mélange eau / dichlorométhane et on effectue un retour à la base par addition de soude. La phase aqueuse est extraite 2 fois dans le dichlorométhane. Les phases organiques rassemblées sont séchées sur MgSO₄ puis évaporées pour donner 1,63 g d'un solide jaune.
PF = 125°C

### Préparation I.3

### 4-(5-Cyclohexyl-2-méthoxyphényl)-1,3-thiazol-2-amine

A) A une solution de 5 g de 4-cyclohéxylphénol dans 60 mL de DMF, on ajoute 7,84 g de K₂CO₃, puis 3,53 mL d'iodure de méthyle. Le milieu est chauffé à 60°C pendant une nuit. Après retour à température ambiante, le milieu est filtré sur Célite^{®}, puis dilué dans l'éther et hydrolysé à l'eau. La phase aqueuse est acidifiée, puis extraite dans 3 fois 50 mL d'éther. Les phases organiques rassemblées sont lavées avec une solution de soude diluée, puis lavées à l'eau 2 fois et avec une solution saturée de NaCl. La phase organique est séchée sur MgSO₄, puis évaporée pour donner 4,31 g du composé attendu sous forme d'un solide.
PF = 67°C.

### B) 1-(5-Cyclohexyl-2-méthoxyphényl)-éthanone

Une suspension de 5,6 g d'AlCl₃ dans 40 mL de dichlorométhane est refroidie à -10°C. On ajoute 3 mL d'AcCl et 4 g du composé obtenu à l'étape précédente. Le milieu est agité 1 heure à -10°C, puis versé dans un bécher contenant de la glace mélangée à de l'HCl à 35%. Après décantation, les phases organiques rassemblées sont séchées sur MgSO₄, puis évaporées pour donner 4,54 g du produit attendu.

### C) 4-(5-Cyclohexyl-2-méthoxyphényl)-1,3-thiazol-2-amine

A une solution de 4,5 g du produit de l'étape précédente dans 25 mL de méthanol, on ajoute, au goutte-à-goutte 1,16 mL de brome. Le milieu est agité 30 minutes à température ambiante, puis devient très visqueux. 5 mL de méthanol sont rajoutés, suivis de 3,23 g de thiourée. Le milieu est chauffé à reflux pendant 2 heures. Après retour à température ambiante, un solide précipite. Le solide est collecté, puis rincé avec un peu de méthanol froid. Le solide est mis en suspension dans un mélange eau/dichlorométhane et on effectue un retour à la base par addition de soude. La phase aqueuse est extraite 2 fois dans le dichlorométhane. Les phases organiques rassemblées sont séchées sur MgSO₄, puis évaporées pour donner 3,33 g du composé attendu sous forme d'un solide. PF = 113°C.

### Préparation I.4

### 4-(2-Méthoxy-5-propyl-phényl)-1,3-thiazol-2-amine

### A) 1-(2-Méthoxy-5-propyl-phényl)-éthanone

Une suspension de 10,6 g d'AlCl₃ dans 150 mL de dichlorométhane est refroidie à -10°C. On ajoute 5,7 mL d'AcCl et 6 g de 4-propyl anisole. Le milieu est agité 30 min à -10°C puis versé dans un bécher contenant de la glace mélangée à de l'HCl à 35%. Après décantation, la phase aqueuse est extraite 3 fois dans le DCM, les phases organiques rassemblées sont lavées à l'eau, avec une solution saturée de NaCl, séchées sur MgSO₄ puis évaporées pour donner 7,86 g d'une huile brune.

### B) 2-Bromo-1-(2-méthoxy-5-propyl-phényl)-éthanone

A une solution de 7,86 g du composé obtenu à l'étape précédente dans 80 mL de méthanol, on ajoute au goutte-à-goutte 2,46 mL de brome dilué dans 40 mL de méthanol. Le milieu est agité 30 min à TA puis évaporé. L'huile obtenue est reprise dans le dichlorométhane, lavée 3 fois dans l'eau puis séchée sur MgSO₄ et évaporée pour donner 11,25 g d'une huile jaune.

### C) 4-(2-Méthoxy-5-propyl-phényl)-1,3-thiazol-2-amine

A une solution de 8 g du composé obtenu à l'étape précédente dans 60 mL d'éthanol sont ajoutés 4,94 g de thiourée. Le milieu est porté au reflux pendant 1 h30 min. Le milieu est alors placé au réfrigérateur pendant 12 h puis filtré. Le solide ainsi collecté est rincé avec un peu d'éthanol froid puis à l'éther. La procédure est répétée une deuxième fois. Le solide est mis en suspension dans un mélange eau/dichlorométhane et on effectue un retour à la base par addition de soude. La phase aqueuse est extraite 2 fois dans le dichlorométhane. Les phases organiques rassemblées sont séchées sur MgSO₄ puis évaporées pour donner 4,89 g d'une huile brune qui cristallise lentement (67%).

Les eaux mères sont évaporées puis agitées dans un mélange eau/dichlorométhane et on effectue un retour à la base par addition de soude. La phase aqueuse est extraite 2 fois dans le dichlorométhane. Les phases organiques rassemblées sont séchées sur MgSO₄ puis évaporées. L'huile obtenue est chromatographiée sur gel de silice pour donner 580 mg du produit attendu.
Rdt (total) : 75%
PF = 84°C

### Préparation I.26

### 4-(5-Cyclohexyl-2-méthoxy-phényl)-5-fluoro-thiazol-2-ylamine

A une solution de 2,5 g du composé obtenu à la préparation I.3 décrite ci-dessus dans 30 mL de DMF, on ajoute à 0°C, 3,4 g de Selectfluor^{©} et le milieu est agité pendant 2 h à TA. Le milieu est hydrolysé par de l'ammoniac 2M dans l'éthanol, concentré puis dilué dans l'eau. Le brut est filtré, le solide est repris dans le DCM, lavé à l'eau puis à la soude 1 M et avec une solution saturée de NaCl. Après séchage de la phase organique sur MgSO₄ et évaporation, le brut est purifié par flash-chromatographie.
On obtient 600 mg du composé attendu sous la forme d'une poudre blanche.
PF = 159°C

### Préparation I.27

### 4-(5-Propyl-2-méthoxy-phényl)-5-fluoro-thiazol-2-ylamine

Le composé est préparé selon la préparation I.26 à partir du composé de la préparation I.4.
PF = 107°C

En opérant selon les modes opératoires ci-dessus, on prépare les composés de formule (II) décrits dans le tableau I ci-après.

Dans le tableau qui suit : Me = méthyle, Et = éthyle, Pr = propyle, But = butyle, Hex = hexyle

**Tableau I**

| | | | | | |
|---|---|---|---|---|---|
| 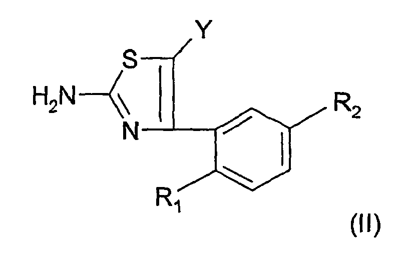 | | | | | |

| **Préparations n°** | **R₁** | **R₂** | **Y** | **Sel** | **PF (°C) MH +** |
|---|---|---|---|---|---|
| I.1 | -OMe | -OPr | H | - | PF = 76 |
| I.2 | -OMe | -nBu | H | - | PF = 48 |
| I.2 bis | -OMe | -nBu | H | HBr | PF = 186 |
| I.3 | -OMe | 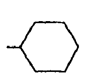 | H | - | PF = 113 |
| I.4 | -OMe | -nPr | H | - | PF = 84 |
| I.5 | -OEt | -Et | H | - | PF = 83 |
| I.6 | -OMe | -Et | H | - | PF = 100 |
| I.7 | -OEt | 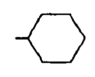 | H | - | PF = 110 |
| I.8 | -OMe | 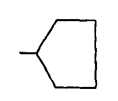 | H | - | PF = 110 |
| I.9 | -OEt | -nBu | H | - | PF = 65 |
| I.10 | -OMe | -CF3 | H | - | PF = 144 |
| I.11 | -OMe | -iPr | H | - | PF = 109 |
| I.12 | -OMe | -Me | H | - | PF = 121 |
| I.13 | 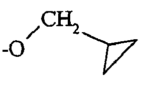 | -nBu | H | - | PF = 59 |
| I.14 | -OMe | 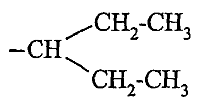 | H | - | PF=91-93 |
| I.16 | -Cl | -CF₃ | H | - | PF = 110 |
| I.17 | -OEt | -Me | H | - | PF = 124 |
| I.18 | -OMe | -CH(nPr)₂ | H | HCl | MH⁺ = 305,4 tr = 7,61 |
| I.19 | -OnPr | -nBu | H | - | PF = 63 |
| I.20 | -OMe | -nHex | H | - | PF = 43 |
| I.21 | -OEt | -nHex | H | - | PF = 75 |
| I.22 | -OMe | -CF₃CF₂ | H | - | PF = 125 |
| I.23 | -OEt | -CF₃CF₂ | H | - | MH⁺ = 338 tr = 7,88 |
| I.24 | -OEt | -nPr | H | - | PF = 87 |
| I.25 | -OEt | cyclopentyle | H | - | PF = 128 |
| I .26 | -OMe | 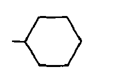 | F | - | PF = 159 |
| I.27 | -OMe | -Pr | F | - | PF = 107 |

Les étapes 1.1 et 1.2 de l'exemple 1 décrit dans ce qui suit pour la préparation du composé n° 120 illustrent la préparation des composés de formule (IV) et (V).

En répétant les modes opératoires décrits dans les étapes 1.1 et 1.2 de l'exemple 1, les composés de formule (V') du tableau II ci-après peuvent être préparés.

**Tableau II**

| | | | | | | | |
|---|---|---|---|---|---|---|---|
| 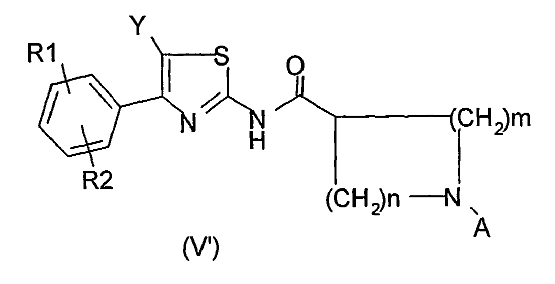 | | | | | | | |

| **Composé** | **R₁** | **R₂** | **Y** | **n** | **m** | **A** | PF (°C), **MH**⁺,**t et/ou RMN** |
|---|---|---|---|---|---|---|---|
| II.1 | 2-OCH₃ | 5-CyHex | H | 0 | 3 | 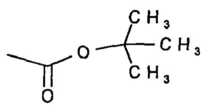 | RMN5 |
| II.2 | 2-OCH₃ | 5-CyHex | H | 0 | 3 | H | RMN6 |
| II.3 | 2-OCH₃ | 5-CyHex | H | 2 | 2 | H | MH+ = 400 tr = 7,25 |
| II.4(S) | 2-OCH₃ | 5-CyHex | H | 1 | 3 | 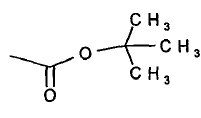 | PF = 110 |
| II.5 | 2-OCH₃ | 5-CyHex | H | 1 | 3 | 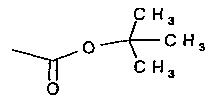 | PF = 107 |
| II.6(R) | 2-OCH₃ | 5-CyHex | H | 1 | 3 | 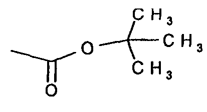 | PF = 73 |
| II.7(R) | 2-OCH₃ | 5-CyHex | H | 1 | 3 | H | PF = 210 |
| II.8(S) | 2-OCH₃ | 5-CyHex | H | 1 | 3 | H | PF = 202 |
| II.9 | 2-OCH₃ | 5-CyHex | H | 1 | 3 | H | PF = 236 |
| II.10 | 2-OCH₃ | 5-CyHex | H | 0 | 4 | 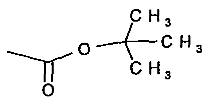 | PF = 110 |
| II.11 | 2-OCH₃ | 5-CyHex | H | 0 | 4 | H | PF = 164 |
| II.12 | 2-OCH₃ | 5-(CH₂)₃CH₃ | H | 1 | 3 | 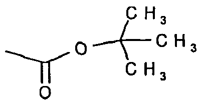 | PF = 88 |
| II.13 | 2-OCH₃ | 5-CyPent | H | 1 | 3 | 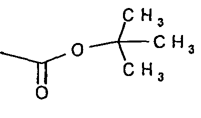 | PF = 107 RMN1 |
| II.14 | 2-OCH₂CH₃ | 5-(CH₂)₃CH₃ | H | 1 | 3 | 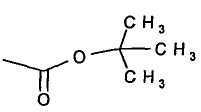 | PF = 97 |
| II.15 | 2-OCH₃ | 5-CyPent | H | 1 | 3 | H | MH+ = 386 tr = 7,02 |
| II.16 | 2-OCH₃ | 5-(CH₂)₃CH₃ | H | 1 | 3 | H | MH+ = 374 tr = 7,01 |
| II.17 | 2-OCH₂CH₃ | 5-(CH₂)₃CH₃ | H | 1 | 3 | H | PF=199 |
| II.18 | 2-OCH₃ | 5-(CH₂)₂CH₃ | H | 1 | 3 | 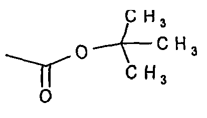 | RMN2 |
| II.19(R) | 2-OCH₃ | 5-(CH₂)₂CH₃ | H | 1 | 3 | H | αD = - 1,0(1 = 1,66, MeOH), e.e = 99,9 % tr = 12,66 |
| II.20(S) | 2-OCH₃ | 5-(CH₂)₂CH₃ | H | 1 | 3 | H | αD = + 0,78(1 =1,66, MeOH), e.e = 99,6 % tr = 9,45 |
| II.21 | 2-OCH₃ | 5-(CH₂)₂CH₃ | H | 1 | 3 | H | RMN3 |
| II.22 | 2-OCH₃ | 5-O(CH₂)₂CH₃ | H | 1 | 3 | 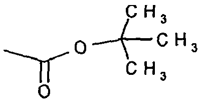 | RMN4 |
| II.23 (R) | 2-OCH₃ | 5-O(CH₂)₂CH₃ | H | 1 | 3 | H | PF = 210 |
| II.24(S) | 2-OCH₃ | 5-O(CH₂)₂CH₃ | H | 1 | 3 | H | PF = 208 |
| II.25 | 2-OCH₃ | 5-cyHex | H | 1 | 2 | 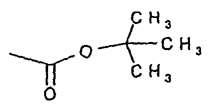 | PF = 188 |
| II.26 | 2-OCH₃ | 5-cyHex | H | 1 | 2 | H | PF = 181 |
| II.27 | 2-OCH₃ | 5-cyHex | H | 2 | 2 | 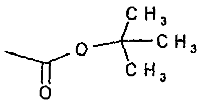 | PF = 193 |

| | | | | | | | |
|---|---|---|---|---|---|---|---|
| RMN1 δ (ppm) = 1.21 (s, 9H), 1.23-2.19 (m, 12H), 2.73 (m, 1H), 2.84-3.15 (m, 2H), 3.77-4.12 (m, 2H), 3.98 (s, 3H), 7.05 (d, 1H), 7.22 (dd, 1H), 7.64 (s, 1H), 8.01 (d, 1H), 12.21 (sél, 1H) RMN2 Voir exemple 2.1 RMN3 δ (ppm) = 0.81 (t, 3H), 1.21-1.90 (m, 6H), 2.38-3.02 (m, 7H), 3.81 (s, 3H), 6.99 (d, 1H), 7.04 (dd, 1H), 7.57 (s, 1H), 7.84 (d, 1H), 12.20 (sél, 1H) RMN4 δ (ppm) = 0.84 (t, 3H), 1.22 (s, 9H), 1.58-1.78 (m, 4H), 1.81-1.90 (m, 2H), 2.58 (m, 1H), 2.80 (m, 1H), 3.0-3.15 (m, 1H), 3.59-3.97 (m, 4H), 3.92 (s, 3H), 6.79 (dd, 1H), 6.98 (d, 1H), 7.58 (d, 1H), 7.60 (s, 1H), 12.17 (sél, 1H) RMN5 δ (ppm) = 1.17 (s, 9H), 1.20-2.21 (m, 13H), 2.16 (m, 1H), 2.39 (m, 1H), 3.18-3.40 (m, 2H), 3.89 (s, 3H), 4.26 (m, 1H), 6.92 (d, 1H), 7.04 (dd, 1H), 7.57 (s, 1H), 7.84 (d, 1H), 12.21 (sél, 1H) RMN6 δ (ppm) = 1.20-1.28 (m, 1H), 1.30-1.49 (m, 4H), 1.66-1.75 (m, 3H), 1.76-1.85 (m, 5H), 2.01-2.17 (m, 1H), 2.47-2.51 (m, 1H), 2.80-2.97 (m, 2H), 3.84 (m, 1H), 3.90 (s, 3H), 6.99 (d, 1H), 7.15 (dd, 1H), 7.62 (s, 1H), 7.94 (d, 1H) | | | | | | | |

Les exemples qui suivent illustrent l'invention sans la limiter.

### Exemple 1 : (composé n° 120) Acide 1-(pyrazine-2-carbonyl)-pipéridine-4-carboxylique-[4-(5-cyclohexyl-2-méthoxy-phényl)-thiazol-2-yl]-amide

Composé de formule générale (I) dans laquelle:
R₁=2-OMe; R₂ = 5-CyHex; m=2; n=2; p=0;
R₃=

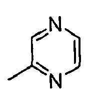

### 1.1. Préparation de l'ester tert-butylique de l'acide 4-[4-(5-cyclohexyl-2-méthoxyphényl)-thiazol-2-ylcarbamoyl]-pipéridine-1-carboxylique (composé II.27)

A une température de 0 °C, on ajoute 1,68 g de BOP, 0,75 g d'acide Bocisonipécotique, puis 1,41 mL de DIPEA, à une solution de 1 g de 4-(2-méthoxy-5-cyclohexyl-phényl)-1,3-thiazol-2-amine, obtenue à la préparation I.3, dans 6 mL d'acétonitrile. On agite le milieu à température ambiante pendant 24 heures puis on filtre. On rince le solide ainsi obtenu à l'acétonitrile, puis on le sèche. On obtient alors 1,13g d'un solide blanc.
PF = 193°C

### 1.2. Préparation de l'acide pipéridine-4-carboxylique-[4-(5-cyclohexyl-2-méthoxyphényl)-thiazal-2-yl]-amide (composé II.3)

On agite une solution de 1,13 g du composé préparé à l'étape 1.1 dans 10 mL d'une solution de HCl 4 M dans le dioxane, pendant 30 minutes à température ambiante. On dilue le milieu dans l'éther éthylique puis on filtre. On rince le solide ainsi obtenu à l'éther puis on le sèche pour donner 1,01 g d'un solide blanc.
MH+ = 400 à tr = 7,25 min.

### 1.3. Préparation de l'acide 1-(pyrazine-2-carbonyl)-pipéridine-4-carboxylique-[4-(5-cyclohexyl-2-méthoxy-phényl)-thiazol-2-yl]-amide

A une température de 0 °C, on ajoute 0,32 g de BOP, 0,08 g d'acide pyrazine carboxylique, puis 0,37 mL de DIPEA, à une solution de 0,25 g du composé préparé à l'étape 1.2, dans 3 mL d'acétonitrile. On agite le milieu à température ambiante pendant 12 heures puis on le concentre. On reprend le milieu dans l'AcOEt, on le lave 3 fois par une solution de Na₂CO₃ à 10%, puis par une solution saturée de NaCl. Après séchage sur MgSO₄, la solution est concentrée puis purifiée par flash-chromatographie pour donner 0,23 g d'un solide jaune foncé.
PF = 129°C

### Exemple 2: (composé n° 93) Chlorhydrate de l'acide (S)-1-(pyridine-2-carbonyl)-pipéridine-3-carboxylique-[4-(2-méthoxy-5-propyl-phényl)-thiazol-2-yl]-amide

Composé de formule générale (I) dans laquelle:
R₁=2-OMe;R₂=5-n-propyl;m=3;n=1;p=0;
R₃=

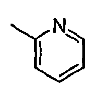

### 2.1. Préparation de l'ester tert-butylique de l'acide (S)-3-[4-(2-méthoxy-5-propylphényl)-thiazol-2-ylcarbamoyl]-pipéridine-1-carboxylique (énantiomère (S) du composé II.18)

A une température de 0 °C, on ajoute 0,5 g de TBTU, 0,1 g de HOBT, 0,35 g d'acide (S)-N-Boc-nipécotique, puis 0,3 mL de DIPEA, à une solution de 0,5 g de 4-(2-méthoxy-5-propyl-phényl)-1,3-thiazol-2-amine, obtenue à la préparation I.4, dans 4 mL de DMF. On agite le milieu à température ambiante pendant 3 heures puis on dilue dans l'AcOEt et on lave 4 fois par une solution de Na₂CO₃ à 10%, puis par une solution saturée de NaCl. Après séchage sur MgSO₄, la solution est concentrée puis purifiée par flash-chromatographie pour donner 0,56 g d'un solide blanc.
RMN δ (ppm) = 0,77 (t, 3H) ; 1.22 (s, 9H) ; 1,24-1,88 (m, 7H) ; 2,43 (m, 2H) ; 2,55 (m, 2H) ; 2,79 (m, 2H) ; 3,6-3,75 (m, 2H) ; 3,78 (s, 3H) ; 6,92 (d, 1H) ; 7,04 (d.d, 1H) ; 7,54 (s, 1H) ; 7,82 (d, 1H) ; 12.19 (sél, 1H).

### 2.2. Préparation de l'acide (S)-pipéridine-3-carboxylique-[4-(2-méthoxy-5-propylphényl)-thiazol-2-yl]-amide (Composé II.20)

A une température de 0 °C, on ajoute 1 mL de TFA à une solution de 0,56 g du composé préparé à l'étape 2.1, dans 3 mL de DCM. On agite le milieu pendant 2 heures à température ambiante. Le milieu est concentré puis repris dans 20 mL de DCM et lavé 3 fois par une solution de Na₂CO₃ à 10% puis par une solution saturée de NaCl. La phase organique est évaporée pour obtenir 0,36 g d'un solide blanc.
α_{D} = + 0,78 (c = 1,66, dans un mélange MeOH/CHCl₃(1/1))

### 2.3. Préparation du chlorhydrate de l'acide (S)-1-(Pyridine-2-carbonyl)-piperidine-3-carboxylique-[4-(2-méthoxy-5-propyl-phényl)-thiazol-2-yl-amide

A une température de 0 °C, on ajoute 0,071 g de chlorure de picolinoyle, puis 0,12 mL de DIPEA, à une solution de 0,12 g du composé préparé à l'étape 2.2, dans 1 mL d'acétonitrile. Le milieu est agité jusqu'à retour à température ambiante. Après 1 heure à température ambiante, le milieu est hydrolysé puis dilué dans l'AcOEt et lavé par une solution de Na₂CO₃ à 10% puis par une solution saturée de NaCl. Après séchage sur MgSO₄, la solution est concentrée. Le brut obtenu est repris dans le DCM. Le chlorhydrate est formé par addition d'éther éthylique chlorhydrique 2 M. Le solide obtenu est filtré et rincé à l'éther éthylique. On obtient la base libre par traitement à la soude 1 M puis on l'extrait dans le DCM, on sèche la phase organique sur MgSO₄ puis on évapore. On reprend la base libre dans le DCM. On obtient le chlorhydrate par addition d'éther éthylique chlorhydrique 2 M. Le solide obtenu est filtré, rincé à l'éther éthylique puis séché pour obtenir 91 mg d'un solide blanc.
PF = 162°C

### Exemple 3: (composé n° 105) Acide (R,S)-1-(1-oxy-pyridine-2-carbonyl)-pyrrolidine-3-carboxylique-[4-(5-cyclohexyl-2-méthoxy-phényl)-thiazol-2-yl]-amide

Composé de formule générale (I) dans laquelle:
R₁ =2-OMe; R₂=5-CyHex; m=2; n =1; P=0;
R₃ =

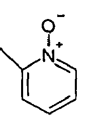

### 3.1. Préparation de l'ester tert-butylique de l'acide (R, S)-3-[4-(5-cyclohexyl-2-méthoxy-phényl)-thiazol-2-ylcarbamoyl]-pyrrolidine-1-carboxylique (composé II.25)

A une température de 0 °C, on ajoute 2,1 g de BOP, 0,9 g d'acide 3-Boc-pyrrolidine carboxylique puis 1,2 mL de DIPEA, à une solution de 1 g de 4-(2-méthoxy-5-cyclohexyl-phényl)-1,3-thiazol-2-amine, obtenue à la préparation I.3, dans 6 mL d'acétonitrile. On agite le milieu à température ambiante pendant 12 heures puis on filtre. On rince le solide ainsi obtenu à l'acétonitrile puis on le sèche pour obtenir 1,37 g d'un solide blanc.
PF = 188°C

### 3.2_{.} Préparation de l'acide (R,S)-pyrrolidine-3-carboxylique-[4-(5-cyclohexyl-2-méthoxy-phényl)-thiazol-2-yl]-amide (composé II.26)

On agite une solution de 1,35 g du composé préparé à l'étape 3.1, dans 40 mL d'une solution de HCl 4 M dans le dioxane, pendant 2 heures à température ambiante. On dilue le milieu dans l'éther éthylique puis on filtre. On rince le solide ainsi obtenu à l'éther puis on le reprend dans un mélange DCM/solution de Na₂CO₃ saturée (1/1). On extrait la phase aqueuse dans le DCM, on la lave à l'eau puis on la sèche sur MgSO₄ et on évapore pour obtenir 1,02 g d'un solide blanc.
PF = 181°C

### 3.3. Préparation de l'acide (R,S)-1-(1-oxy-pyridine-2-carbonyl)-pyrrolidine-3-carboxylique-[4-(5-cyclohexyl-2-méthoxy-phényl)-thiazol-2-yl]-amide

A une température de 0°C, on ajoute 0,108 g d'acide picolinique N-oxyde, 0,4 g de BOP, puis 0,23 mL de DIPEA, à une solution de 0,25 g du composé préparé à l'étape 3.2, dans 3 mL d'acétonitrile. Le milieu est agité 12 heures à température ambiante, puis filtré. Le solide collecté est rincé à l'acétonitrile puis purifié par flash-chromatographie pour donner 0,23 g du composé attendu.
PF = 161°C

En suivant ces procédures à partir de l'acide (R)-3-Boc-pyrrolidine carboxylique on obtient le **composé n° 239** : PF = 155°C, □_{D} = -32 (c = 1.6, HCCl₃), pureté optique = 96.4% (HPLC sur chiralpakAD-H (250mmx4.6), technique : SFC, phase mobile : CO2/isopropanol+0.5% IPA 70/30 2ml/mn, 200bar, 30°C, détection UV à 254 nm à tr = 12.56 mn).

En suivant ces procédures à partir de l'acide (S)- 3-Boc-pyrrolidine carboxylique, on obtient le **composé n° 240** (PF = 157°C,□_{D} = +30 (c = 1.6, HCCl₃), pureté optique = 97.6% (HPLC sur chiralpakAD-H (250mmx4.6), technique : SFC, phase mobile : CO2/isopropanol+0.5% IPA 70/30 2ml/mn, 200bar, 30°C, détection UV à 254 nm à tr = 13.99 mn).

### Exemple 4 : (composé n° 99) Acide (R,S)-1-(pyrazine-2-carbonyl)-pipéridine-3-carboxylique-[4-(5-butyl-2-éthoxy-phényl)-thiazol-2-yl]-amide

Composé de formule générale (I) dans laquelle:
R₁ = 2-éthoxy ; R₂ = 5-butyl ; m = 3 ; n = 1; p = 0 ;
R₃ =

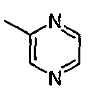

### 4.1. Préparation de l'acide (R,S)-1-(pyrazine-2-carbonyl)-pipéridine-3-carboxylique

On ajoute 7,7 mL de BSA à une solution de 2,1 g d'acide nipécotique dans 50 mL de DCM. On laisse 1 heure à température ambiante. On ajoute ensuite, à une température de 0°C, 2,1 g de chlorure d'acide pyrazine-carboxylique, obtenu par traitement de l'acide pyrazine-carboxylique avec du SOCl₂ (Advanced in Organic Chemistry, J. March, 3nd Edition, Wiley Intersciences, p 523 et réf. citées). On agite le milieu à température ambiante pendant 12 heures, puis on le concentre. On reprend le brut dans un mélange eau/méthanol, puis on dilue dans l'éther éthylique. Après trituration, on collecte un solide par filtration. On le reprend dans le DCM. Après filtration, on évapore le filtrat. On reprend le solide ainsi obtenu dans le DCM. Après filtration, on évapore le filtrat pour obtenir 2,1 g d'un solide blanc.
MH+ = 234 à tr = 3,91 min

### 4.2. Préparation de l'acide (R,S)-1-(pyrazine-2-carbonyl)-pipéridine-3-carboxylique-[4-(5-butyl-2-éthoxy-phényl)-thiazol-2-yl]-amide

A une température de 0 °C, on ajoute 0,3 g de 4-(2-éthoxy-5-butyl-phényl)-1,3-thiazol-2-amine, obtenue à la préparation I.9, 0,67 g de BOP, puis 0,76 mL de DIPEA, à une solution de 0,40 g du composé préparé à l'étape 4.1, dans 3 mL d'acétonitrile. On agite le milieu à température ambiante pendant 12 heures, puis on le filtre. On rince le solide ainsi obtenu à l'acétonitrile, puis on le sèche pour obtenir 0,34 g d'un solide blanc.
PF = 182°C

Le tableau III ci-après illustre les structures chimiques et les propriétés physiques de quelques exemples de composés selon l'invention. Dans ce tableau :
- PF = Point de Fusion, en degré Celsius (°C),
- MH+ = spectrométrie de masse,
- tr = temps de rétention, en minute (min.),
- CyHex représente un groupe cyclohexyle, CyPent un groupe cyclopentyle,
- dans la colonne « sels o, « - » représente un composé sous forme de base libre, alors que « HCl » représente un composé sous sa forme de chlorhydrate,
- (+) indique que le composé est un énantiomère dextrogyre, (-) un énantiomère lévogyre,
- (R) indique que le composé est un énantiomère R, - (S) un énantiomère S.

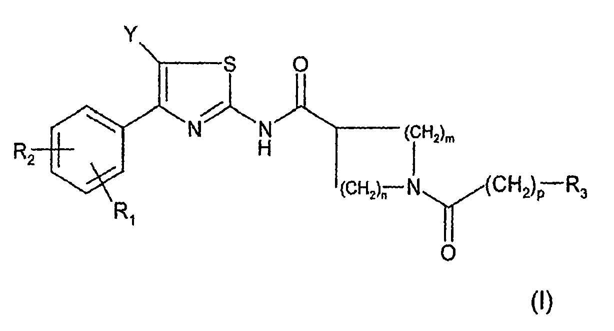

**Tableau III**

| **N°** | **R₁** | **R₂** | **R₃** | **Y** | **p** | **n** | **m** | **sel** | **PF et/ou MH+, tr** |
|---|---|---|---|---|---|---|---|---|---|
| 1 | 2-OCH₃ | 5-O(CH₂)₂CH₃ | 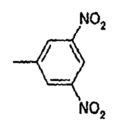 | H | 0 | 1 | 3 | - | MH+ = 570, tr = 10,37 |
| 2 | 2-OCH₃ | 5-O(CH₂)₂CH₃ | 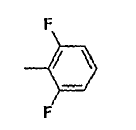 | H | 0 | 1 | 3 | - | MH+ = 516, tr = 10,31 |
| 3 (S) | 2-OCH₃ | 5-CyHex | 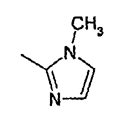 | H | 0 | 1 | 3 | - | PF = 118 |
| 4 (S) | 2-OCH₃ | 5-CyHex | 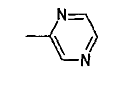 | H | 0 | 1 | 3 | - | MH+ = 506, tr = 10,21 |
| 5 | 2-OCH₃ | 5-O(CH₂)₂CH₃ | 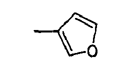 | H | 0 | 1 | 3 | - | MH+ = 470, tr = 9,64 |
| 6 | 2-OCH₃ | 5-O(CH₂)₂CH₃ | 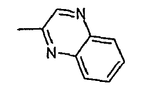 | H | 0 | 1 | 3 | - | MH+ = 532, tr = 9,97 |
| 7 (S) | 2-OCH₃ | 5-CyHex | 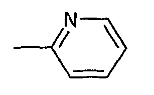 | H | 0 | 1 | 3 | - | MH+ = 505, tr = 10,61 PF = 142 |
| 8 | 2-OCH₃ | 5-O(CH₂)₂CH₃ | 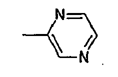 | H | 0 | 1 | 3 | - | MH+ = 482, tr = 9,05 |
| 9 | 2-OCH₃ | 5-O(CH₂)₂CH₃ | 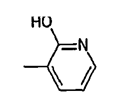 | H | 0 | 1 | 3 | - | MH+ = 497, tr = 8,26 |
| 10 | 2-OCH₃ | 5-O(CH₂)₂CH₃ | 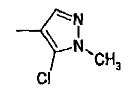 | H | 0 | 1 | 3 | - | MH+ = 518, tr = 9,39 |
| 11 | 2-OCH₃ | 5-(CH₂)₃CH₃ | 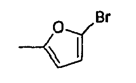 | H | 0 | 1 | 3 | - | MH+ = 546, tr= 11,13 |
| | 2-OCH₃ | 5-CyHex | 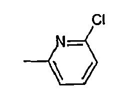 | H | 0 | 1 | 3 | - | MH+ = 539, tr = 11,24 |
| 13 | 2-OCH₃ | 5-CyHex | 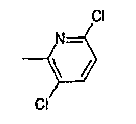 | H | 0 | 1 | 3 | - | MH+ = 573, tr = 11,49 |
| | 2-OCH₃ | 5-CyHex | 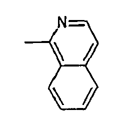 | H | 0 | 1 | 3 | - | MH+ = 555, tr= 11,13 |
| 15 | 2-OCH₃ | 5-CyHex | 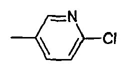 | H | 0 | 1 | 3 | - | MH+ = 538, tr= 11,05, |
| 16 | 2-OCH₃ | 5-CyHex | 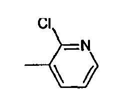 | H | 0 | 1 | 3 | - | MH+ = 539, tr = 10,84 |
| 17 | 2-OCH₃ | 5-CyHex | 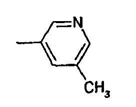 | H | 0 | 1 | 3 | - | MH+ = 519, tr = 9,96 |
| 18 | 2-OCH₃ | 5-CyHex | 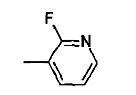 | H | 0 | 1 | 3 | - | MH+ = 523, tr = 10,81 |
| 19 | 2-OCH₃ | 5-CyHex | 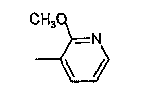 | H | 0 | 1 | 3 | - | MH+ = 535, tr = 10,92 |
| 20 | 2-OCH₃ | 5-CyHex | 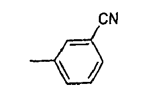 | H | 0 | 1 | 3 | - | MH+ = 529, tr = 11,08 |
| 21 | 2-OCH₃ | 5-CyHex | 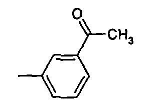 | H | 0 | 1 | 3 | - | MH+ = 546, tr = 10,99 |
| 22 | 2-OCH₃ | 5-CyHex | 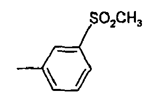 | H | 0 | 1 | 3 | - | MH+ = 582, tr= 10,61 |
| | 2-OCH₃ | 5-CyHex | 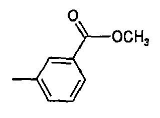 | H | 0 | 1 | 3 | - | MH+ = 562, tr = 11,28 |
| 24 | 2-OCH₃ | 5-CyHex | 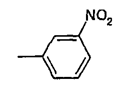 | H | 0 | 1 | 3 | - | MH+ = 549, tr = 11,29 |
| 25 | 2-OCH₃ | 5-CyHex | 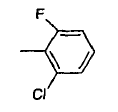 | H | 0 | 1 | 3 | - | MH+ = 556, tr = 11,74 |
| 26 | 2-OCH₃ | 5-CyHex | 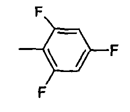 | H | 0 | 1 | 3 | - | MH+ = 558, tr = 11,63 |
| 27 | 2-OCH₃ | 5-CyHex | 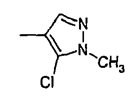 | H | 0 | 1 | 3 | - | MH+ = 543, tr = 10,67 |
| 28 | 2-OCH₃ | 5-CyHex | 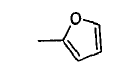 | H | 0 | 1 | 3 | - | MH+ = 494, tr = 11,00 |
| 29 | 2-OCH₃ | 5-CyHex | 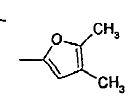 | H | 0 | 1 | 3 | - | MH+ = 522, tr = 11,61 |
| 30 | 2-OCH₃ | 5-CyHex | 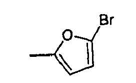 | H | 0 | 1 | 3 | - | MH+ = 572, tr = 11,57 |
| 31 | 2-OCH₃ | 5-CyHex | 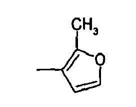 | H | 0 | 1 | 3 | - | MH+ = 508, tr = 11,20 |
| 32 | 2-OCH₃ | 5-CyHex | 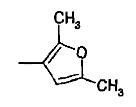 | H | 0 | 1 | 3 | - | MH+ = 522, tr = 11,57 |
| 33 | 2-OCH₃ | 5-CyHex | 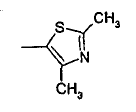 | H | 0 | 1 | 3 | - | MH+ = 539, tr = 10,70 |
| 34 | 2-OCH₃ | 5-O(CH₂)₂CH₃ | 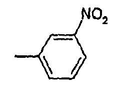 | H | 0 | 1 | 3 | - | MH+ = 525, tr = 10,12 |
| 35 | 2-OCH₃ | 5-(CH₂)₃CH₃ | 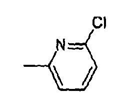 | H | 0 | 1 | 3 | - | MH+ = 513, tr =10,79 |
| 36 | 2-OCH₃ | 5-(CH₂)₃CH₃ | 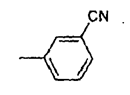 | H | 0 | 1 | 3 | - | MH+ = 503, tr = 10,64 |
| 37 | 2-OCH₃ | 5-(CH₂)₃CH₃ | 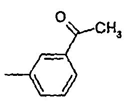 | H | 0 | 1 | 3 | - | MH+ = 520, tr = 10,55 |
| 38 | 2-OCH₃ | 5-(CH₂)₃CH₃ | 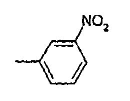 | H | 0 | 1 | 3 | - | MH+ = 523, tr = 10,86 |
| 39 | 2-OCH₃ | 5-(CH₂)₃CH₃ | 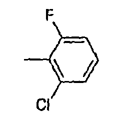 | H | 0 | 1 | 3 | - | MH+ = 530, tr = 11,32 |
| 40 | 2-OCH₃ | 5-(CH₂)₃CH₃ | 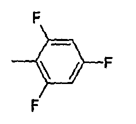 | H | 0 | 1 | 3 | - | MH+ = 532, tr=11,20 |
| 41 | 2-OCH₃ | 5-(CH₂)₃CH₃ | 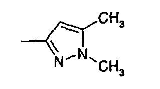 | H | 0 | 1 | 3 | - | MH+ = 496, tr = 10,28 |
| 42 | 2-OCH₃ | 5-(CH₂)₃CH₃ | 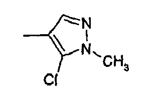 | H | 0 | 1 | 3 | - | MH+ = 516, tr = 10,20 |
| 43 | 2-OCH₃ | 5-(CH₂)₃CH₃ | 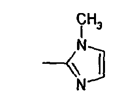 | H | 0 | 1 | 3 | - | MH+ = 482, tr = 9,24 |
| 44 | 2-OCH₃ | 5-(CH₂)₃CH₃ | 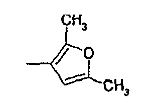 | H | 0 | 1 | 3 | - | MH+ = 496, tr= 11,13 |
| 45 | 2-OCH₃ | 5-(CH₂)₃CH₃ | 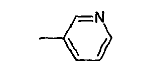 | H | 0 | 1 | 3 | - | MH+ = 479, tr = 9,49 |
| 46 | 2-OCH₃ | 5-(CH₂)₃CH₃ | 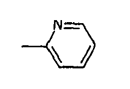 | H | 0 | 1 | 3 | - | MH+ = 479, tr = 10,12 |
| 47 | 2-OCH₃ | 5-(CH₂)₃CH₃ | 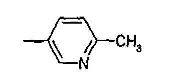 | H | 0 | 1 | 3 | - | MH+ = 493, tr = 9,12 |
| 48 | 2-OCH₃ | 5-(CH₂)₃CH₃ | 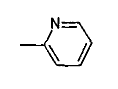 | H | 1 | 1 | 3 | - | MH+ = 493, tr = 8,50 |
| 49 | 2-OCH₃ | 5-(CH₂)₃CH₃ | 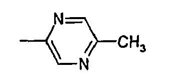 | H | 0 | 1 | 3 | - | MH+ = 494, tr = 10,09 |
| 50 | 2-OCH₃ | 5-CyHex | 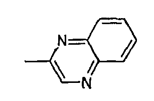 | H | 0 | 1 | 3 | - | MH+ = 556, tr = 11,23 |
| 51 | 2-OCH₃ | 5-CyHex | 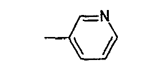 | H | 0 | 1 | 3 | - | MH+ = 505, tr = 9,91 |
| 52 | 2-OCH₃ | 5-CyHex | 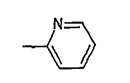 | H | 0 | 1 | 3 | - | MH+ = 505, tr = 10,62 |
| 53 | 2-OCH₃ | 5-CyHex | 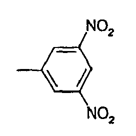 | H | 0 | 1 | 3 | - | MH+ = 594, tr = 11,46 |
| 54 | 2-OCH₃ | 5-CyHex | 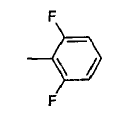 | H | 0 | 1 | 3 | - | MH+ = 540, tr = 11,49 |
| 55 | 2-OCH₃ | 5-CyHex | 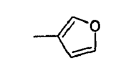 | H | 0 | 1 | 3 | - | MH+ = 494, tr = 10,92 |
| 56 | 2-OCH₃ | 5-CyHex | 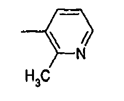 | H | 0 | 1 | 3 | - | MH+ = 519, tr = 9,28 |
| 57 | 2-OCH₃ | 5-CyHex | 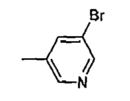 | H | 0 | 1 | 3 | - | MH+ = 584, tr = 11,06 |
| 58 | 2-OCH₃ | 5-CyHex | 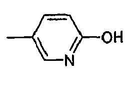 | H | 0 | 1 | 3 | - | MH+ = 521, tr = 9,44 |
| 59 | 2-OCH₃ | 5-CyHex | 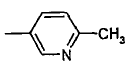 | H | 0 | 1 | 3 | - | MH+ = 519, tr = 9,51 |
| 60 | 2-OCH₃ | 5-CyHex | 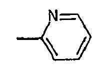 | H | 1 | 1 | 3 | - | MH+ = 519, tr = 8,90 |
| 61 | 2-OCH₃ | 5-CyHex | 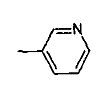 | H | 2 | 1 | 3 | - | MH+ = 533, tr = 8,16 |
| 62 | 2-OCH₃ | 5-CyHex | 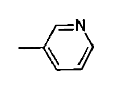 | H | 1 | 1 | 3 | - | MH+ = 519, tr = 8, 37 |
| 63 | 2-OCH₃ | 5-CyHex | 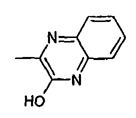 | H | 0 | 1 | 3 | - | MH+ = 572, tr = 10,34 |
| 64 | 2-OCH₃ | 5-CyHex | 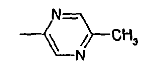 | H | 0 | 1 | 3 | - | MH+ = 520, tr = 10,56 |
| 65 | 2-OCH₃ | 5-CyHex | 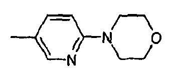 | H | 0 | 1 | 3 | - | MH+ = 590, tr = 10,20 |
| 66 | 2-OCH₂CH₃ | 5-CH₂CH₃ | 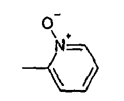 | H | 0 | 1 | 3 | - | MH+ = 481, tr = 8,66 |
| 67 | 2-OCH₂CH₃ | 5-CH₂CH₃ | 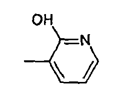 | H | 0 | 1 | 3 | - | MH+ = 480, tr = 8,59 |
| 68 | 2-OCH₂CH₃ | 5-CH₂CH₃ | 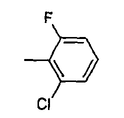 | H | 0 | 1 | 3 | - | MH+ = 516, tr = 10,90 |
| 69 | 2-OCH₂CH₃ | 5-CH₂CH₃ | 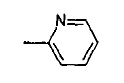 | H | 0 | 1 | 3 | - | MH+ = 465, tr = 9,64 |
| 70 | 2-OCH₂CH₃ | 5-CH₂CH₃ | 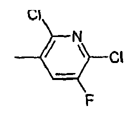 | H | 0 | 1 | 3 | - | MH+ = 551, tr = 11,02 |
| 71 | 2-OCH₂CH₃ | 5-CH₂CH₃ | 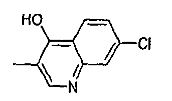 | H | 0 | 1 | 3 | - | MH+ = 566, tr = 9,56 |
| 72 | 2-OCH₂CH₃ | 5-CH₂CH₃ | 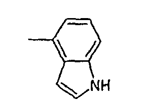 | H | 0 | 1 | 3 | - | MH+ = 503, tr= 10,12 |
| 73 | 2-OCH₃ | 5-O(CH₂)₂CH₃ | 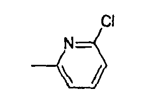 | H | 0 | 1 | 3 | - | MH+ = 515, tr = 9,99 |
| 74 | 2-OCH₃ | 5-(CH₂)₃CH₃ | 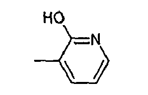 | H | 0 | 1 | 3 | - | MH+ = 495, tr = 9,03 |
| 75 | 2-OCH₃ | 5-CyHex | 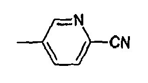 | H | 0 | 1 | 3 | - | MH+ = 530, tr = 10,8 |
| 76 | 2-OCH₃ | 5-CyHex | 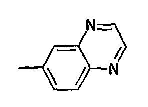 | H | 0 | 1 | 3 | - | MH+ = 556, tr = 10,49 |
| 77 | 2-OCH₃ | 5-O(CH₂)₂CH₃ | 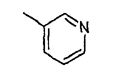 | H | 0 | 1 | 3 | - | PF = 192 |
| 78 (-) | 2-OCH₃ | 5-O(CH₂)₂CH₃ | 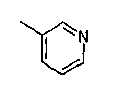 | H | 0 | 1 | 3 | - | PF = 104 |
| 79 (+) | 2-OCH₃ | 5-O(CH₂)₂CH₃ | 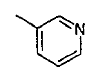 | H | 0 | 1 | 3 | - | PF=104 |
| 80 (-) | 2-OCH₃ | 5-O(CH₂)₂CH₃ | 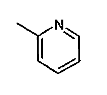 | H | 0 | 1 | 3 | - | PF = 90 MH+ = 482, tr = 8,61 |
| 81 (+) | 2-OCH₃ | 5-O(CH₂)₂CH₃ | 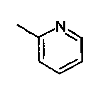 | H | 0 | 1 | 3 | - | PF = 90 MH+ = 482, tr = 8,63 |
| 82 | 2-OCH₃ | 5-O(CH₂)₂CH₃ | 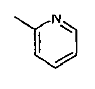 | H | 0 | 1 | 3 | - | PF = 52 |
| 83 (+) | 2-OCH₃ | 5-O(CH₂)₂CH₃ | 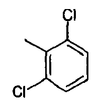 | H | 0 | 1 | 3 | - | PF = 241 |
| 84 | 2-OCH₃ | 5-O(CH₂)₂CH₃ | 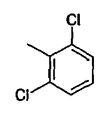 | H | 0 | 1 | 3 | - | PF = 240 |
| 85 (-) | 2-OCH₃ | 5-O(CH₂)₂CH₃ | 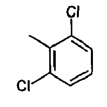 | H | 0 | 1 | 3 | - | PF = 241 |
| 86 | 2-OCH₃ | 5-(CH₂)₂CH₃ | 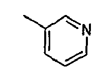 | H | 0 | 1 | 3 | - | PF=212 |
| 87 | 2-OCH₃ | 5-(CH₂)₂CH₃ | 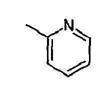 | H | 0 | 1 | 3 | - | PF = 104 |
| 88 (S) | 2-OCH₃ | 5-CyPent | 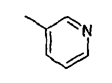 | H | 0 | 1 | 3 | HCl | PF = 176 |
| 89 (S) | 2-OCH₃ | 5-CyPent | 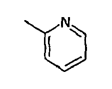 | H | 0 | 1 | 3 | HCl | PF=162 |
| 90 (R) | 2-OCH₃ | 5-CyPent | 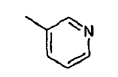 | H | 0 | 1 | 3 | HCl | PF = 178,9 |
| 91 (R) | 2-OCH₃ | 5-CyPent | 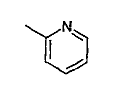 | H | 0 | 1 | 3 | HCl | PF = 161 |
| 92 (S) | 2-OCH₃ | 5-(CH₂)₂CH₃ | 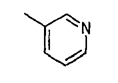 | H | 0 | 1 | 3 | HCl | PF = 170 |
| 93 (S) | 2-OCH₃ | 5-(CH₂)₂CH₃ | 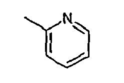 | H | 0 | 1 | 3 | HCl | PF = 162,4 |
| 94 (R) | 2-OCH₃ | 5-(CH₂)₂CH₃ | 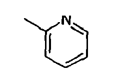 | H | 0 | 1 | 3 | HCl | PF = 156 |
| 95 | 2-OCH₃ | 5-CyPent | 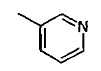 | H | 0 | 1 | 3 | HCl | MH+ = 492, tr = 8,15 |
| 96 | 2-OCH₃ | 5-CyPent | 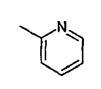 | H | 0 | 1 | 3 | HCl | PF = 166,7 |
| 97 | 2-OCH₃ | 5-CyHex | 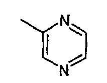 | H | 0 | 1 | 3 | - | PF = 206,2 |
| 98 | 2-OCH₂CH₃ | 5-CyHex | 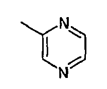 | H | 0 | 1 | 3 | - | PF = 186,1 |
| 99 | 2-OCH₂CH₃ | 5-(CH₂)₃CH₃ | 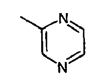 | H | 0 | 1 | 3 | - | PF = 182 |
| 100 | 2-OCH₃ | 5-CyPent | 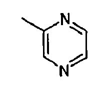 | H | 0 | 1 | 3 | - | PF = 192,8 |
| 101 | 2-OCH₃ | 5-CyHex | 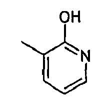 | H | 0 | 1 | 3 | - | PF = 179,4 |
| 102 | 2-OCH₃ | 5-CyHex | 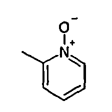 | H | 0 | 1 | 3 | - | PF = 188,3 |
| 103 | 2-OCH₃ | 5-(CH₂)₃CH₃ | 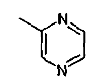 | H | 0 | 1 | 3 | - | PF = 106 |
| 104 | 2-OCH₃ | 5-CyHex | 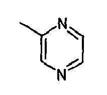 | H | 0 | 1 | 2 | - | PF = 115 |
| 105 | 2-OCH₃ | 5-CyHex | 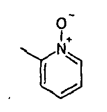 | H | 0 | 1 | 2 | - | PF = 161 |
| 106 (R) | 2-OCH₃ | 5-CyHex | 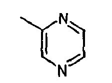 | H | 0 | 1 | 3 | - | PF = 119,8 |
| 107 | 2-OCH₃ | 5-CyHex | 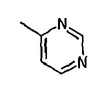 | H | 0 | 1 | 3 | - | PF = 115,8 |
| 108 (S) | 2-OCH₃ | 5-CyHex | 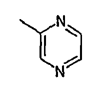 | H | 0 | 1 | 3 | - | PF = 115 |
| 109 | 2-OCH₃ | 5-CyHex | 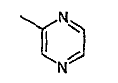 | H | 0 | 0 | 4 | - | PF=109 |
| 110 | 2-OCH₃ | 5-CyHex | 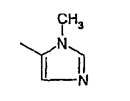 | H | 0 | 1 | 3 | - | PF = 228 |
| 111 | 2-OCH₃ | 5-CyHex | 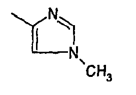 | H | 0 | 1 | 3 | - | PF = 237 |
| 112 | 2-OCH₃ | 5-CyHex | 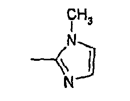 | H | 0 | 1 | 3 | - | PF = 113 |
| 113 | 2-OCH₃ | 5-CyHex | 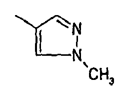 | H | 0 | 1 | 3 | - | PF = 187 |
| 114 | 2-OCH₃ | 5-CyHex | 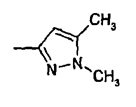 | H | 0 | 1 | 3 | - | PF = 113 |
| 115 | 2-OCH₃ | 5-CyHex | 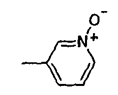 | H | 0 | 1 | 3 | - | PF = 242 |
| 116 | 2-OCH₃ | 5-CyHex | 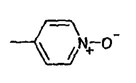 | H | 0 | 1 | 3 | - | PF = 250 |
| 117 | 2-OCH₃ | 5-(CH₂)₃CH₃ | 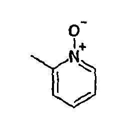 | H | 0 | 1 | 3 | - | PF = 134 |
| 118 | 2-OCH₃ | 5-CyPent | 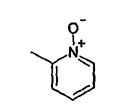 | H | 0 | 1 | 3 | - | PF = 160 |
| 119 | 2-OCH₂CH₃ | 5-(CH₂)₃CH₃ | 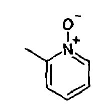 | H | 0 | 1 | 3 | - | PF = 152 |
| 120 | 2-OCH₃ | 5-CyHex | 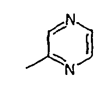 | H | 0 | 2 | 2 | - | PF = 129 |
| 121 (R) | 2-OCH₃ | 5-CyHex | 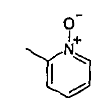 | H | 0 | 1 | 3 | - | PF = 178 |
| 122 | 2-OCH₃ | 5-CyHex | 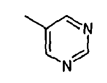 | H | 0 | 1 | 3 | - | PF = 138 |
| 123 | 2-OCH₃ | 5-CyHex | 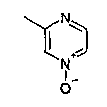 | H | 0 | 1 | 3 | - | PF = 143 |
| 124 (S) | 2-OCH₃ | 5-CyHex | 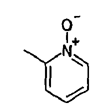 | H | 0 | 1 | 3 | - | PF = 181 |
| 125 | 2-OCH₂CH₃ | 5-CyPent | 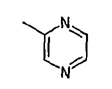 | H | 0 | 1 | 3 | - | PF = 206 |
| 126 | 2-OCH₃ | 5-O(CH₂)₂CH₃ | 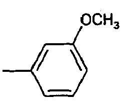 | H | 0 | 1 | 3 | - | MH+=510, tr= 10,16 |
| 127 | 2-OCH₃ | 5-O(CH₂)₂CH₃ | 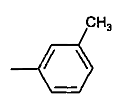 | H | 0 | 1 | 3 | - | MH+ = 494, tr = 10,45 |
| 128 | 2-OCH₃ | 5-O(CH₂)₂CH₃ | 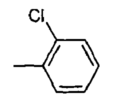 | H | 0 | 1 | 3 | - | MH+ = 514, tr = 10,41 |
| 129 | 2-OCH₃ | 5-O(CH₂)₂CH₃ | 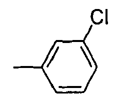 | H | 0 | 1 | 3 | - | MH+ = 514, tr = 10,57 |
| 130 | 2-OCH₃ | 5-O(CH₂)₂CH₃ | 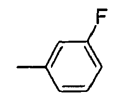 | H | 0 | 1 | 3 | - | MH+ = 498, tr =10,22 |
| 131 | 2-OCH₃ | 5-O(CH₂)₂CH₃ | 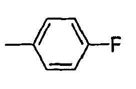 | H | 0 | 1 | 3 | - | MH+ = 498, tr= 10,18 |
| 132 | 2-OCH₃ | 5-O(CH₂)₂CH₃ | 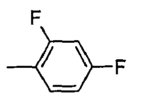 | H | 0 | 1 | 3 | - | MH+ = 516, tr = 10,31 |
| 133 | 2-OCH₃ | 5-O(CH₂)₂CH₃ | 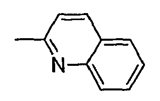 | H | 0 | 1 | 3 | - | MH+ = 531, tr= 10,14 |
| 134 | 2-OCH₃ | 5-O(CH₂)₂CH₃ | 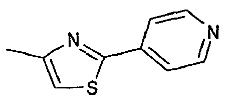 | H | 0 | 1 | 3 | - | MH+ = 564, tr = 8,66 |
| 135 | 2-OCH₃ | 5-O(CH₂)₂CH₃ | 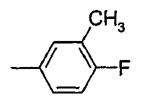 | H | 0 | 1 | 3 | - | MH+ = 512, tr = 10,54 |
| 136 | 2-OCH₃ | 5-O(CH₂)₂CH₃ | 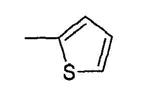 | H | 0 | 1 | 3 | - | MH+ = 486, tr = 10,07 |
| 137 | 2-OCH₃ | 5-O(CH₂)₂CH₃ | 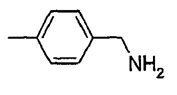 | H | 0 | 1 | 3 | - | MH+ = 509, tr = 6,83 |
| 138 | 2-OCH₃ | 5-O(CH₂)₂CH₃ | 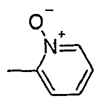 | H | 0 | 1 | 3 | - | MH+ = 497, tr = 8,33 |
| 139 | 2-OCH₃ | 5-O(CH₂)₂CH₃ | 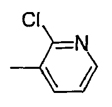 | H | 0 | 1 | 3 | - | MH+ = 515, tr = 9,58 |
| 140 | 2-OCH₃ | 5-O(CH₂)₂CH₃ | 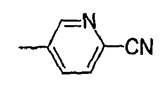 | H | 0 | 1 | 3 | - | MH+ = 506, tr = 9,59 |
| 141 | 2-OCH₃ | 5-O(CH₂)₂CH₃ | 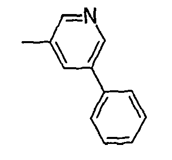 | H | 0 | 1 | 3 | - | MH+ = 557, tr = 10,03 |
| 142 | 2-OCH₃ | 5-O(CH₂)₂CH₃ | 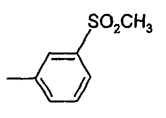 | H | 0 | 1 | 3 | - | MH+ = 558, tr = 9,41 |
| 143 | 2-OCH₃ | 5-O(CH₂)₂CH₃ | 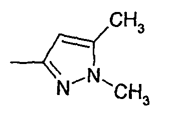 | H | 0 | 1 | 3 | - | MH+ = 498, tr = 9,46 |
| 144 | 2-OCH₃ | 5-O(CH₂)₂CH₃ | 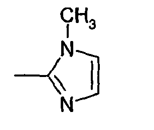 | H | 0 | 1 | 3 | - | MH+ = 484, tr = 8,49 |
| 145 | 2-OCH₃ | 5-O(CH₂)₂CH₃ | 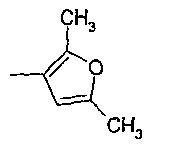 | H | 0 | 1 | 3 | - | MH+ = 498, tr = 10,35 |
| 146 | 2-OCH₃ | 5-O(CH₂)₂CH₃ | 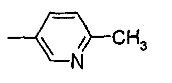 | H | 0 | 1 | 3 | - | MH+ = 495, tr = 8,38 |
| 147 | 2-OCH₃ | 5-O(CH₂)₂CH₃ | 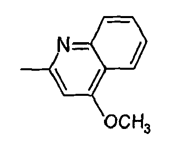 | H | 0 | 1 | 3 | - | MH+ = 561, tr= 10,11 |
| 148 | 2-OCH₃ | 5-O(CH₂)₂CH₃ | 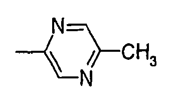 | H | 0 | 1 | 3 | - | MH+ = 494, tr= 11,0 |
| 149 | 2-OCH₃ | 5-(CH₂)₃CH₃ | 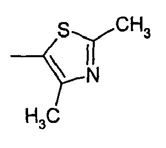 | H | 0 | 1 | 3 | - | MH+ = 513, tr = 10,24 |
| 150 | 2-OCH₃ | 5-(CH₂)₃CH₃ | 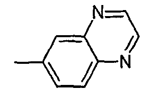 | H | 0 | 1 | 3 | - | MH+ = 530, tr = 10,04 |
| 151 | 2-OCH₃ | 5-CyHex | 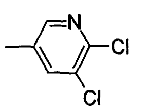 | H | 0 | 1 | 3 | - | MH+ = 573, tr = 11,67 |
| 152 | 2-OCH₃ | 5-CyHex | 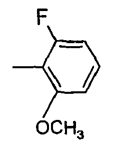 | H | 0 | 1 | 3 | - | MH+ = 552, tr = 11,39 |
| 153 | 2-OCH₃ | 5-CyHex | 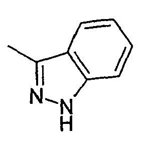 | H | 0 | 1 | 3 | - | MH+ = 544, tr = 11,02 |
| 154 | 2-OCH₃ | 5-O(CH₂)₂CH₃ | 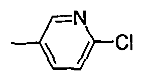 | H | 0 | 1 | 3 | - | MH+ = 515, tr = 9,80 |
| 155 | 2-OCH₃ | 5-O(CH₂)₂CH₃ | 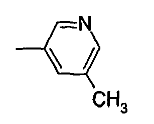 | H | 0 | 1 | 3 | - | MH+ = 495, tr = 8,74 |
| 156 | 2-OCH₃ | 5-O(CH₂)₂CH₃ | 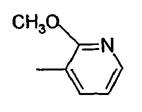 | H | 0 | 1 | 3 | - | MH+ = 511, tr = 9,64 |
| 157 | 2-OCH₃ | 5-O(CH₂)₂CH₃ | 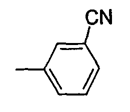 | H | 0 | 1 | 3 | - | MH+ = 505, tr = 9,89 |
| 158 | 2-OCH₃ | 5-O(CH₂)₂CH₃ | 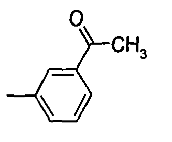 | H | 0 | 1 | 3 | - | MH+ = 522, tr = 9,78 |
| 159 | 2-OCH₃ | 5-O(CH₂)₂CH₃ | 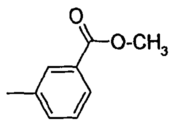 | H | 0 | 1 | 3 | - | MH+ = 538, tr= 10,10 |
| 160 | 2-OCH₃ | 5-(CH₂)₃CH₃ | 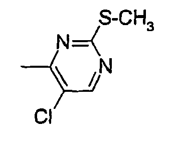 | H | 0 | 1 | 3 | - | MH+ = 560, tr=11,25 |
| 161 | 2-OCH₃ | 5-(CH₂)₃CH₃ | 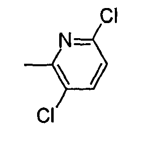 | H | 0 | 1 | 3 | - | MH+ = 547, tr = 11,07 |
| 162 | 2-OCH₃ | 5-(CH₂)₃CH₃ | 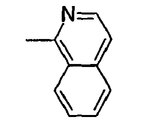 | H | 0 | 1 | 3 | - | MH+ = 529, tr = 10,69 |
| 163 | 2-OCH₃ | 5-(CH₂)₃CH₃ | 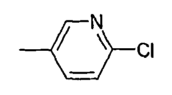 | H | 0 | 1 | 3 | - | MH+ = 513, tr = 10,62 |
| 164 | 2-OCH₃ | 5-(CH₂)₃CH₃ | 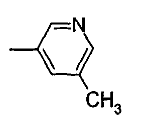 | H | 0 | 1 | 3 | - | MH+ = 493, tr = 6,52 |
| 165 | 2-OCH₃ | 5-(CH₂)₃CH₃ | 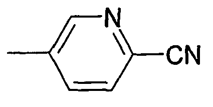 | H | 0 | 1 | 3 | - | MH+ = 504, tr =10, 38 |
| 166 | 2-OCH₃ | 5-(CH₂)₃CH₃ | 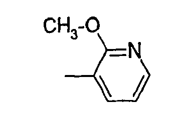 | H | 0 | 1 | 3 | - | MH+ = 509, tr = 10,46 |
| 167 | 2-OCH₃ | 5-(CH₂)₃CH₃ | 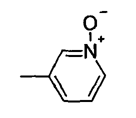 | H | 0 | 1 | 3 | - | MH+ = 495, tr = 8, 96 |
| 168 | 2-OCH₃ | 5-(CH₂)₃CH₃ | 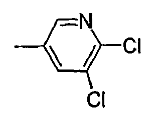 | H | 0 | 1 | 3 | - | MH+ = 547, tr = 11,24 |
| 169 | 2-OCH₃ | 5-(CH₂)₃CH₃ | 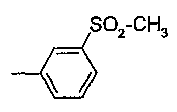 | H | 0 | 1 | 3 | - | MH+ = 556, tr= 10,19 |
| 170 | 2-OCH₃ | 5-(CH₂)₃CH₃ | 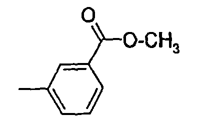 | H | 0 | 1 | 3 | - | MH+ = 536, tr = 10,84 |
| 171 | 2-OCH₃ | 5-(CH₂)₃CH₃ | 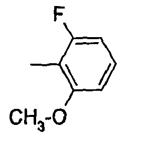 | H | 0 | 1 | 3 | - | MH+ = 526, tr = 10,98 |
| 172 | 2-OCH₃ | 5-(CH₂)₃CH₃ | 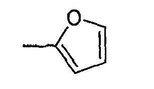 | H | 0 | 1 | 3 | - | MH+ = 468, tr = 10,56 |
| 173 | 2-OCH₃ | 5-(CH₂)₃CH₃ | 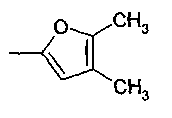 | H | 0 | 1 | 3 | - | MH+ = 496, tr= 11,17 |
| 174 | 2-OCH₃ | 5-(CH₂)₃CH₃ | 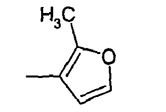 | H | 0 | 1 | 3 | - | MH+ = 482, tr = 10,77 |
| 175 | 2-OCH₃ | 5-(CH₂)₃CH₃ | 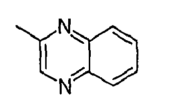 | H | 0 | 1 | 3 | - | MH+ = 530, tr = 10,79 |
| 176 | 2-OCH₃ | 5-(CH₂)₃CH₃ | 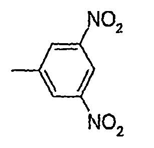 | H | 0 | 1 | 3 | - | MH+ = 568, tr = 14,07 |
| 177 | 2-OCH₃ | 5-(CH₂)₃CH₃ | 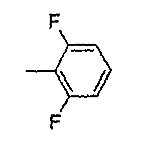 | H | 0 | 1 | 3 | - | MH+ = 514, tr = 11,07 |
| 178 | 2-OCH₃ | 5-(CH₂)₃CH₃ | 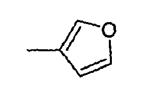 | H | 0 | 1 | 3 | - | MH+ = 468, tr = 10,45 |
| 179 | 2-OCH₃ | 5-(CH₂)₃CH₃ | 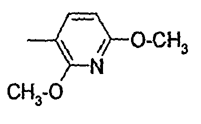 | H | 0 | 1 | 3 | - | MH+ = 539, tr=11,11 |
| 180 | 2-OCH₃ | 5-(CH₂)₃CH₃ | 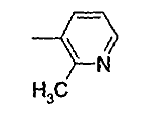 | H | 0 | 1 | 3 | - | MH+ = 493, tr = 8,91 |
| 181 | 2-OCH₃ | 5-(CH₂)₃CH₃ | 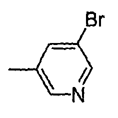 | H | 0 | 1 | 3 | - | MH+ = 558, tr = 10,62 |
| 182 | 2-OCH₃ | 5-(CH₂)₃CH₃ | 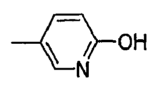 | H | 0 | 1 | 3 | - | MH+ = 495, tr = 9,04 |
| 183 | 2-OCH₃ | 5-(CH₂)₃CH₃ | 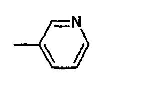 | H | 2 | 1 | 3 | - | MH+ = 507, tr = 7,84 |
| 184 | 2-OCH₃ | 5-(CH₂)₃CH₃ | 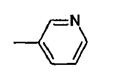 | H | 1 | 1 | 3 | - | MH+ = 493, tr = 8,01 |
| 185 | 2-OCH₃ | 5-(CH₂)₃CH₃ | 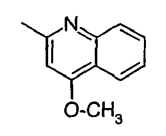 | H | 0 | 1 | 3 | - | MH+ = 559, tr = 10,88 |
| 186 | 2-OCH₃ | 5-(CH₂)₃CH₃ | 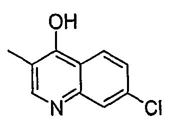 | H | 0 | 1 | 3 | - | MH+ = 579, tr = 9,99 |
| 187 | 2-OCH₃ | 5-(CH₂)₃CH₃ | 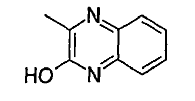 | H | 0 | 1 | 3 | - | MH+ = 546, tr = 9,93 |
| 188 | 2-OCH₃ | 5-(CH₂)₃CH₃ | 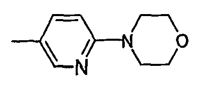 | H | 0 | 1 | 3 | - | MH+ = 564, tr = 9,78 |
| 189 | 2-OCH₃ | 5-(CH₂)₃CH₃ | 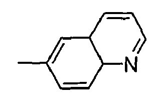 | H | 0 | 1 | 3 | - | MH+ = 529, tr = 9, 57 |
| 190 | 2-OCH₃ | 5-CyHex | 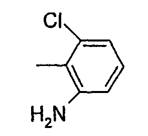 | H | 0 | 1 | 3 | - | MH+ = 553, tr = 11,38 |
| 191 | 2-OCH₃ | 5-CyHex | 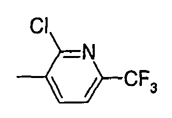 | H | 0 | 1 | 3 | - | MH+ = 607, tr = 11,80 |
| 192 | 2-OCH₃ | 5-CyHex | 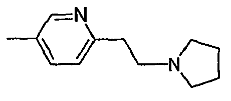 | H | 0 | 1 | 3 | - | MH+ = 602, tr = 7,68 |
| 193 | 2-OCH₃ | 5-CyHex | 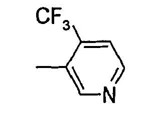 | H | 0 | 1 | 3 | - | MH+ = 573, tr = 11,09 |
| 194 | 2-OCH₃ | 5-CyHex | 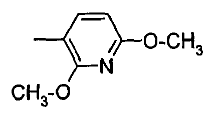 | H | 0 | 1 | 3 | - | MH+ = 565, tr = 11,53 |
| 195 (S) | 2-OCH₃ | 5-CyHex | 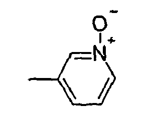 | H | 0 | 1 | 3 | - | MH+ = 521 tr = 9,37 PF = 137 |
| 196 | 2-OCH₃ | 5-CyHex | 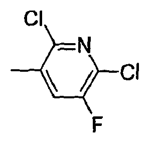 | H | 0 | 1 | 3 | - | MH+ = 591, tr= 11,84 |
| 197 | 2-OCH₃ | 5-CyHex | 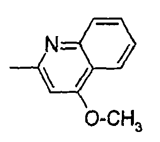 | H | 0 | 1 | 3 | - | MH+ = 584, tr = 11,33 |
| 198 | 2-OCH₃ | 5-CyHex | 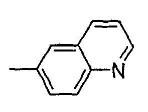 | H | 0 | 1 | 3 | - | MH+ = 554, tr = 9,99 |
| 199 | 2-OCH₂CH₃ | 5-CH₂CH₃ | 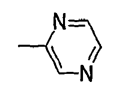 | H | 0 | 1 | 3 | - | MH+ = 466, tr = 9,42 |
| 200 | 2-OCH₂CH₃ | 5-CH₂CH₃ | 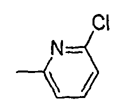 | H | 0 | 1 | 3 | - | MH+ = 499, tr = 10,36 |
| 201 | 2-OCH₂CH₃ | 5-CH₂CH₃ | 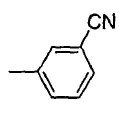 | H | 0 | 1 | 3 | - | MH+ = 489, tr = 10,23 |
| 202 | 2-OCH₂CH₃ | 5-CH₂CH₃ | 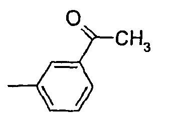 | H | 0 | 1 | 3 | - | MH+ = 506, tr= 10,12 |
| 203 | 2-OCH₂CH₃ | 5-CH₂CH₃ | 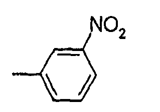 | H | 0 | 1 | 3 | - | MH+ = 509, tr = 10,45 |
| 204 | 2-OCH₂CH₃ | 5-CH₂CH₃ | 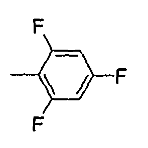 | H | 0 | 1 | 3 | - | MH+ = 518, tr = 10,81 |
| 205 | 2-OCH₂CH₃ | 5-CH₂CH₃ | 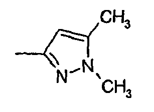 | H | 0 | 1 | 3 | - | MH+ = 482, tr = 9,82 |
| 206 | 2-OCH₂CH₃ | 5-CH₂CH₃ | 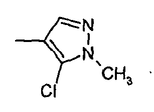 | H | 0 | 1 | 3 | - | MH+ = 502, tr = 9,76 |
| 207 | 2-OCH₂CH₃ | 5-CH₂CH₃ | 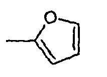 | H | 0 | 1 | 3 | - | MH+ = 454, tr= 10,11 |
| 208 | 2-OCH₂CH₃ | 5-CH₂CH₃ | 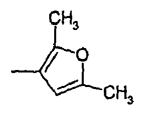 | H | 0 | 1 | 3 | - | MH+ = 482, tr = 9,82 |
| 209 | 2-OCH₂CH₃ | 5-CH₂CH₃ | 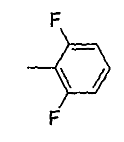 | H | 0 | 1 | 3 | - | MH+ = 500, tr = 10,66 |
| 210 | 2-OCH₂CH₃ | 5-CH₂CH₃ | 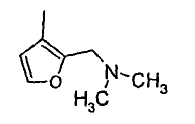 | H | 0 | 1 | 3 | - | MH+ = 511, tr = 7,16 |
| 211 | 2-OCH₂CH₃ | 5-CH₂CH₃ | 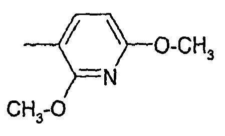 | H | 0 | 1 | 3 | - | MH+ = 525, tr = 10,60 |
| 212 | 2-OCH₂CH₃ | 5-CH₂CH₃ | 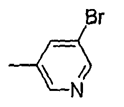 | H | 0 | 1 | 3 | - | MH+ = 543, tr = 10,17 |
| 213 | 2-OCH₂CH₃ | 5-CH₂CH₃ | 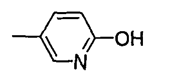 | H | 0 | 1 | 3 | - | MH+ = 481, tr = 8,61 |
| 214 | 2-OCH₂CH₃ | 5-CH₂CH₃ | 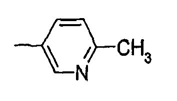 | H | 0 | 1 | 3 | - | MH+ = 479, tr = 8,69 |
| 215 | 2-OCH₂CH₃ | 5-CH₂CH₃ | 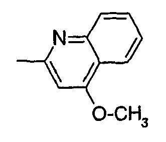 | H | 0 | 1 | 3 | - | MH+ = 545, tr = 10,45 |
| 216 | 2-OCH₂CH₃ | 5-CH₂CH₃ | 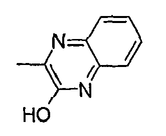 | H | 0 | 1 | 3 | - | MH+ = 532, tr = 9,51 |
| 217 | 2-OCH₂CH₃ | 5-CH₂CH₃ | 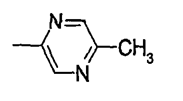 | H | 0 | 1 | 3 | - | MH+ = 480, tr = 9,62 |
| 218 | 2-OCH₂CH₃ | 5-CH₂CH₃ | 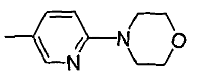 | H | 0 | 1 | 3 | - | MH+ = 550, tr = 9,35 |
| 219 | 2-OCH₂CH₃ | 5-CH₂CH₃ | 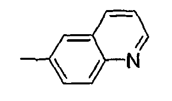 | H | 0 | 1 | 3 | - | MH+ = 515, tr = 9,13 |
| 220 | 2-OCH₃ | 5-(CH₂)₃CH₃ | 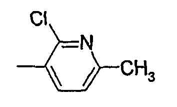 | H | 0 | 1 | 3 | - | MH+ = 527, tr = 10,62 |
| 221 | 2-OCH₃ | 5-CyHex | 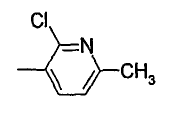 | H | 0 | 1 | 3 | - | MH+ = 553, tr= 11,10 |
| 222 | 2-OCH₃ | 5-O(CH₂)₂CH₃ | 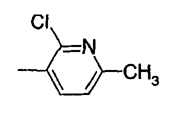 | H | 0 | 1 | 3 | - | MH+ = 529, tr = 9,82 |
| 223 | 2-OCH₂CH₃ | 5-CH₂CH₃ | 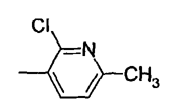 | H | 0 | 1 | 3 | - | MH+ = 513, tr = 10,17 |
| 224 | 2-OCH₃ | 5-O(CH₂)₂CH₃ | 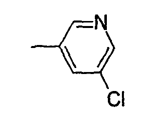 | H | 0 | 1 | 3 | - | MH+ = 515, tr = 9,96 |
| 225 | 2-OCH₃ | 5-(CH₂)₃CH₃ | 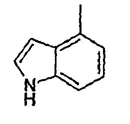 | H | 0 | 1 | 3 | - | MH+ = 517, tr = 10,55 |
| 226 | 2-OCH₃ | 5-(CH₂)₃CH₃ | 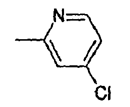 | H | 0 | 1 | 3 | - | MH+ =513, tr = 10,80 |
| 227 | 2-OCH₃ | 5-CyHex | 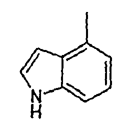 | H | 0 | 1 | 3 | - | MH+ = 543, tr = 10,95 |
| 228 | 2-OCH₃ | 5-CyHex | 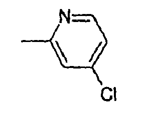 | H | 0 | 1 | 3 | - | MH+ = 539, tr = 11,31 |
| 229 | 2-OCH₃ | 5-CyHex | 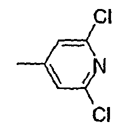 | H | 0 | 1 | 3 | - | MH+ = 573, tr = 11,72 |
| 230 | 2-OCH₂CH₃ | 5-CH₂CH₃ | 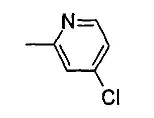 | H | 0 | 1 | 3 | - | MH+ = 499, tr = 10,33 |
| 231 | 2-OCH₃ | 5-(CH₂)₃CH₃ | 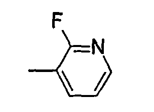 | H | 0 | 1 | 3 | - | MH+ = 497, tr =10,36 |
| 232 | 2-OCH₃ | 5-(CH₂)₃CH₃ | 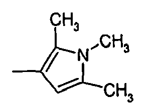 | H | 0 | 1 | 3 | - | MH+ = 509, tr = 10,95 |
| 233 | 2-OCH₃ | 5-CyHex | 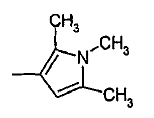 | H | 0 | 1 | 3 | - | MH+ = 535, tr= 11,38 |
| 234 | 2-OCH₂CH₃ | 5-CH₂CH₃ | 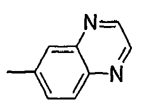 | H | 0 | 1 | 3 | - | MH+ = 516, tr = 9,58 |
| 235 | 2-OCH₃ | 5-(CH₂)₂CH₃ | 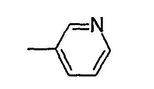 | H | 0 | 1 | 3 | HCl | PF = 172 |
| 236 | 2-OCH₃ | 5-(CH₂)₂CH₃ | 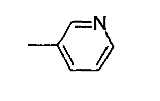 | F | 0 | 1 | 3 | - | MH+ = 483, tr = 9,21, PF=160 |
| 237 (S) | 2-OCH₃ | 5-CyHex | 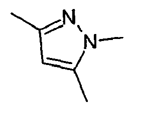 | H | 0 | 1 | 3 | - | PF = 112 |
| 238 (S) | 2-OCH₃ | 5-CyHex | 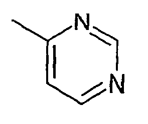 | H | 0 | 1 | 3 | - | PF = 124 |
| 239 (R) | 2-OCH₃ | 5-CyHex | 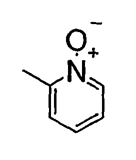 | H | 0 | 1 | 2 | - | PF= 155 |
| 240 (S) | 2-OCH₃ | 5-CyHex | 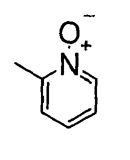 | H | 0 | 1 | 2 | - | PF= 157 |
| 241 | 2-OCH₃ | 5-CyHex | 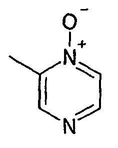 | H | 0 | 1 | 3 | - | PF = 137 |
| 242 (S) | 2-OCH₂CH₃ | 5-CyPent | 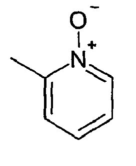 | H | 0 | 1 | 3 | - | -PF = 155 |
| 243 (S) | 2-OCH₂CH₃ | 5-CyPent | 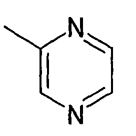 | H | 0 | 1 | 3 | - | PF=137 |
| 244 (R) | 2-OCH₃ | 5-CyPent | 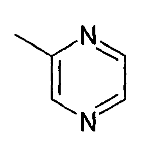 | H | 0 | 1 | 3 | - | PF = 117 |
| 245 (R) | 2-OCH₃ | 5-CyPent | 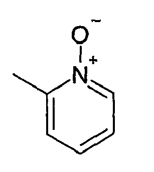 | H | 0 | 1 | 3 | - | PF = 160 |

Les composés selon l'invention ont fait l'objet d'essais pharmacologiques permettant de déterminer leur effet modulateur de l'activité des récepteurs aux chimiokines. Les chimiokines sont des protéines de bas poids moléculaire qui appartiennent à la famille des cytokines proinflammatoires et sont impliquées dans le chimiotactisme des leucocytes et des cellules endothéliales. Les chimiokines contrôlent de nombreux processus biologiques et sont associées à des désordres inflammatoires apparaissant lors des états de stress, lors de blessures ou d'infections ; la modulation des effets des chimiokines permet de prévenir ou de traiter des pathologies comme l'asthme, l'arthrite, les allergies, les maladies auto-immunes, l'athérosclérose ou l'angiogénèse (C.D. Paavola et al., J. Biol. Chem., 1998, 273, (50), 33157-33165). Parmi les chimiokines, on distingue le hMCP-1 (de l'anglais human Monocyte Chemotactic Protein) qui appartient au groupe des CC chimiokines et qui est un agoniste naturel du récepteur CCR2b.

On a mesuré l'activité inhibitrice des composés selon l'invention sur des cellules exprimant le récepteur CCR2b humain. La concentration d'agoniste naturel hMCP-1 qui inhibe 50 % (Cl₅₀) de l'activité du récepteur CCR2b est de 0,57 nM. Les composés selon l'invention présentent une Cl₅₀ généralement inférieure à 1 µM. Par exemple, le composé n° 20 présente une Cl₅₀ de 25 nM, le composé n° 31 présente une Cl₅₀ de 44 nM, le composé n° 1 présente une Cl₅₀ de 290 nM, le composé n°107 présente une Cl₅₀ de 5 nM.

On a également mesuré l'inhibition du chimiotactisme sur des cellules monocytaires THP-1 humaines (commercialisées par DSMZ - Allemagne) en utilisant une technique adaptée de celle décrite par A. Albini et al., Cancer Res., 1987, 47, 3239-3245. Dans ces conditions le hMCP-1 présente une Cl₅₀ de 6 nM. Les composés selon l'invention présentent une Cl₅₀ généralement inférieure à 1 µM.
L'inhibition du chimiotactisme par les composés selon l'invention est le signe de leur activité antagoniste sur les récepteurs des chimiokines et en particulier du CCR2b.
Il apparaît donc que les composés selon l'invention sont des antagonistes de l'effet des chimiokines, en particulier du hMCP-1.
Les composés selon l'invention peuvent donc être utilisés pour la préparation de médicaments, en particulier de médicaments antagonistes de l'effet des chimiokines.

Ainsi, selon un autre de ses aspects, la présente invention a pour objet des médicaments qui comprennent un composé de formule (I), ou un sel d'addition de ce dernier à un acide pharmaceutiquement acceptable, ou encore un hydrate ou un solvat.
Ces médicaments trouvent leur emploi en thérapeutique, notamment dans la prévention et le traitement de différentes pathologies telles que :
- les maladies et les syndromes immuno-inflammatoires aigus et chroniques comme l'athérosclérose, les resténoses, les maladies pulmonaires chroniques, en particulier COPD (de l'anglais chronic obstructive pulmonary disease) ; le syndrome de détresse respiratoire ; l'hyperactivité bronchique; les colites; la silicose ; les pathologies fibreuses, les fibroses pulmonaires, les fibroses kystiques ; les infections virales ou bactériennes, SIDA (Syndrome d'Immunodéficience Acquise), méningite , malaria, lèpre, tuberculose, herpès, infections par cytomégalovirus; les chocs septiques, la septicémie, les chocs endotoxiques ; les rejets de greffes ; les pathologies osseuses telles que l'ostéoporose, les ostéoarthrites ; les conjonctivites ; les dermatites atypiques ou de contact; les eczémas ; les glomérulonéphrites ; les pancréatites ; les colites ulcéreuses, les maladies auto-immunes comme la polyarthrite rhumatoïde, la sclérose en plaques, la sclérose amyotrophique latérale, la maladie de Crohn, le lupus érythémateux, la sclérodermie, le psoriasis; la maladie de Parkinson ; la maladie d'Alzheimer ; le diabète ; la cachexie; l'obésité;
- le traitement des cancers;
- le traitement de la douleur, en particulier neuropathique et inflammatoire;
- les maladies allergiques comme les maladies respiratoires allergiques, l'asthme, les rhinites, l'hypersensibilité pulmonaire, l'hypersensibilité retardée;
- les maladies et les désordres dans lesquels les processus angiogéniques sont impliqués (angiogénèse intratumorale par exemple), les maladies rétiniennes (dégénérescence maculaire liée à l'age : DMLA );
- les pathologies cardiaques : choc hémodynamique ; les ischémies cardiaques ; les attaques de réinfusion post-ischémique ; l'infarctus du myocarde, la thrombose coronarienne, l'insuffisance cardiaque, l'angine de poitrine.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 0,1 à 1000 mg/kg, en une ou plusieurs prises.
Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.
La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé de formule (I) suivante : 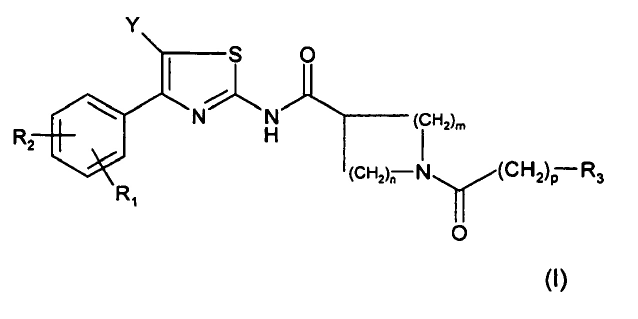 dans laquelle :
- R₁ représente un atome d'hydrogène ou d'halogène, un groupe (C₁-C₄)alkyle, trifluoro(C₁-C₄)alkyle, hydroxyle, (C₁-C₄)alcoxy, trifluoro(C₁-C₄)alcoxy, (C₃-C₁₀)cycloalkyl(C₁-C₄)alcoxy, (C₃-C₁₀)cycloalkyloxy, allyloxy, (C₁-C₄)alkylthio ;
- R₂ représente un atome d'hydrogène ou d'halogène, un groupe hydroxyle, (C₁-C₄)alkyle, trifluoro(C₁-C₄)alkyle, perfluoro(C₁-C₄)alkyle, (C₃-C₁₀)cycloalkyle, (C₁-C₄)alcoxy, (C₃-C₁₀)cycloalkyl(C₁-C₄)alcoxy, (C₃-C₁₀)cycloalkyloxy, allyloxy, (C₃-C₈)cycloalkyl(C₁-C₄)alkyle ;
- Y représente un atome d'hydrogène ou un halogène;
- R₃ représente :
• un groupe phényle, substitué par un ou plusieurs atomes d'halogène et/ou par un ou plusieurs groupes :
(C₁-C₆)alkyle,
(C₁-C₆)alcoxy,
-NO₂,
cyano,
-COR₄,
-SO₂R₄,
-CO₂R₄, dans lequel R₄ représente un groupe (C₁-C₄)alkyle,
-(CH₂)_{q}NR₅R₆ dans lequel R₅ et R₆ représentent indépendamment l'un de l'autre un atome d'hydrogène, un groupe (C₁-C₄)alkyle ou R₅ et R₆ forment ensemble avec l'atome d'azote auquel ils sont liés un groupe cycloalkyle à 5 ou 6 chaînons et q représente 0, 1 ou 2 ;
• un groupe hétérocyclique éventuellement substitué par un ou plusieurs halogènes et/ou par un ou plusieurs groupes :
(C₁-C₄)alkyle,
hydroxyle,
(C₁-C₄)alcoxy,
cyano,
morpholine,
trifluoro(C₁-C₄)alkyle,
-COR₄,
-SO₂R₄ , dans lequel R₄ est tel que défini dans ce qui précède,
-(CH₂)_{q}NR₅R₆, dans lequel R₅, R₆ et q sont tels que définis dans ce qui précède,
phényle,
pyridine,
-SCH₃ ;
le ou les atomes d'azote du groupe hétérocyclique étant éventuellement substitués par un groupe (C₁-C₄)alkyle,
le ou les atomes d'azote du groupe hétérocyclique étant éventuellement sous leur forme N-oxyde ;
• un groupe hétérobicyclique éventuellement substitué par un ou plusieurs halogènes et/ou par un ou plusieurs groupes hydroxyles, (C₁-C₄)alkyle ou (C₁-C₄)alcoxy ;
- m représente 2, 3 ou 4 ;
- n représente 0, 1 ou 2 ;
- p représente 0, 1, 2 ou 3.
à l'état de base ou de sel d'addition à un acide pharmaceutiquement acceptable, à l'état d'hydrates ou de solvats, ainsi que sous forme d'énantiomères, de diastéréoisomères et leur mélange.

2. Composé selon la revendication 1, de formule (I) dans laquelle :
- R₁ et R₂ sont tels que définis dans la revendication 1 ;
- Y représente un atome d'hydrogène ou un halogène ;
- R₃ représente :
• un groupe phényle, substitué par un ou plusieurs atomes d'halogène et/ou par un ou plusieurs groupes :
(C₁-C₆)alkyle,
(C₁-C₆)alcoxy,
-NO₂,
cyano,
-COR₄,
-SO₂R₄,
-CO₂R₄, dans lequel R₄ représente un groupe (C₁-C₄)alkyle,
-(CH₂)_{q}NR₅R₆, dans lequel R₅, R₆ et q sont tels que définis dans la revendication 1;
• un groupe hétérocyclique éventuellement substitué par un ou plusieurs halogènes et/ou par un ou plusieurs groupes :
(C₁-C₄)alkyle,
hydroxyle,
(C₁-C₄)alcoxy,
cyano,
morpholine,
trifluoro(C₁-C₄)alkyle,
-COR₄,
-SO₂R₄, dans lequel R₄ est tel que défini dans la revendication 1,
-(CH₂)_{q}NR₅R₆, dans lequel R₅, R₆ et q sont tels que définis dans la revendication 1,
phényle,
pyridine,
-SCH₃ ;
le ou les atomes d'azote du groupe hétérocyclique étant éventuellement substitués par un groupe (C₁-C₄)alkyle,
le ou les atomes d'azote du groupe hétérocyclique étant éventuellement sous leur forme N-oxyde ;
• un groupe hétérobicyclique éventuellement substitué par un ou plusieurs halogènes et/ou par un ou plusieurs groupes hydroxyles, (C₁-C₄)alkyle ou (C₁-C₄)alcoxy ;
- m représente 3 ;
- n représente 1 ;
- p représente 0 ;
à l'état de base ou de sel d'addition à un acide pharmaceutiquement acceptable, à l'état d'hydrates ou de solvats, ainsi que sous forme d'énantiomères, de diastéréoisomères et leur mélange.

3. Composé selon la revendication 1, de formule (I) dans laquelle :
- R₁ et R₂ sont tels que définis dans la revendication 1 ;
- Y est tel que défini dans la revendication 1 ;
- R₃ représente :
• un groupe phényle, substitué par un ou plusieurs atomes d'halogène et/ou par un ou plusieurs groupes :
(C₁-C₆)alkyle,
(C₁-C₆)alcoxy,
-NO₂,
cyano,
-COR₄,
-SO₂R₄,
-CO₂R₄, dans lequel R₄ représentant un groupe (C₁-C₄)alkyle,
-(CH₂)_{q}NR₅R₆, dans lequel R₅, R₆ et q sont tels que définis dans la revendication 1 ;
• un groupe pyridinyle, pyrazinyle, pyrimidinyle, pyridazinyle, pyrazolyle, imidazolyle, thiazolyle, isothiazolyle, oxazolyle, isooxazolyle, oxadiazolyle, thiophényle, pyrrolyle, furanyle, lesdits groupes précités étant éventuellement substitués par un ou plusieurs halogènes et/ou par un ou plusieurs groupes :
(C₁-C₄)alkyle,
hydroxyle,
(C₁-C₄)alcoxy,
cyano,
morpholine,
trifluoro(C₁-C₄)alkyle,
-COR₄,
-SO₂R₄ , dans lequel R₄ est tel que défini dans la revendication 1,
-(CH₂)_{q}NR₅R₆, dans lequel R₅, R₆ et q sont tels que définis dans la revendication 1,
phényle,
pyridine,
-SCH₃ ;
le ou les atomes d'azote desdits groupes cités ci-dessus étant éventuellement substitués par un groupe (C₁-C₄)alkyle,
le ou les atomes d'azote desdits groupes cités ci-dessus étant éventuellement sous leur forme N-oxyde ;
• un groupe quinoxaline, quinoline, isoquinoline, indole, isoindole, indoline, indazole, benzothiazole, benzimidazole, benzoxazole, lesdits groupes précités étant éventuellement substitués par un ou plusieurs halogènes et/ou par un ou plusieurs groupes hydroxyles, (C₁-C₄)alkyle ou (C₁-C₄)alcoxy ;
- m représente 3 ;
- n représente 1 ;
- p représente 0 ;
à l'état de base ou de sel d'addition à un acide pharmaceutiquement acceptable, à l'état d'hydrates ou de solvats, ainsi que sous forme d'énantiomères, de diastéréoisomères et leur mélange.

4. Composé selon la revendication 1, de formule (I) dans laquelle :
- R₁ et R₂ sont tels que définis dans la revendication 1 ;
- Y représente un atome d'hydrogène ou un halogène;
- R₃ représente :
• un groupe phényle, substitué par un ou plusieurs atomes d'halogène et/ou par un ou plusieurs groupes :
(C₁-C₆)alkyle,
(C₁-C₆)alcoxy,
-NO₂,
cyano,
-COR₄,
-SO₂R₄,
-CO₂R₄, dans lequel R₄ représente un groupe (C₁-C₄)alkyle,
-(CH₂)_{q}NR₅R₆, dans lequel R₅, R₆ et q sont tels que définis dans la revendication 1;
• un groupe hétérocyclique éventuellement substitué par un ou plusieurs halogènes et/ou par un ou plusieurs groupes :
(C₁-C₄)alkyle,
hydroxyle,
(C₁-C₄)alcoxy,
cyano,
morpholine,
trifluoro(C₁-C₄)alkyle,
-COR₄,
-SO₂R₄, dans lequel R₄ est tel que défini dans la revendication 1,
-(CH₂)_{q}NR₅R₆, dans lequel R₅, R₆ et q sont tels que définis dans la revendication 1,
phényle,
pyridine,
-SCH₃ ;
le ou les atomes d'azote du groupe hétérocyclique étant éventuellement substitués par un groupe (C₁-C₄)alkyle,
le ou les atomes d'azote du groupe hétérocyclique étant éventuellement sous leur forme N-oxyde ;
• un groupe hétérobicyclique éventuellement substitué par un ou plusieurs halogènes et/ou par un ou plusieurs groupes hydroxyles, (C₁-C₄)alkyle ou (C₁-C₄)alcoxy ;
- m représente 2 ou 4 ; et/ou
- n représente 0, 1 ou 2 ; et/ou
- p représente 0, 1 ou 2 ;
à l'état de base ou de sel d'addition à un acide pharmaceutiquement acceptable, à l'état d'hydrates ou de solvats, ainsi que sous forme d'énantiomères, de diastéréoisomères et leur mélange.

5. Composé selon la revendication 1, de formule (I) dans laquelle :
- R₁ et R₂ sont tels que définis dans la revendication 1 ;
- Y représente un atome d'hydrogène ou un halogène ;
- R₃ représente :
• un groupe phényle, substitué par un ou plusieurs atomes d'halogène et/ou par un ou plusieurs groupes :
(C₁-Cₑ)alkyle,
(C₁-C₆)alcoxy,
-NO₂,
cyano,
-COR₄,
-SO₂R₄,
-CO₂R₄, dans lequel R₄ représente un groupe (C₁-C₄)alkyle,
-(CH₂)_{q}NR₅R₆, dans lequel R₅, R₆ et q sont tels que définis dans la revendication 1 ;
• un groupe pyridinyle, pyrazinyle, pyrimidinyle, pyridazinyle, pyrazolyle, imidazolyle, thiazolyle, isothiazolyle, oxazolyle, isooxazolyle, oxadiazolyle, thiophényle, pyrrolyle, furanyle, lesdits groupes précités étant éventuellement substitués par un ou plusieurs halogènes et/ou par un ou plusieurs groupes :
(C₁-C₄)alkyle,
hydroxyle,
(C₁-C₄)alcoxy,
cyano,
morpholine,
trifluoro(C₁-C₄)alkyle,
-COR₄,
-SO₂R₄, dans lequel R₄ est tel que défini dans la revendication 1,
-(CH₂)_{q}NR₅R₆, dans lequel R₅, R₆ et q sont tels que définis dans la revendication 1,
phényle,
pyridine,
-SCH₃ ;
le ou les atomes d'azote desdits groupes cités ci-dessus étant éventuellement substitués par un groupe (C₁-C₄)alkyle,
le ou les atomes d'azote desdits groupes cités ci-dessus étant éventuellement sous leur forme N-oxyde ;
• un groupe quinoxaline, quinoline, isoquinoline, indole, isoindole, indoline, indazole, benzothiazole, benzimidazole, benzoxazole, lesdits groupes précités étant éventuellement substitués par un ou plusieurs halogènes et/ou par un ou plusieurs groupes hydroxyles, (C₁-C₄)alkyle ou (C₁-C₄)alcoxy ;
- m représente 2 ou 4 ; et/ou
- n représente 0, 1 ou 2 ; et/ou
- p représente 0, 1 ou 2 ;
à l'état de base ou de sel d'addition à un acide pharmaceutiquement acceptable, à l'état d'hydrates ou de solvats, ainsi que sous forme d'énantiomères, de diastéréoisomères et leur mélange.

6. Composé selon la revendication 1, de formule (I) dans laquelle :
- R₁ et R₂ sont tels que définis dans la revendication 1 ;
- Y représente un atome d'hydrogène ou un halogène;
- R₃ représente :
• un groupe phényle, substitué par un ou plusieurs atomes d'halogène et/ou par un ou plusieurs groupes :
(C₁-C₆)alkyle,
(C₁-C₆)alcoxy,
-NO₂,
cyano,
-COR₄,
-SO₂R₄,
-CO₂R₄, dans lequel R₄ représente un groupe (C₁-C₄)alkyle,
-(CH₂)qNR₅R₆, dans lequel R₅, R₆ et q sont tels que définis dans la revendication 1;
• un groupe pyridinyle, pyrazinyle, pyrimidinyle, pyridazinyle, pyrazolyle, imidazolyle, thiazolyle, isothiazolyle, oxazolyle, isooxazolyle, oxadiazolyle, thiophényle, pyrrolyle, furanyle, lesdits groupes précités étant éventuellement substitués par un ou plusieurs halogènes et/ou par un ou plusieurs groupes :
(C₁-C₄)alkyle,
hydroxyle,
(C₁-C₄)alcoxy,
cyano,
morpholine,
trifluoro(C₁-C₄)alkyle,
-CORa,
-SO₂R₄, dans lequel R₄ est tel que défini dans la revendication 1,
-(CH₂)qNR₅R₆, dans lequel R₅, R₆ et q sont tels que définis dans la revendication 1,
phényle,
pyridine,
-SCH₃ ;
le ou les atomes d'azote desdits groupes cités ci-dessus étant éventuellement substitués par un groupe (C₁-C₄)alkyle,
le ou les atomes d'azote desdits groupes cités ci-dessus étant éventuellement sous leur forme N-oxyde ;
• un groupe quinoxaline, quinoline, isoquinoline, indole, isoindole, indoline, indazole, benzothiazole, benzimidazole, benzoxazole, lesdits groupes précités étant éventuellement substitués par un ou plusieurs halogènes et/ou par un ou plusieurs groupes hydroxyles, (C₁-C₄)alkyle ou (C₁-C₄)alcoxy;
- m représente 2 ou 4 ; et/ou
- n représente 0, 1 ou 2 ; et/ou
- p représente 0 ;
à l'état de base ou de sel d'addition à un acide pharmaceutiquement acceptable, à l'état d'hydrates ou de solvats, ainsi que sous forme d'énantiomères, de diastéréoisomères et leur mélange.

7. Composés selon l'une quelconque des revendications 1 à 6, choisis parmi :
• Acide 1-(1,5-diméthyl-1H-pyrazole-3-carbonyl)-pipéridine-3-carboxylique-[4-(5-cyclohexyl-2-méthoxy-phényl)-thiazol-2-yl]-amide,
• Acide 1-(1-méthyl-1H-imidazole-2-carbonyl)-pipéridine-3-carboxylique-[4-(5-cyclohexyl-2-méthoxy-phényl)-thiazol-2-yl]-amide,
• Acide 1-(pyrimidine-4-carbonyl)-pipéridine-3-carboxylique-[4-(5-cyclohexyl-2-méthoxy-phényl)-thiazol-2-yl]-amide,
• Acide 1-(pyrazine-2-carbonyl)-pipéridine-3-carboxylique-[4-(5-cyclohexyl-2-éthoxy-phényl)-thiazol-2-yl]-amide,
• Acide 1-(1-oxy-pyridine-2-carbonyl)-pipéridine-3-carboxylique-[4-(5-cyclopentyl-2-méthoxy-phényl)-thiazol-2-yl]-amide,
• Acide 1-(2=hydroxy-pyridine-3-carbonyl)-pipéridine-3-carboxylique-[4-(5-cyclohexyl-2-méthoxy-phényl)-thiazol-2-yl]-amide,
• Acide 1-(1-oxy-pyridine-3-carbonyl)-pipéridine-3-carboxylique-[4-(5-cyclohexyl-2-méthoxy-phényl)-thiazol-2-yl]-amide,
• Acide 1-(5-chloro-1-méthyl-1H-pyrazole-4-carbonyl)-pipéridine-3-carboxylique-[4-(5-cyclohexyl-2-méthoxy-phényl)-thiazol-2-yl]-amide,
• Acide 1-(furan-2-carbonyl)-pipéridine-3-carboxylique-[4-(5-cyclohexyl-2-méthoxy-phényl)-thiazol-2-yl]-amide,
• Acide 1-(pyrazine-2-carbonyl)-pipéridine-4-carboxylique-[4-(5-cyclohexyl-2-méthoxy-phényl)-thiazol-2-yl]-amide,
• Acide 1-(pyrazine-2-carbonyl)-pipéridine-2-carboxylique-[4-(5-cyclohexyl-2-méthoxy-phényl)-thiazol-2-yl]-amide,
• Acide 1-(2-pyridin-2-yl-acétyl)-pipéridine-3-carboxylique-[4-(5-cyclohexyl-2-méthoxy-phényl)-thiazol-2-yl]-amide,
• Acide 1-(1-oxy-pyridine-2-carbonyl)-pipéridine-3-carboxylique-[4-(5-cyclohexyl-2-méthoxy-phényl)-thiazol-2-yl]-amide,
• Acide (S)-1-(1-oxy-pyridine-2-carbonyl)-pipéridine-3-carboxylique-[4-(5-cyclohexyl-2-méthoxy-phényl)-thiazol-2-yl]-amide.
• Acide 1-(pyrazine-2-carbonyl)-pipéridine-3-carboxylique-[4-(5-cyclohexyl-2-méthoxy-phényl)-thiazol-2-yl]-amide.
• Acide (S)-1-(pyrazine-2-carbonyl)-pipéridine-3-carboxylique-[4-(5-cyclohexyl-2-méthoxy-phényl)-thiazol-2-yl]-amide,
• Acide 1-(1-oxy-pyridine-2-carbonyl)-pipéridine-3-carboxylique-[4-(5-butyf-2-méthoxy-phényl)-thiazol-2-yl]-amide,
• Acide 1-(pyrazine-2-carbonyl)-pipéridine-3-carboxylique-[4-(5-cyclopentyl-2-méthoxy-phényl)-thiazol-2-yl]-amide,
• Acide (R)-1-(pyrazine-2-carbonyl)-pipéridine-3-carboxylique-[4-(5-cyclohexyl-2-méthoxy-phényl)-thiazol-2-ylj-amide,
• Acide 1-(1-Oxy-pyridine-2-carbonyl)-pyrrolidine-3-carboxylique-[4-(5-cyclohexyl-2-méthoxy-phényl)-thiazol-2-yl]-amide,
• Acide (R)-1-(1-Oxy-pyridine-2-carbonyl)-pyrrolidine-3-carboxylique-[4-(5-cyclohexyl-2-méthoxy-phényl)-thiazol-2-yl]-amide.

8. Procédé de préparation d'un composé de formule (1) selon l'une quelconque des revendications 1 à 7, **caractérisé en ce que** l'on fait réagir un composé de formule (V) qui suit : 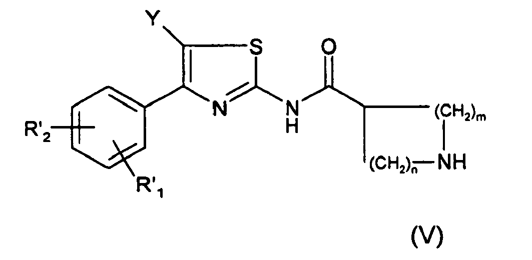 dans laquelle R'₁ et R'₂ représentent des précurseurs de R₁ et R₂ tels que définis dans la revendication 1 et Y, m et n sont tels que définis dans la revendication 1, avec un composé de formule (VI) qui suit : 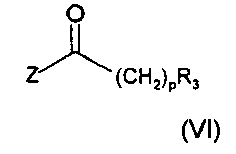 dans laquelle Z est un groupe partant et R₃ et p sont tels que définis dans la revendication 1, en présence d'un agent de couplage, à une température variant entre 0 °C et 150 °C.

9. Composé de formule (V'): 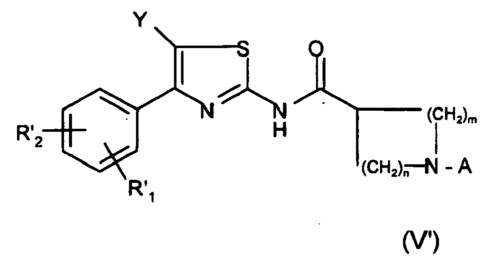 dans laquelle R'₁ et R'₂ représentent des précurseurs de R₁ et R₂ tels que définis dans la revendication 1 , Y, m et n sont tels que définis dans la revendication 1, et A représente un atome d'hydrogène ou un groupe protecteur(Gp) qui est le *tert-*butoxycarbonyle (Boc).

10. Médicament, **caractérisé en ce qu'**il comprend un composé de formule (I) selon l'une quelconque des revendications 1 à 7.

11. Composition pharmaceutique, **caractérisée en ce qu'**elle comprend un composé de formule (I) selon l'une quelconque des revendications 1 à 7, ainsi qu'au moins un excipient pharmaceutiquement acceptable.

12. Utilisation d'un composé de formule (I) selon l'une quelconque des revendications 1 à 7, pour la préparation d'un médicament destiné au traitement et à la prévention des maladies et des syndromes immuno-inflammatoires aigus ou chroniques, tels que l'athérosclérose, des maladies allergiques, des cancers ainsi que des maladies dans lesquels les processus angiogéniques sont impliqués.

13. Utilisation d'un composé de formule (I) selon l'une quelconque des revendications 1 à 7, pour la préparation d'un médicament destiné au traitement et à la prévention des maladies virales ou bactériennes, des pathologies cardiaques, de l'obésité, des cancers.

## Claims

1. Compound of the following formula (I) 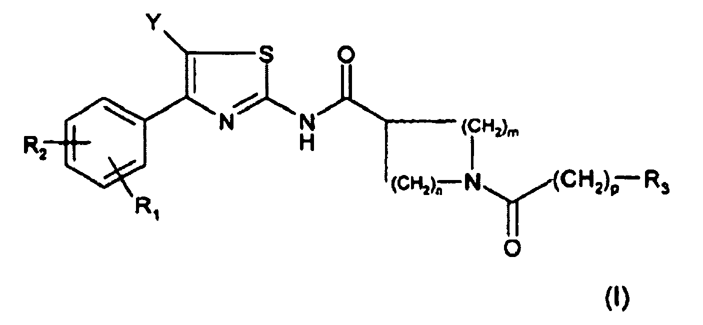 in which:
- R₁ represents a hydrogen or halogen atom, a (C₁-C₄)alkyl, trifluoro(C₁-C₄) alkyl, hydroxyl, (C₁-C₄) alkoxy, trifluoro (C₁-C₄) alkoxy, (C₃-C₁₀) cycloalkyl) (C₁-C₄)alkoxy, (C₃-C₁₀) cycloalkyloxy, allyloxy, (C₁-C₄) alkylthio group;
- R₂ represents a hydrogen or halogen atom, a hydroxyl, (C₁-C₄) alkyl, trifluoro (C₁-C₄) alkyl, perfluoro (C₁-C₄) alkyl, (C₃-C₁₀) cycloalkyl, (C₁-C₄) alkoxy, (C₃-C₁₀) cycloalkyl (C₁-C₄) alkoxy, (C₃-C₁₀) cycloalkyloxy, allyloxy, (C₃-C₈) cycloalkyl (C₁-C₄) alkyl group;
- Y represents a hydrogen atom or a halogen;
- R₃ represents:
• a phenyl group substituted by one or more halogen atoms and/or by one or more groups:
(C₁-C₆) alkyl
(C₁-C₆) alkoxy,
-NO₂,
cyano
-COR_{4,}
-SO₂R₄,
-CO₂R₄, in which R₄ represents a (C₁-C₄)alkyl group,
-(CH₂)qNR₅R₆, in which R₅ and R₆ independently of each other represent a hydrogen atom, a (C₁-C₄)alkyl group or R₅ and R₆ together with the nitrogen atom to which they are bonded form a cycloalkyl group having 5 or 6 chain members and q represents 0, 1 or 2;
• a heterocyclic group optionally substituted by one or more halogens and/or by one or more groups:
(C₁-C₄) alkyl,
hydroxyl,
(C₁-C₄) alkoxy,
cyano,
morpholine,
trifluoro (C₁-C₄) alkyl,
-COR₄,
-SO₂R₄, in which R₄ is as defined above,
-(CH₂)qNR₅R₆, in which R₅, R₆ and q are as defined above,
phenyl,
pyridine,
-SCH₃;
the nitrogen atom or atoms of the heterocyclic group being optionally substituted by a (C₁-C₄)alkyl group,
the nitrogen atom or atoms of the heterocyclic group optionally being in their N-oxide form;
• a heterobicyclic group optionally substituted by one or more halogens and/or by one or more hydroxyl groups, (C₁-C₄) alkyl or (C₁-C₄) alkoxy;
- m represents 2, 3 or 4;
- n represents 0, 1 or 2;
- p represents 0, 1, 2 or 3,
in the form of a base or an addition salt with a pharmaceutically acceptable acid, in the form of hydrates or solvates, and in the form of enantiomers, diastereoisomers and their mixture.

2. Compound according to claim 1, of the formula (I), in which:
- R₁ and R₂ are as defined in claim 1;
- Y represents a hydrogen atom or a halogen;
- R₃ represents:
• a phenyl group substituted by one or more halogen atoms and/or by one or more groups:
(C₁-C₆) alkyl,
(C₁-C₆) alkoxy,
-NO₂,
cyano,
-COR₄,
-SO₂R₄, in which R₄ represents a (C₁-C₄) alkyl group,
-(CH₂)qNR₅R₆, in which R₅, R₆ and q are as defined in claim 1;
• a heterocyclic group optionally substituted by one or more halogens and/or by one or more groups:
(C₁-C₄) alkyl,
hydroxyl,
(C₁-C₄) alkoxy,
cyano,
morpholine,
trifluoro (C₁-C₄) alkyl,
-COR₄,
-SO₂R₄, in which R₄ is as defined in claim 1,
-(CH₂)_{q}NR₅R₆, in which R₅, R₆ and q are as defined in claim 1,
phenyl,
pyridine,
-SCH₃;
the nitrogen atom or atoms of the heterocyclic group being optionally substituted by a (C₁-C₄)alkyl group,
the nitrogen atom or atoms of the heterocyclic group optionally being in their N-oxide form;
• a heterobicyclic group optionally substituted by one or more halogens and/or by one or more hydroxyl groups, (C₁-C₄) alkyl or (C₁-C₄) alkoxy;
- m represents 3;
- n represents 1;
- p represents 0;
in the form of a base or an addition salt with a pharmaceutically acceptable acid, in the form of hydrates or solvates, and in the form of enantiomers, diastereoisomers and their mixture.

3. Compound according to claim 1, of the formula (I), in which:
- R₁ and R₂ are as defined in claim 1;
- Y is as defined in claim 1;
- R₃ represents:
• a phenyl group substituted by one or more halogen atoms and/or by one or more groups:
(C₁-C₆) alkyl,
(C₁-C₆) alkoxy,
-NO₂,
cyano,
-COR₄,
-SO₂R₄,
-CO₂R₄, in which R₄ represents a (C₁-C₄) alkyl group,
-(CH₂)_{q}NR₅R₆, in which R₅, R₆ and q are as defined in claim 1;
• a pyridinyl, pyrazinyl, pyrimidinyl, pyridazinyl, pyrazolyl, imidazolyl, thiazolyl, isothiazolyl, oxazolyl, isooxazolyl, oxadiazolyl, thiophenyl, pyrrolyl, furanyl group, the said abovementioned groups being optionally substituted by one or more halogens and/or by one or more groups:
(C₁-C₄) alkyl,
hydroxyl,
(C₁-C₄) alkoxy,
cyano,
morpholine,
trifluoro (C₁-C₄)alkyl,
-COR₄,
-SO₂R₄, in which R₄ is as defined in claim 1,
-(CH₂)_{q}NR₅R₆, in which R₅, R₆ and q are as defined in claim 1,
phenyl,
pyridine,
-SCH₃;
the nitrogen atom or atoms of the said abovementioned groups being optionally substituted by a (C₁-C₄)alkyl group,
the nitrogen atom or atoms of the said abovementioned groups optionally being in their N-oxide form;
• a quinoxaline, quinoline, isoquinoline, indole, isoindole, indoline, indazole, benzothiazole, benzimidazole, benzoxazole group, the said abovementioned groups being optionally substituted by one or more halogens and/or by one or more hydroxyl groups, (C₁-C₄)alkyl or (C₁-C₄) alkoxy;
- m represents 3;
- n represents 1;
- p represents 0;
in the form of a base or an addition salt with a pharmaceutically acceptable acid, in the form of hydrates or solvates, and in the form of enantiomers, diastereoisomers and their mixture.

4. Compound according to claim 1, of the formula (I), in which:
- R₁ and R₂ are as defined in claim 1;
- Y represents a hydrogen atom or a halogen;
- R₃ represents:
• a phenyl group substituted by one or more halogen atoms and/or by one or more groups:
(C₁-C₆) alkyl,
(C₁-C₆) alkoxy,
-NO₂,
cyano,
-COR₄,
-SO₂R₄,
-CO₂R₄, in which R₄ represents a (C₁-C₄) alkyl group,
-(CH₂)_{q}NR₅R₆, in which R₅, R₆ and q are as defined in claim 1;
• a heterocyclic group optionally substituted by one or more halogens and/or by one or more groups:
(C₁-C₄)alkyl,
hydroxyl,
(C₁-C₄)alkoxy,
cyano,
morpholine,
trifluoro (C₁-C₄) alkyl,
-COR₄,
-SO₂R₄, in which R₄ is as defined in claim 1,
-(CH₂)_{q}NR₅R₆, in which R₅, R₆ and q are as defined in claim 1,
phenyl,
pyridine,
-SCH₃ ;
the nitrogen atom or atoms of the heterocyclic group being optionally substituted by a (C₁-C₄)alkyl group,
the nitrogen atom or atoms of the heterocyclic group optionally being in their N-oxide form;
• a heterobicyclic group optionally substituted by one or more halogens and/or by one or more hydroxyl groups, (C₁-C₄) alkyl or (C₁-C₄) alkoxy;
- m represents 2 or 4; and/or
- n represents 0, 1 or 2; and/or
- p represents 0, 1 or 2;
in the form of a base or an addition salt with a pharmaceutically acceptable acid, in the form of hydrates or solvates, and in the form of enantiomers, diastereoisomers and their mixture.

5. Compound according to claim 1, of the formula (I), in which:
- R₁ and R₂ are as defined in claim 1;
- Y represents a hydrogen atom or a halogen;
- R₃ represents:
• a phenyl group substituted by one or more halogen atoms and/or by one or more groups:
(C₁-C₆)alkyl,
(C₁-C₆)alkoxy,
-NO₂,
cyano,
-COR₄,
-SO₂R₄,
-CO₂R₄ in which R₄ represents a (C₁-C₄)alkyl group,
- (CH₂)_{q}NR₅R₆, in which R₅, R₆ and q are as defined in claim 1;
• a pyridinyl, pyrazinyl, pyrimidinyl, pyridazinyl, pyrazolyl, imidazolyl, thiazolyl, isothiazolyl, oxazolyl, isooxazolyl, oxadiazolyl, thiophenyl, pyrrolyl, furanyl group, the said abovementioned groups being optionally substituted by one or more halogens and/or by one or more groups:
(C₁-C₄) alkyl,
hydroxyl,
(C₁-C₄) alkoxy,
cyano,
morpholine,
trifluoro(C₁-C₄) alkyl,
-COR₄ ,
-SO₂R₄, in which R₄ is as defined in claim 1,
-(CH₂)_{q}NR₅R₆, in which R₅, R₆ and q are as defined in claim 1,
phenyl,
pyridine,
-SCH₃;
the nitrogen atom or atoms of the said abovementioned groups being optionally substituted by a (C₁-C₄)alkyl group,
the nitrogen atom or atoms of the said abovementioned groups optionally being in their N-oxide form;
• a quinoxaline, quinoline, isoquinoline, indole, isoindole, indoline, indazole, benzothiazole, benzimidazole, benzoxazole group, the said abovementioned groups being optionally substituted by one or more halogens and/or by one or more hydroxyl groups, (C₁-C₄) alkyl or (C₁-C₄) alkoxy;
- m represents 2 or 4; and/or
- n represents 0, 1 or 2; and/or
- p represents 0, 1 or 2;
in the form of a base or an addition salt with a pharmaceutically acceptable acid, in the form of hydrates or solvates, and in the form of enantiomers, diastereoisomers and their mixture.

6. Compound according to claim 1, of the formula (I), in which:
- R₁ and R₂ are as defined in claim 1;
- Y represents a hydrogen atom or a halogen;
- R₃ represents:
• a phenyl group substituted by one or more halogen atoms and/or by one or more groups:
(C₁-C₆) alkyl,
(C₁-C₆) alkoxy,
-NO₂,
cyano,
-COR₄,
-SO₂R₄,
-CO₂R₄, in which R₄ represents a (C₁-C₄) alkyl group,
-(CH₂)_{q}NR₅R₆, in which R₅, R₆ and q are as defined in claim 1;
• a pyridinyl, pyrazinyl, pyrimidinyl, pyridazinyl, pyrazolyl, imidazolyl, thiazolyl, isothiazolyl, oxazolyl, isooxazolyl, oxadiazolyl, thiophenyl, pyrrolyl, furanyl group, the said abovementioned groups being optionally substituted by one or more halogens and/or by one or more groups:
(C₁-C₄) alkyl,
hydroxyl,
(C₁-C₄) alkoxy,
cyano,
morpholine,
trifluoro (C₁-C₄) alkyl,
-COR₄,
-SO₂R₄, in which R₄ is as defined in claim 1,
-(CH₂)_{q}NR₅R₆, in which R₅, R₆ and q are as defined in claim 1,
phenyl,
pyridine,
-SCH₃;
the nitrogen atom or atoms of the said abovementioned groups being optionally substituted by a (C₁-C₄)alkyl group,
the nitrogen atom or atoms of the said abovementioned groups optionally being in their N-oxide form;
• a quinoxaline, quinoline, isoquinoline, indole, isoindole, indoline, indazole, benzothiazole, benzimidazole, benzoxazole group, the said abovementioned groups being optionally substituted by one or more halogens and/or by one or more hydroxyl groups, (C₁-C₄) alkyl or (C₁-C₄) alkoxy;
- m represents 2 or 4; and/or
- n represents 0, 1 or 2; and/or
- p represents 0;
in the form of a base or an addition salt with a pharmaceutically acceptable acid, in the form of hydrates or solvates, and in the form of enantiomers, diastereoisomers and their mixture.

7. Compounds according to any one of claims 1 to 6, chosen from:
• 1-(1,5-dimethyl-1H-pyrazole-3-carbonyl)-piperidine-3-carboxylic acid [4-(5-cyclohexyl-2-methoxy-phenyl)-thiazol-2-yl]-amide,
• 1-(1-methyl-1H-imidazole-2-carbonyl)-piperidine-3-carboxylic acid [4-(5-cyclohexyl-2-methoxy-phenyl)-thiazol-2-yl]-amide,
• 1-(pyrimidine-4-carbonyl)-piperidine-3-carboxylic acid [4-(5-cyclohexyl-2-methoxy-phenyl)-thiazol-2-yl]-amide,
• 1-(pyrazine-2-carbonyl)-piperidine-3-carboxylic acid [4-(5-cyclohexyl-2-ethoxy-phenyl)-thiazol-2-yl]-amide,
• 1-(1-oxy-pyridine-2-carbonyl)-piperidine-3-carboxylic acid [4-(5-cyclopentyl-2-methoxy-phenyl)-thiazol-2-yl]-amide,
• 1-(2-hydroxy-pyridine-3-carbonyl)-piperidine-3-carboxylic acid [4-(5-cyclohexyl-2-methoxy-phenyl)-thiazol-2-yl]-amide,
• 1-(1-oxy-pyridine-3-carbonyl)-piperidine-3-carboxylic acid [4-(5-cyclohexyl-2-methoxy-phenyl)-thiazol-2-yl]-amide,
• 1-(5-chloro-1-methyl-1H-pyrazole-4-carbonyl)-piperidine-3-carboxylic acid [4-(5-cyclohexyl-2-methoxy-phenyl)-thiazol-2-yl]-amide,
• 1-(furan-2-carbonyl)-piperidine-3-carboxylic acid [4-(5-cyclohexyl-2-methoxy-phenyl)-thiazol-2-yl]-amide,
• 1-(pyrazine-2-carbonyl)-piperidine-4-carboxylic acid [4-(5-cyclohexyl-2-methoxy-phenyl)-thiazol-2-yl]-amide,
• 1-(pyrazine-2-carbonyl)-piperidine-2-carboxylic acid [4-(5-cyclohexyl-2-methoxy-phenyl)-thiazol-2-yl]-amide,
• 1-(2-pyridin-2-yl-acetyl)-piperidine-3-carboxylic acid [4-(5-cyclohexyl-2-methoxy-phenyl)-thiazol-2-yl]-amide,
• 1-(1-oxy-pyridine-2-carbonyl)-piperidine-3-carboxylic acid [4-(5-cyclohexyl-2-methoxy-phenyl)-thiazol-2-yl]-amide,
• (S)-1-(1-oxy-pyridine-2-carbonyl)-piperidine-3-carboxylic acid [4-(5-cyclohexyl-2-methoxy-phenyl)-thiazol-2-yl]-amide,
• 1-(pyrazine-2-carbonyl)-piperidine-3-carboxylic acid [4-(5-cyclohexyl-2-methoxy-phenyl)-thiazol-2-yl]-amide,
• (S)1-(pyrazine-2-carbonyl)-piperidine-3-carboxylic acid [4-(5-cyclohexyl-2-methoxy-phenyl)-thiazol-2-yl]-amide,
• 1-(1-oxy-pyridine-2-carbonyl)-piperidine-3-carboxylic acid [4-(5-butyl-2-methoxy-phenyl)-thiazol-2-yl]-amide,
• 1-(pyrazine-2-carbonyl)-piperidine-3-carboxylic acid [4-(5-cyclopentyl-2-methoxy-phenyl)-thiazol-2-yl]-amide,
• (R)-1-(pyrazine-2-carbonyl)-piperidine-3-carboxylic acid [4-(5-cyclohexyl-2-methoxy-phenyl)-thiazol-2-yl]-amide,
• 1-(1-oxy-pyridine-2-carbonyl)-pyrrolidine-3-carboxylic acid [4-(5-cyclohexyl-2-methoxy-phenyl)-thiazol-2-yl]-amide,
• (R)-1-(1-oxy-pyridine-2-carbonyl)-pyrrolidine-3-carboxylic acid [4-(5-cyclohexyl-2-methoxy-phenyl)-thiazol-2-yl]-amide,

8. Process for the preparation of a compound of the formula (I) according to any one of claims 1 to 7, **characterized in that** a compound of the following formula (V): 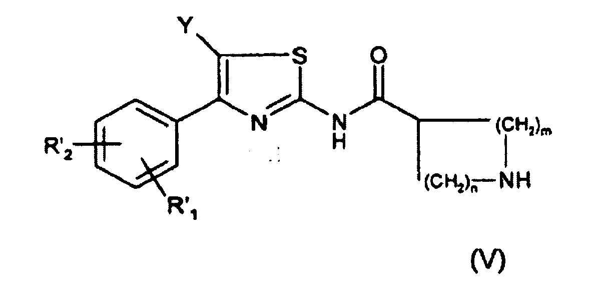 in which R'₁ and R'₂ represent the precursors of R₁ and R₂ as defined in claim 1 and Y, m and n are as defined in claim 1, is reacted with a compound of the following formula (VI): 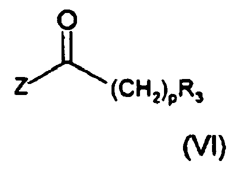 in which Z is a leaving group and R₃ and p are as defined in claim 1, in the presence of a coupling agent, at a temperature ranging between 0 °c and 150 °C.

9. Compound of the formula (V'): 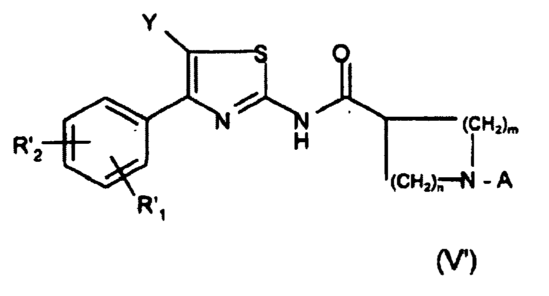 in which R'₁ and R'₂ represent precursors of R₁ and R₂ as defined in claim 1, Y, m and n are as defined in claim 1, and A represents a hydrogen atom or a protective group (Gp), which is tert-butoxycarbonyl (Boc).

10. Medicament, **characterized in that** it contains a compound of the formula (I) according to any one of claims 1 to 7.

11. Pharmaceutical composition, **characterized in that** it contains a compound of the formula (I) according to any one of claims 1 to 7, and at least one pharmaceutically acceptable excipient.

12. Use of a compound of the formula (I) according to any one of claims 1 to 7 for the preparation of a medicament for treatment and prevention of acute or chronic immunoinflammatory diseases and syndromes, such as atherosclerosis, allergic diseases, cancers and diseases involving angiogenic processes.

13. Use of a compound of the formula (I) according to any one of claims 1 to 7 for the preparation of a medicament for treatment and prevention of viral or bacterial diseases, cardiopathologies, obesity, cancers.

## Patentansprüche

1. Verbindung gemäß der folgenden Formel (I) 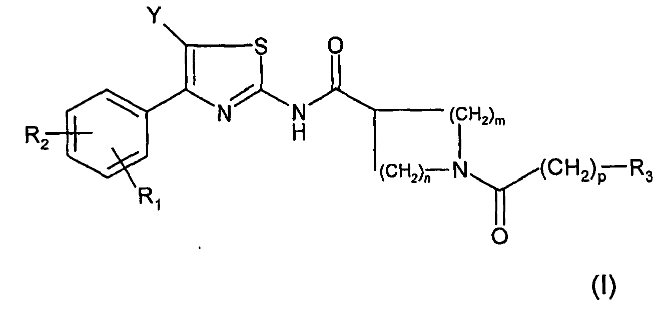 in welcher:
- R₁ ein Wasserstoff- oder Halogenatom, eine (C₁-C₄)alkyl, trifluor(C₁-C₄) alkyl, hydroxyl, (C₁-C₄)alkoxy, trifluor(C₁-C₄)alkoxy, (C₃-C₁₀)cycloalkyl(C₁-C₄)alkoxy, (C₃-C₁₀)cycloalkyloxy, allyloxy, (C₁-C₄)alkylthio Gruppe ist;
- R₂ ein Wasserstoff- oder Halogenatom, eine Hydroxyl, (C₁-C₄)alkyl, trifluor(C₁-C₄)alkyl, perfluor(C₁-C₄)alkyl, (C₃-C₁₀)cycloalkyl, (C₁-C₄)alkoxy, (C₃-C10) cycloalkyl(C₁-C₄)alkoxy, (C₃-C₁₀)cycloalkyloxy, allyloxy, (C₃-C₈)cycloalkyl(C₁-C₄)alkyl Gruppe ist;
- Y ein Wasserstoff- oder Halogenatom ist;
- R₃
• eine Phenylgruppe, substituiert durch ein oder mehrere Halogenatome und/oder durch eine oder mehrere Gruppen
(C₁-C₆)alkyl,
(C₁-C₈)alkoxy,
-NO₂,
cyano,
-COR₄,
-SO₂R₄,
-CO₂R₄, worin R₄ eine (C₁-C₄)alkyl Gruppe ist,
-(CH₂)_{q}NR₅R₆, worin R₅ und R₆ unabhängig voneinander ein Wasserstoffatom, eine (C₁-C₄)alkyl Gruppe darstellen oder R₅ und R₆ bilden zusammen mit einem Stickstoffatom, mit welchem sie verbunden sind, eine cycloalkyl Gruppe mit 5 oder 6 Gliedern und q ist 0, 1 oder 2;
• eine heterozyklische Gruppe gegebenenfalls substituiert durch eine oder mehrere Halogene und/oder durch ein oder mehrere Gruppen:
(C₁-C₄)alkyl,
hydroxyl,
(C₁-C₄)alkoxy,
Cyano,
Morpholin,
Trifluor(C₁-C₄)alkyl,
-COR₄,
-SO₂R₄, worin R₄ wie oben definiert ist,
-(CH₂)_{q}NR₅R₆, worin R₅, R₆ und q wie oben definiert sind, phenyl,
pyridin,
-SCH₃;
das oder die Stickstoffatome der heterozyklischen Gruppe gegebenenfalls durch eine (C₁-C₄) alkyl Gruppe substituiert sind,
das oder die Stickstoffatome der heterozyklischen Gruppe gegebenenfalls in ihrer N-oxide Form vorliegen;
• eine heterozyklische Gruppe gegebenenfalls substituiert durch ein oder mehrere Halogene und/oder durch eine oder mehrere Hydroxyl-, (C₁-C₄)alkyl oder (C₁-C₄)alkoxy Gruppen, ist;
- m ist 2, 3 oder 4;
- n ist 0, 1 oder 2;
- p ist 0, 1, 2 oder 3;
als Base oder Additionssalz in einer pharmazeutisch brauchbaren Säure, als Hydrate oder Solvate, sowie in Form von Enantiomeren, Diastereomeren und ihren Gemischen.

2. Verbindung gemäß Anspruch 1, der Formel (I) in welcher:
- R₁ und R₂ wie im Anspruch 1 definiert sind;
- Y ein Wasserstoff- oder Halogenatom ist;
- R₃
• eine Phenylgruppe, durch ein oder mehrere Halogenatome und/oder durch ein oder mehrere Gruppen:
(C₁-C₆)alkyl,
(C₁-C₆)alkoxy,
-NO₂,
Cyano,
-COR₄.
-SO₂R₄,
-CO₂R₄, worin R₄ eine (C₁-C₄) alkyl Gruppe ist,
-(CH₂)_{q}NR₅R₆, worin R₅, R₆ und q wie in Anspruch 1 definiert sind, substituiert;
• eine heterozyklische Gruppe gegebenenfalls durch ein oder mehrere Halogene und/oder durch eine oder mehrere Gruppen:
(C₁-C₄)alkyl,
Hydroxyl,
(C₁-C₄) alkoxy,
Cyano,
Morpholin,
Trifluor(C₁-C₄)alkyl,
-COR₄,
-SO₂R₄, worin R₄ wie in Anspruch 1 definiert ist,
-(CH₂)_{q}NR₅R₆, worin R₅, R₆ und q wie in Anspruch 1 definiert sind,
Phenyl,
Pyridin,
-SCH₃, substituiert;
das oder die Stickstoffatome der heterozyklischen Gruppe gegebenenfalls durch eine (C₁-C₄)alkyl Gruppe substituiert sind,
das oder die Stickstoffatome der heterozyklischen Gruppe gegebenenfalls in ihrer N-oxide Form vorliegen;
• eine heterozyklische Gruppe gegebenenfalls durch ein oder mehrere Halogene und/oder durch eine oder mehrere Hydroxyl-, (C₁-C₄)alkyl oder (C₁-C₄)alkoxy Gruppen substituiert, ist;
- m ist 3;
- n ist 1;
- p ist 0;
als Base oder Additionssalz in einer pharmazeutisch akzeptablen Säure, als Hydrate oder Solvate, sowie in Form von Enantiomeren, Diastereomeren und ihren Gemischen.

3. Verbindung gemäß Anspruch 1, der Formel (I) in welcher:
- R₁ und R₂ wie im Anspruch 1 definiert sind;
- Y ein Wasserstoff- oder Halogenatom ist;
- R₃
• eine Phenylgruppe, durch ein oder mehrere Halogenatome und/oder durch ein oder mehrere Gruppen:
(C₁-C₆)alkyl,
(C₁-C₆)alkoxy,
-NO₂,
Cyano,
-COR₄,
-SO₂R₄,
-CO₂R₄, worin R₄ eine (C₁-C₄)alkyl Gruppe ist,
-(CH₂)_{q}NR₅R₆, worin R₅, R₆ und q wie in Anspruch 1 definiert sind, substituiert;
• eine Pyridinyl-, Pyrazinyl-, Pyrimidinyl-, Pyridazinyl-, Pyrazolyl-, Imidazolyl-, Thiazolyi-, Isothiazolyi-, Oxazolyl-, Isooxazolyl-, Oxadiazolyl-, Thiophenyl-, Pyrrolyl-, Furanylgruppe, die besagten vorangehenden Gruppen gegebenenfalls durch ein oder mehrere Halogene und/oder durch eine oder mehrere Gruppen:
(C₁-C₄)alkyl,
Hydroxyl,
(C₁-C₄)alkoxy,
Cyano,
Morpholin,
Trifluor(C₁-C₄)alkyl,
-COR₄,
-SO₂R₄, worin R₄ wie in Anspruch 1 definiert ist,
-(CH₂)_{q}NR₅R₆, worin R₅, R₆ und q wie in Anspruch 1 definiert sind,
Phenyl,
Pyridin,
-SCH₃, substituiert;
das oder die Stickstoffatome der der besagten, obenstehend genannten Gruppe gegebenenfalls durch eine (C₁-C₄)alkyl Gruppe substituiert sind,
das oder die Stickstoffatome der besagten, obenstehend genannten Gruppe gegebenenfalls in ihrer N-oxide Form vorliegen;
• eine Quinoxalin-, Quinolin-, Isoquinolin-, Indol-, Isoindol-, Indolin-, Indazol-, Benzothiazol-, Benzimidazol-, Benzoxazolgruppe, die besagten vorangehenden Gruppen gegebenenfalls durch ein oder mehrere Halogene und/oder durch eine oder mehrere Hydroxyl-, (C₁-C₄)alkyl oder (C₁-C₄)alkoxy Gruppen substituiert, ist;
- m ist 3;
- n ist 1;
- p ist 0;
als Base oder Additionssalz in einer pharmazeutisch akzeptablen Säure, als Hydrate oder Solvate, sowie in Form von Enantiomeren, Diastereomeren und ihren Gemischen.

4. Verbindung gemäß Anspruch 1, der Formel (I) in welcher:
- R₁ und R₂ wie im Anspruch 1 definiert sind;
- Y ein Wasserstoff- oder Halogenatom ist;
- R₃
• eine Phenylgruppe, durch ein oder mehrere Halogenatome und/oder durch ein oder mehrere Gruppen:
(C₁-C₆)alkyl,
(C₁-C₆)alkoxy,
-NO₂,
Cyano,
-COR₄,
-SO₂R₄,
-CO₂R₄, worin R₄ eine (C₁-C₄)alkyl Gruppe ist,
-(CH₂)_{q}NR₅R₆, worin R₅, R₆ und q wie in Anspruch 1 definiert sind, substituiert;
• eine heterozyklische Gruppe gegebenenfalls durch ein oder mehrere Halogene und/oder durch eine oder mehrere Gruppen:
(C₁-C₄)alkyl,
Hydroxyl,
(C₁-C₄)alkoxy,
Cyano,
Morpholin,
Trifluor(C₁-C₄)alkyl,
-COR₄,
-SO₂R₄, worin R₄ wie in Anspruch 1 definiert ist,
-(CH₂)_{q}NR₅R₆, worin R₅, R₆ und q wie in Anspruch 1 definiert sind,
Phenyl,
Pyridin,
-SCH₃, substituiert;
das oder die Stickstoffatome der heterozyklischen Gruppe gegebenenfalls durch eine (C₁-C₄)alkyl Gruppe substituiert sind,
das oder die Stickstoffatome der heterozyklischen Gruppe gegebenenfalls in ihrer N-oxide Form vorliegen;
• eine heterobizyklische Gruppe gegebenenfalls durch ein oder mehrere Halogene und/oder durch eine oder mehrere Hydroxyl-, (C₁-C₄)alkyl oder (C₁-C₄)alkoxy Gruppen substituiert, ist;
- m ist 2 oder 4; und/oder
- n ist 0, 1 oder 2; und/oder
- p ist 0, 1 oder 2;
als Base oder Additionssalz in einer pharmazeutisch akzeptablen Säure, als Hydrate oder Solvate, sowie in Form von Enantiomeren, Diastereomeren und ihren Gemischen.

5. Verbindung gemäß Anspruch 1, der Formel (I) in welcher:
- R₁ und R₂ wie im Anspruch 1 definiert sind;
- Y ein Wasserstoff- oder Halogenatom ist;
- R₃
• eine Phenylgruppe, durch ein oder mehrere Halogenatome und/oder durch ein oder mehrere Gruppen:
(C₁-C₆)alkyl,
(C₁-C₆)alkoxy,
-NO₂,
Cyano,
-COR₄,
-SO₂R₄,
-CO₂R₄, worin R₄ eine (C₁-C₄)alkyl Gruppe ist,
-(CH₂)_{q}NR₅R₆, worin R₅, R₆ und q wie in Anspruch 1 definiert sind, substituiert;
• eine Pyridinyl-, Pyrazinyl-, Pyrimidinyl-, Pyridazinyl-, Pyrazolyl-, Imidazolyl-, Thiazolyl-, Isothiazolyl-, Oxazolyl-, Isooxazolyl-, Oxadiazolyl-, Thiophenyl-, Pyrrolyl-, Furanylgruppe, die besagten vorangehenden Gruppen gegebenenfalls durch ein oder mehrere Halogene und/oder durch eine oder mehrere Gruppen:
(C₁-C₄)alkyl,
Hydroxyl,
(C₁-C₄)alkoxy,
Cyano,
Morpholin,
Trifluor(C₁-C₄)alkyl,
-COR₄,
-SO₂R₄, worin R₄ wie in Anspruch 1 definiert ist,
-(CH₂)_{q}NR₅R₆, worin R₅, R₆ und q wie in Anspruch 1 definiert sind,
Phenyl,
Pyridin,
-SCH₃, substituiert;
das oder die Stickstoffatome der der besagten, obenstehend genannten Gruppe gegebenenfalls durch eine (C₁-C₄)alkyl Gruppe substituiert sind,
das oder die Stickstoffatome der besagten, obenstehend genannten Gruppe gegebenenfalls in ihrer N-oxide Form vorliegen;
• eine Quinoxalin-, Quinolin-, Isoquinolin-, Indol-, Isoindol-, Indolin-, Indazol-, Benzothiazol-, Benzimidazol-, Benzoxazolgruppe, die besagten vorangehenden Gruppen gegebenenfalls durch ein oder mehrere Halogene und/oder durch eine oder mehrere Hydroxyl-, (C₁-C₄)alkyl oder (C₁-C₄)alkoxy Gruppen substituiert, ist;
- m ist 2 oder 4; und/oder
- n ist 0, 1 oder 2; und/oder
- p ist 0, 1 oder 2;
als Base oder Additionssalz in einer pharmazeutisch akzeptablen Säure, als Hydrate oder Solvate, sowie in Form von Enantiomeren, Diastereomeren und ihren Gemischen.

6. Verbindung gemäß Anspruch 1, der Formel (I) in welcher:
- R₁ und R₂ wie im Anspruch 1 definiert sind;
- Y ein Wasserstoff- oder Halogenatom ist;
- R₃
• eine Phenylgruppe, durch ein oder mehrere Halogenatome und/oder durch ein oder mehrere Gruppen:
(C₁-C₆)alkyl,
(C₁-C₆)alkoxy,
-NO₂,
Cyano,
-COR₄,
-SO₂R₄,
-CO₂R₄, worin R₄ eine (C₁-C₄)alkyl Gruppe ist,
-(CH₂)_{q}NR₅R₆, worin R₅, R₆ und q wie in Anspruch 1 definiert sind, substituiert;
• eine Pyridinyl-, Pyrazinyl-, Pyrimidinyl-, Pyridazinyl-, Pyrazolyl-, Imidazolyl-, Thiazolyl-, Isothiazolyl-, Oxazolyl-, Isooxazolyl-, Oxadiazolyl-, Thiophenyl-, Pyrrolyl-, Furanylgruppe, die besagten vorangehenden Gruppen gegebenenfalls durch ein oder mehrere Halogene und/oder durch eine oder mehrere Gruppen:
(C₁-C₄)alkyl,
Hydroxyl,
(C₁-C₄)alkoxy,
Cyano,
Morpholin,
Trifluor(C₁-C₄)alkyl,
-COR₄,
-SO₂R₄, worin R₄ wie in Anspruch 1 definiert ist,
-(CH₂)_{q}NR₅R₆, worin R₅, R₆ und q wie in Anspruch 1 definiert sind,
Phenyl,
Pyridin,
-SCH₃, substituiert;
das oder die Stickstoffatome der der besagten, obenstehend genannten Gruppe gegebenenfalls durch eine (C₁-C₄)alkyl Gruppe substituiert sind,
das oder die Stickstoffatome der besagten, obenstehend genannten Gruppe gegebenenfalls in ihrer N-oxide Form vorliegen;
• eine Quinoxalin-, Quinolin-, Isoquinolin-, Indol-, Isoindol-, Indolin-, Indazol-, Benzothiazol-, Benzimidazol-, Benzoxazolgruppe, die besagten vorangehenden Gruppen fallweise durch ein oder mehrere Halogene und/oder durch eine oder mehrere Hydroxyl-, (C₁-C₄)alkyl oder (C₁-C₄)alkoxy Gruppen substituiert, ist;
- m ist 2 oder 4; und/oder
- n ist 0, 1 oder 2; und/oder
- p ist 0;
als Base oder Additionssalz in einer pharmazeutisch akzeptablen Säure, als Hydrate oder Solvate, sowie in Form von Enantiomeren, Diastereomeren und ihren Gemischen.

7. Verbindung nach einem der Ansprüche 1 bis 6, ausgewählt aus
• 1-(1,5-dimethyl-1H-pyrazol-3-carbonyl)-piperidin-3-carboxyl[4-(5-cyclohexyl-2-methoxy-phenyl)-thiazol-2-yl]-amidsäure,
• 1-(1-methyl-1H-imidazole-2-carbonyl)-piperidin-3-carboxyl-[4(5-cyclohexyl-2-methoxy-phenyl)-thiazol-2-yl]-amidsäure,
• 1-(pyrimidin-4-carbonyl)-piperidin-3-carboxyl-[4-(5-cyclohexyl-2-methoxy-phenyl)-thiazol-2-yl]-amidsäure,
• 1-(pyrazin-2-carbonyl)-piperidin-3-carboxyl-[4-(5-cyclohexyl-2-ethoxyphenyl)-thiazol-2-yl]-amidsäure,
• 1-(1-oxy-pyridin-2-carbonyl)-piperidin-3-carboxyl-[4-(5-cyclopentyl-2-methoxy-phenyl)-thiazol-2-yl]-amidsäure,
• 1-(2-hydroxy-pyridin-3-carbonyl)-piperidin-3-carboxyl-[4-(5-cyclohexyl-2-methoxy-phenyl)-thiazol-2-yl]-amidsäure,
• 1-(1-oxy-pyridin-3-carbonyl)-piperidin-3-carboxyl-[4-(5-cyclohexyl-2-methoxy-phenyl)-thiazol-2-yl]-amidsäure,
• 1-(5-chloro-1-methyl-1H-pyrazol-4-carbonyl)-piperidin-3-carboxyl-[4-(5-cyclohexyl-2-methoxy-phenyl)-thiazol-2-yl]-amidsäure,
• 1-(furan-2-carbonyl)-piperidine-3-carboxyl-[4-(5-cyclohexyl-2-methoxy-phenyl)-thiazol-2-yl]-amidsäure,
• 1-(pyrazin-2-carbonyl)-piperidin-4-carboxyl-[4-(5-cyclohexyl-2-methoxy-phenyl)-thiazol-2-yl]-amidsäure,
• 1-(pyrazin-2-carbonyl)-piperidin-2-carboxyl-[4-(5-cyclohexyl-2-methoxy-phenyl)-thiazol-2-yl]-amidsäure,
• 1-(2-pyridin-2-yl-acetyl)-piperidin-3-carboxyl-[4-(5-cyclohexyl-2-methoxy-phenyl)-thiazol-2-yl]-amidsäure,
• 1-(1-oxy-pyridin-2-carbonyl)-piperidin-3-carboxyl-[4-(5-cyclohexyl-2-methoxy-phenyl)-thiazol-2-yl]-amidsäure,
• (S)-1-(1-oxy-pyridin-2-carbonyl)-piperidin-3-carboxyl-[4-(5-cyclohexyl-2-methoxy-phenyl)-thiazol-2-yl]-amidsäure,
• 1-(pyrazin-2-carbonyl)-piperidin-3-carboxyl-[4-(5-cyclohexyl-2-methoxy-phenyl)-thiazol-2-yl]-amidsäure,
• (S)-1-(pyrazin-2-carbonyl)-piperidin-3-carboxyl-[4-(5-cyclohexyl-2-methoxy-phenyl)-thiazol-2-yl]-amidsäure,
• 1-(1-oxy-pyridin-2-carbonyl)-piperidin-3-carboxyl-[4-(5-butyl-2-methoxy-phenyl)-thiazol-2-yl]-amidsäure,
• 1-(pyrazin-2-carbonyl)-piperidin-3-carboxy-[4-(5-cyclopentyl-2-methoxy-phenyl)-thiazol-2-yl]-amidsäure,
• (R)-1-(pyrazin-2-carbonyl)-piperidin-3-carboxyl-[4-(5-cyclohexyl-2-methoxy-phenyl)-thiazol-2-yl]-amidsäure,
• 1-(1-oxy-pyridin-2-carbonyl)-pyrrolidin-3-carboxyl-[4-(5-cyclohexyl-2-methoxy-phenyl)-thiazol-2-yl]-amidsäure,
• (R)-1-(1-oxy-pyridin-2-carbonyl)-pyrrolidin-3-carboxyl-[4-(5-cyclohexyl-2-methoxy-phenyl)-thiazol-2-yl]-amidsäure.

8. Methode zur Herstellung einer Verbindung der Formel (I) nach einem der Ansprüche 1 bis 7, **dadurch gekennzeichnet, dass** eine Verbindung der folgenden Formel (V): 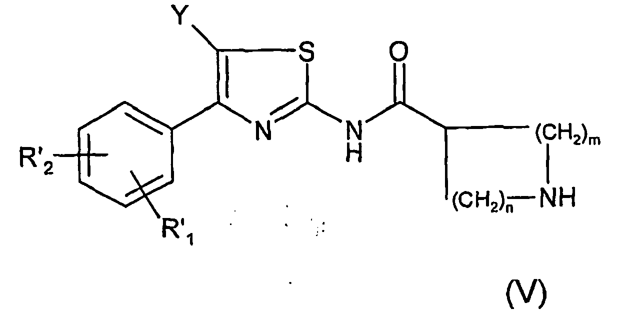 worin R'₁ und R'₂ Precursor von R₁ und R₂, welche wie im Anspruch 1 definiert sind, darstellen, und Y, m und n wie in Anspruch 1 definiert sind, mit einer Verbindung der folgenden Formel (V1): 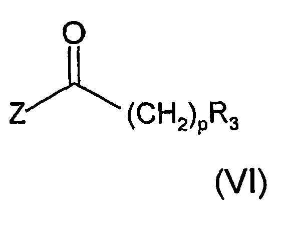 worin Z eine Abgangsgruppe ist und R₃ und p wie im Anspruch 1 definiert sind, in Gegenwart eines Haftvermittlers bei einer Temperatur zwischen 0 °C und 150 °C reagiert.

9. Verbindung der Formel (V'): 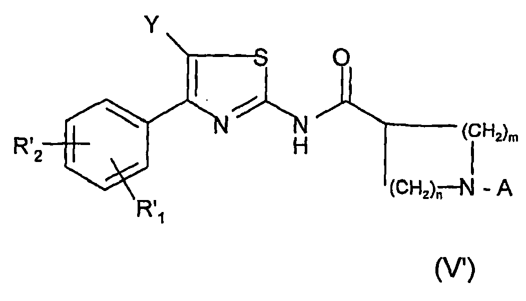 worin R'₁ und R'₂ Precursor von R₁-und R₂ sind, welche wie im Anspruch 1 definiert sind, und Y, m und n wie in Anspruch 1 definiert sind, und A ein Wasserstoffatom oder eine Schutzgruppe (Gp), welche tert-butoxycarbonyl (Boc) ist, darstellt.

10. Arzneimittel, **dadurch gekennzeichnet, dass** es eine Verbindung der Formel (I) nach einem der Ansprüche 1 bis 7 enthält.

11. Pharmazeutische Verbindung, **dadurch gekennzeichnet, dass** sie eine Verbindung der Formel (I) nach einem der Ansprüche 1 bis 7, sowie mindestens ein pharmazeutisch brauchbares Trägermaterial enthält.

12. Verwendung einer Verbindung der Formel (I) nach einem der Ansprüche 1 bis 7 für die Herstellung eines Arzneimittels zur Behandlung von und zur Vorbeugung von akuten oder chronischen immuno-entzündlichen Krankheiten und Syndromen, wie Arteriosklerose, allergische Erkrankungen, Krebs sowie Erkrankungen an welchen angiogene Prozesse beteiligt sind.

13. Verwendung einer Verbindung der Formel (I) nach einem der Ansprüche 1 bis 7 für die Herstellung eines Arzneimittels zur Behandlung von und Vorbeugung gegen Viruserkrankungen oder bakteriellen Erkrankungen, Herzkrankheiten, Fettsucht, Krebs.
